# EUROPEAN PATENT APPLICATION

(11) **EP 4 306 637 A1**
(43) Date of publication of application: **17.01.2024**
(21) Application number: 22306064.1
(22) Date of filing: 13.07.2022
(51) Int. Cl.: C12N 7/00, C07K 14/005

(54) **BACTERIOPHAGE(S) TARGETING CAPSULAR DEFICIENT KLEBSIELLA PNEUMONIAE (KP), COMPOSITIONS COMPRISING IT(THEM) AND USE(S) THEREOF**

(71) Applicant: INSTITUT PASTEUR, 75724 Paris Cedex 15 (FR)
(72) Inventor: MANSOS LOURENÇO, Marta, 75724 PARIS CEDEX 15 (FR); BRISSE, Sylvain, 75724 PARIS CEDEX 15 (FR)
(74) Representative: Ernest Gutmann - Yves Plasseraud S.A.S.

(57) **Abstract**

The invention relates to a bacteriophage targeting capsular-deficient *Klebsiella pneumoniaee* (Kp), comprising particular tail fiber polypeptides, which is capable of infecting at least two capsular-deficient *Klebsiella pneumoniae* of distinct O-types, in particular at least three distinct O-types of capsular-deficient *Klebsiella pneumoniae.* The invention also relates to a pharmaceutical composition comprising the same, a nucleic acid molecule having at least 99% of identity with the genomic sequence of the said bacteriophage, and the use of the said bacteriophage or composition(s) as a medicament, especially against an infection caused by at least one type of *Klebsiella pneumoniae* (Kp). The invention also relates to a method for decreasing the bacterial load of at least one *Klebsiella pneumoniae* (Kp) strain in the environment, making use of said bacteriophage.

## Description

The present invention pertains to the field of bacteriophages and their uses, especially in therapy.

The invention relates to a bacteriophage suitable for targeting a capsular-deficient *Klebsiella pneumoniae* strain, wherein the said bacteriophage has a peculiar polypeptide which is or is a part of a tail fiber protein or polypeptide, and the said bacteriophage is capable of infecting a broad range of capsular-deficient *Klebsiella pneumoniae* strains, i.e., of distinct O-types. Herein, the targeted *K*. *pneumoniae* should be understood as designating *K. pneumoniae* and closely related species, collectively grouped under the *"Klebsiella pneumoniae* species complex" as described by Wyres KL, Lam MMC, Holt KE. in Population genomics of Klebsiella pneumoniae. Nature Reviews Microbiology 2020;18:344-359. These species are *K. pneumoniae* (in the strict sense), *K. quasipneumoniae, K. variicola, K. quasivariicola* and *K. africana* are typically identified as *K. pneumoniae* or *K. variicola* in routine microbiology diagnostic laboratories. The invention also relates to compositions, in particular pharmaceutical compositions comprising the same or so-called "cocktails" of bacteriophages of the invention in admixture with other types of bacteriophages, in particular targeting capsular *Klebsiella pneumoniae* strains, nucleic acid molecules encoding the polypeptides described herein, and therapeutic uses of the means described herein, especially against an infection caused by *Klebsiella pneumoniae* (Kp) in a human or an animal.

Is also encompassed a method for decreasing the bacterial load for at least one type *Klebsiella pneumoniae* (Kp), in the environment, comprising putting the environment in contact with an effective amount of bacteriophages described herein, to the proviso that the environment is not an animal or human body.

Antimicrobial resistance in bacterial pathogens is a critical health threat, with *Klebsiella pneumoniae* (Kp) or a closely related species from the *"Klebsiella pneumoniae* species complex", as one of its major players. *Klebsiella pneumoniae* (Kp) is a common human gut colonizer and an opportunistic pathogen responsible for a broad range of infections including urinary tract or lung infections, liver abscess, meningitis and septicaemia. The increasing trends in the number of multidrug resistant (MDR) Kp infections have led to its recognition as a critical priority pathogen by public health agencies (Rice 2008). In a recent study, MDR Kp ranked third among the six leading pathogens responsible for approximately 1.27 million deaths in 2019 attributable to antimicrobial resistance (AMR) (Antimicrobial Resistance Collaborators 2022). In addition, the growing number of reports of pandrug-resistant Kp strains (Rodrigues et al. 2022), untreatable by current antimicrobial therapy, stresses the need of developing alternative and complementary therapies targeting MDR Kp.

One of these complementary approaches, known as phage therapy, relies on the use of virulent bacteriophages (henceforth, phages), which are viruses that target and kill bacteria, while replicating within them (Roach and Debarbieux 2017). The killing efficacy of phages relies, among others, on their ability to recognize and attach to the bacterial surface, implying a high specificity towards surface structures. As a downside to this strategy however, for Klebsiella, classically isolated phages are highly specific for capsular types (anti-K phages), and the polymorphism of Klebsiella capsule structures has limited phage therapy prospects.

In Kp, the most external structure is the capsule (K-antigen), a thick layer of polysaccharides that surrounds the bacteria and masks other cell wall structures, such as the lipopolysaccharide (LPS) and its O-antigen. This makes the capsule the primary receptor for most phages that have been isolated against Kp up to now, since the strains used for isolation are usually in their wild-type capsule expressing state (C. R. Hsu et al. 2011; Hoyles et al. 2015; Gorodnichev et al. 2021; Hao et al. 2021; Bonilla et al. 2021; Eckstein et al. 2021; Pertics et al. 2021).

However, capsular structure polymorphism is high in Kp, and varies even within phylogenetically narrow clonal groups (Rodrigues et al. 2020; Wyres et al. 2019; Bowers et al. 2015; Rodrigues et al. 2022). Using serological methods, 77 distinct K-types (K1 to K82) have been recognized in *Klebsiella* (Ørskov and Ørskov 1984), but genomic analyses have uncovered many additional capsular polysaccharide synthesis (*cps*) loci (KL-types, KL101-KL186) (Lam, Wick, Watts, et al. 2021; Lam, Wick, Judd, et al. 2021), which likely synthesize yet distinct capsular structures. The analysis of public genomes (Lam, Wick, Watts, et al. 2021) revealed that a cocktail of therapeutic phages targeting the six most frequent K-types (KL107, K2, K24, KL106, K64, K17) would only cover 38% of Kp isolates involved in human infections; and 17 additional K-types would be needed to reach 75%.

Resistance to phages targeting the capsule has been shown to evolve relatively fast, and predominantly via loss of capsule production (Eckstein et al. 2021; Hesse et al. 2020; Fang et al. 2022; Majkowska-Skrobek et al. 2021). This phage escape mechanism thus results in the exposure of other membrane structures such as the LPS O-antigen or outer membrane proteins. O-types (8 types serologically defined and 4 putative other ones defined by genomic analysis) and other surface proteins tend to be less diverse. For example, types O1, O2 and O3b cover 80% of Kp human infections (Lam, Wick, Watts, et al. 2021; Lam, Wick, Judd, et al. 2021). Furthermore, *in vivo* the capsular polysaccharide may not always be present around Kp cells. A recent study has shown the presence of capsule appears to be necessary for Kp dissemination in the bloodstream to different organs, but it is associated with a fitness cost in the gut (Y. H. Tan et al. 2020).

Given their exposure in some *in vivo* conditions or upon anti-capsule phage use, and their higher conservation, molecular structures lying below the capsule could represent interesting phage targets (Bengoechea and Sa Pessoa 2019; Y. H. Tan et al. 2020).

The present invention arises from an original anti-Klebsiella phage isolation strategy aiming at isolating phages that target capsule-deficient *Klebsiella pneumoniae* bacteria.

In these experiments, inventors have isolated and characterised phages targeting the structures discussed above, here collectively referred as anti-K^{d} [for anti-capsular-deficient] phages, and then compared them *in silico, in vitro* and *in vivo,* with anti-K phages (capsule-targeting phages).

Surprisingly, the isolated anti-K^{d} phages have broad host ranges. Therefore, the invention paves the way to therapeutic strategies based on either the use of such broad host range bacteriophages of the invention alone or within phage cocktails combining anti-K^{d} phages with capsule-targeting phages (anti-K phages) and/or capsule degrading components (e.g., depolymerases), with great promises. Such strategies may also address the issue of anti-phage resistance emergence during treatment, as simultaneous modifications in different membrane structures that are needed for simultaneous resistance to anti-K and anti-Kd phages, would require additional mutations and would probably result in high fitness costs (Koskella et al. 2012).

The invention relates to a bacteriophage targeting a capsular-deficient *Klebsiella pneumoniae* (Kp), wherein:
a. the bacteriophage comprises a tail fiber polypeptide of SEQ ID NO:1 (mtp5) or SEQ ID NO: 2 (mtp7) or a variant thereof having at least 96% identity with SEQ ID NO: 1 or SEQ ID NO: 2, and
b. said phage is capable of infecting at least two capsular-deficient *Klebsiella pneumoniae* strains of distinct O-types, in particular at least three distinct capsular-deficient *Klebsiella pneumoniae.*

In the context of present invention, when reference is made to the targeted *K. pneumoniae,* and as stated above, it should be understood that reference is made to *K. pneumoniae* and closely related species, collectively grouped under the *"Klebsiella pneumoniae* species complex" as described by Wyres KL, Lam MMC, Holt KE. in Population genomics of Klebsiella pneumoniae. Nature Reviews Microbiology 2020;18:344-359. These species are *K. pneumoniae* (in the strict sense), *K. quasipneumoniae, K. variicola, K. quasivariicola* and *K. africana.* As stated in Box 1 page 345 of Nature Reviews Microbiology 2020;18:344-359, incorporated herein by reference, the skilled person can readily assess whether a bacteria pertains to the *Klebsiella pneumoniae* species complex using the *Klebsiella pneumoniae* multilocus sequence typing (MLST) scheme.

According to a particular embodiment, the targeted *K. pneumoniae* is *K. pneumoniae* in the strict sense.

By "capsular-deficient *Klebsiella pneumoniae"* or "capsular-deficient *Klebsiella pneumoniae* strain" it is meant a bacterium where the capsule (K-antigen), i.e., a thick layer of polysaccharides that surrounds the bacteria and masks other cell wall structures, such as the lipopolysaccharide (LPS) and its O-antigen, is not present or is degraded to an extent where the lipopolysaccharide (LPS) and its O-antigen become accessible to targeting moieties, such a K^{d} phages. Expressions such as "capsule-deficient *Klebsiella pneumoniae",* "non-capsule *Klebsiella pneumoniae",* "non-capsulated *Klebsiella pneumoniae"* and "capsule-disrupted *Klebsiella pneumoniae"* may be used interchangeably for designation of capsular-deficient *Klebsiella pneumoniae.* As stated above, according to particular embodiments and unless stated differently, *Klebsiella pneumoniae* in this context encompass bacteria from the *"Klebsiella pneumoniae* species complex".

By "tail fiber protein" or "tail fiber polypeptide" it is meant a polypeptide that is capable of a specific recognition of a bacterial surface structure, in present case of a capsular-deficient *Klebsiella pneumoniae,* during the early step of viral infection.

Reference tail fiber proteins are provided herein under SEQ ID NO: 1 (mtp5 phage) and SEQ ID NO: 2 (mtp7 phage).

According to a particular embodiment, the "tail fiber protein" or "tail fiber polypeptide" referred to in present description is a L-shaped tail fiber protein of a bacteriophage. In particular, SEQ ID NO: 1 or SEQ ID NO: 2 or variants thereof as defined herein, are L-shaped tail fiber proteins. "L-shaped tail fiber proteins" are documented in the literature. Those are part of the "tail fibers" or "tail fibers" portions of bacteriophages, in the sense given in layman terms.

By "having at least 96% identity with SEQ ID NO: 1", it is meant at least 96%, at least 97%, at least 98%, at least 99% identity with SEQ ID NO: 1. Identity percentages can be calculated according to the common knowledge of the skilled person in the field, or following the guidance provided later in present description. Software tools for carrying out identity percentage calculation are commonly known and readily accessible to the skilled person: they can in particular be freely accessible over the internet. The literature provides details regarding available tools. In particular, identity percentages can conventionally be calculated through local or global, sequence alignment algorithms and their available computerized implementations. In a particular embodiment, identity percentages are calculated over the entire length of the compared sequences. Global alignments, which attempt to align every residue in every sequence, are most useful when the sequences in the query set are similar and of roughly equal size. Computerized implementations of the algorithms used are generally associated with default parameters in the literature, which can be used for running on or the other of such algorithm(s). The skilled person can readily adapt the same taking into account its objective or the sequences comparison made.

According to a particular embodiment, identity percentages are calculated over the whole length of a considered reference sequence (e.g., SEQ ID NO: 1) to which another sequence is compared to, using the standard parameters of the BLAST algorithm, especially blastp (protein-protein BLAST) algorithm, available at https: //blast.ncbi.nlm.nih.gov/Blast.cgi . The same applies to other SEQ ID Nos as detailed herein.

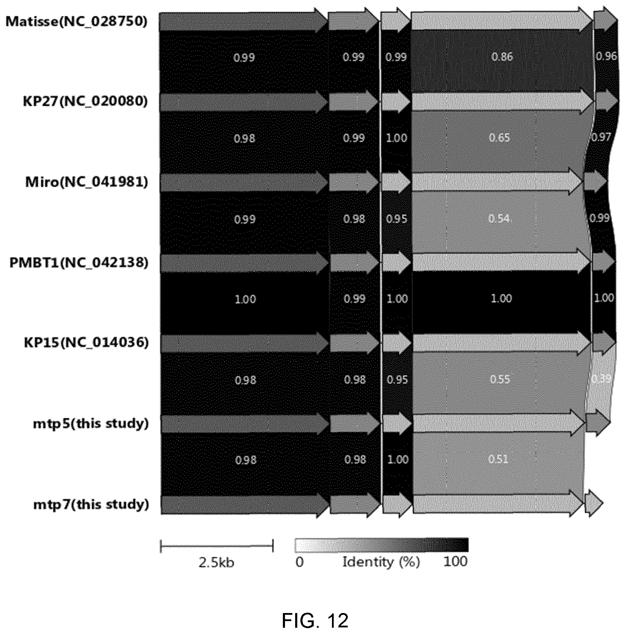

According to a particular embodiment, identity percentages are calculated over the whole length of a considered reference sequence (e.g., SEQ ID NO: 1) to which another sequence is compared to, using the algorithm of the "clinker" software available at https: //github.com/gamcil/clinker. This software, which was used for obtaining the data depicted in Figure 12 herein, automatically extract protein translations from a given set of fasta sequences, and perform global alignments between sequences in each cluster to determine the optimal order for visualization display based on cluster similarity (Colours are generally given randomly by the software taking in consideration the homology of the different genes).

According to a particular embodiment, a bacteriophage of the invention is a recombinant bacteriophage, and/or is an isolated bacteriophage, and/or is a bacteriophage that differs from known bacteriophages, especially natural bacteriophages, in particular is a mutated bacteriophage that differs from a bacteriophage of reference (especially a natural bacteriophage) by at least 1, 2 or 3 nucleotide substitutions or deletions within the phage genome, and/or amino-acid substitutions or deletions within the tail fiber portion corresponding to SEQ ID NO: 1 or SEQ ID NO: 2.

In a particular embodiment, the bacteriophage is a recombinant bacteriophage that has been recombined for the gene of the tail fiber protein to express the polypeptide of SEQ ID NO: 1 or SEQ ID NO: 2 or variants thereof as detailed herein, in particular a variant still capable of infecting at least two capsular-deficient *Klebsiella pneumoniae* strains of distinct O-types, in particular at least three distinct capsular-deficient *Klebsiella pneumoniae.*

Of note, it is commonly acknowledged that the boundary of 95% identity percentage is the percentage that distinguishes two species. Furthermore, currently nucleotide or protein sequence homology is the most used definition for distinguishing families of phages - see Adriaenssens E, Brister JR. How to Name and Classify Your Phage: An Informal Guide. Viruses. 2017 Apr 3;9(4):70. doi: 10.3390/v9040070. PMID: 28368359; PMCID: PMC5408676. SEQ ID NO: 1 and SEQ ID NO: 2 have been determined to be the sequences that distinguishes the best between the phages determined to be the most representative of present invention, and other phages, especially from the art (see experimental section herein). And finally, the skilled person can appreciate that there is a link between the tail fiber protein(s) of a phage, in particular the L-shaped tail fiber protein and the specificity of said phage, including scope of infection of said phage, see Heller K, Braun V. Polymannose O-antigens of Escherichia coli, the binding sites for the reversible adsorption of bacteriophage T5+ via the L-shaped tail fibers. J Virol. 1982 Jan;41(1):222-7. doi: 10.1128/JVI.41.1.222-227.1982. PMID: 7045389; PMCID: PMC256742 and Garcia-Doval C, Castón JR, Luque D, Granell M, Otero JM, Llamas-Saiz AL, Renouard M, Boulanger P, van Raaij MJ. Structure of the Receptor-Binding Carboxy-Terminal Domain of the Bacteriophage T5 L-Shaped Tail Fibre with and without Its Intra-Molecular Chaperone. Viruses. 2015 Dec 8;7(12):6424-40. doi: 10.3390/v7122946. PMID: 26670244; PMCID: PMC4690869.

By "O-types" it is meant the types corresponding to the O-antigens that form the outermost component of the lipopolysaccharide (LPS) layer of *Klebsiella pneumoniae,* as determined classicaly by serotyping of Klebsiella spp., as commonly achieved by a skilled person having knowledge in Klebsiella spp. manipulations. As of now, there are 11 known O-types, 7 types serologically defined (O1, O2, O3, O4, O5, O8, O12) and 4 putative other ones defined by genomic analysis (OL101, OL102, OL103, OL104) - see Follador R, Heinz E, Wyres KL, Ellington MJ, Kowarik M, Holt KE, Thomson NR. Of note, some authors may consider that O3/O3A is a O-type that is different from the O3b O-type. In this latter case, the total amount of known O-types is of 12, as of today. Unless the contrary is apparent from the context, it is considered herein that O3 refers to a single O-type, and there are 11 known O-types to date. The diversity of Klebsiella pneumoniae surface polysaccharides. Microb Genom. 2016 Aug 25;2(8):e000073. doi: 10.1099/mgen.0.000073. PMID: 28348868; PMCID: PMC5320592 and Lam MMC, Wick RR, Judd LM, Holt KE, Wyres KL. Kaptive 2.0: updated capsule and lipopolysaccharide locus typing for the Klebsiella pneumoniae species complex. Microb Genom. 2022 Mar;8(3):000800. doi: 10.1099/mgen.0.000800. PMID: 35311639; PMCID: PMC9176290.

By "at least two capsular-deficient *Klebsiella pneumoniae* strains of distinct O-types" it is intended to mean that the bacteriophage targets, or infects, or targets and infects a plurality of capsular-deficient *Klebsiella pneumoniae* strains that distinguish from one another by their O-antigen and accordingly may be characterized as distinct O-types or O-serotypes. The bacteriophage targeting, or infecting, or targeting and infecting at least two capsular-deficient *Klebsiella pneumoniae* strains has accordingly a broad specificity that extends at least to two distinct, i.e., different or distinguishable between them, O-types of *Klebsiella pneumoniae* bacteria.

By "at least two", it is meant, according to a particular embodiment, 3, 4, 5, 6, 7, 8, 9 or 10 distinct O-types or at least these numbers of distinct O-types. For example, Table 1 describes 7 capsule-deficient *Klebsiella pneumoniae* strains, corresponding to 3 distinct O-types, i.e., O-types O1, O2 and O3. The skilled person is knowledgeable regarding O-antigen serotyping, and can readily implement a method aimed at determining the O-type classification of *Klebsiella pneumoniae* strains. An exemplary technique of serotyping is described in Orskov, I., and F. Orskov. 1984b. Serotyping of Klebsiella. Meth. Microbiol. 14:143-164, which can readily be used or followed for guidance by the skilled person. O-antigens can also be predicted more conveniently by defining the O-locus type of the strains, based on the genomic sequence of the strains - see Lam MMC, Wick RR, Judd LM, Holt KE, Wyres KL. Kaptive 2.0: updated capsule and lipopolysaccharide locus typing for the Klebsiella pneumoniae species complex. Microb Genom. 2022 Mar;8(3):000800. doi: 10.1099/mgen.0.000800. PMID: 35311639; PMCID: PMC9176290 already cited above. Furthermore, a comprehensive description of capsule deficient *Klebsiella pneumoniae* strains can be found in present description, e.g., 7 mutant strains have been tested (Table 1 and herein). Furthermore, such strains may derive from natural evolutionary mechanisms, for example by escaping the selective pressure exerted by the use of anti-K phages, which often results in the loss of the capsule production.

According to particular embodiments and as a proviso to the bacteriophages encompassed within present invention, a bacteriophage of the invention does not encompass the anti-K. *pneumoniae* phages Matisse (NC_028750.1), Kp27 (NC_020080.1), Kp15 (NC_014036.1), Miro (NC_041981.1) and PMBT1 (NC_042138.1).

In a particular embodiment the bacteriophage of the invention does not encompass such bacteriophages Matisse (NC_028750.1), Kp27 (NC_020080.1), Kp15 (NC_014036.1), Miro (NC_041981.1) and PMBT1 (NC_042138.1), especially those bacteriophages when they target a capsular-deficient *Klebsiella pneumoniae* strain or when they have structural features conferring to them the property of targeting a capsular-deficient *Klebsiella pneumoniae* strain.

According to particular embodiments, detailed hereafter, the tail fiber is a complex of proteins, or an assembly of proteins. When carried out, substitution of phage tail fibers can be made with the inclusion of polypeptide portions which are around or connected to the considered core tail fiber protein. For instance, Figure 12 depicts the genetic structure of the anti-K^{d} phages mtp5 and mtp7 tail fibers genes with the sequences that are the long tail fiber proximal unit, the hinge connector of long tail fiber proximal connector, the hinge connector of long tail fiber distal connector and the distal long tail fiber assembly catalyst in addition to the L-shaped tail fiber protein.

Therefore, according to a particular embodiment, a bacteriophage of the invention further comprises a polypeptide that is a hinge connector of long tail fiber distal connector of SEQ ID NO: 3 (mtp5) or SEQ ID NO: 4 (mtp7) or a variant thereof having at least 99.9% identity with SEQ ID NO: 3 or SEQ ID NO: 4. The said polypeptide is to be found with the tail fiber as referred to herein (or to be found in, or part of, the tail fiber of the said bacteriophage, in layman's terms).

According to a particular embodiment, taken alone or in combination with any other embodiment described herein, a bacteriophage of the invention further comprises a polypeptide that is a hinge connector of long tail fiber proximal connector of SEQ ID NO: 5 (mtp5) or SEQ ID NO: 6 (mtp7) or a variant thereof having at least 99.5% identity with SEQ ID NO: 5 or SEQ ID NO: 6. The said polypeptide is to be found with the tail fiber protein as referred to herein (or to be found in, or part of, the tail fiber of the said bacteriophage, in layman's terms).

According to a particular embodiment, taken alone or in combination with any other embodiment described herein, a bacteriophage of the invention further comprises a polypeptide that is a long tail fiber proximal unit of SEQ ID NO: 7 (mtp5) or SEQ ID NO: 8 (mtp7) or a variant thereof having at least 99.9% identity with SEQ ID NO: 7 or SEQ ID NO: 8. The said polypeptide is to be found with the tail fiber protein as referred to herein (or to be found in, or part of, the tail fiber of the said bacteriophage, in layman's terms).

According to a particular embodiment, taken alone or in combination with any other embodiment described herein, a bacteriophage of the invention further comprises a polypeptide that is a distal long tail fiber assembly catalyst of SEQ ID NO: 9 (mtp5) or SEQ ID NO: 10 (mtp7) or a variant thereof having at least 99.8% identity with SEQ ID NO: 9 or SEQ ID NO: 10.

It nonetheless has to be observed that the other portions of the tail fibers recited above, apart the tail fiber polypeptide (e.g., the L-shaped tail fiber protein referred to), are deemed to be small proteins connecting the tail fibers, which are not necessarily necessary for the resulting phage in order for it to recognize its target.

According to another particular embodiment, taken alone or in combination with any other embodiment described herein, a bacteriophage of the invention has a virion particle structure of a Myoviridae bacteriophage, e.g., is a Myoviridae-like bacteriophage, or is a Myoviridae bacteriophage.

By "has a virion particle structure of a Myoviridae bacteriophage" it is meant that the bacteriophage has the features commonly found in Myoviridae bacteriophages at the level of the bacteriophage tail, i.e., a more or less rigid contractile tail, the bacteriophage is long and relatively thick, and consist of a central core built of stacked rings of six subunits and surrounded by a helical contractile sheath. See also Zinke M, Schroder GF, Lange A. Major tail proteins of bacteriophages of the order Caudovirales. J Biol Chem. 2022 Jan;298(1):101472. doi: 10.1016/j.jbc.2021.101472. Epub 2021 Dec 8. PMID: 34890646; PMCID: PMC8718954 for a description of the structural features of Myoviridae bacteriophages.

The Myoviridae bacteriophage family is for example described in "Family - Myoviridae", Editors: Andrew M.Q. King, Michael J. Adams, Eric B. Carstens, Elliot J. Lefkowitz, Virus Taxonomy, Elsevier, 2012, Pages 46-62, ISBN 9780123846846, https: //doi.org/10.1016/B978-0-12-384684-6.00002-1. Lavigne R, Darius P, Summer EJ, Seto D, Mahadevan P, Nilsson AS, Ackermann HW, Kropinski AM. Also, "Classification of Myoviridae bacteriophages using protein sequence similarity". BMC Microbiol. 2009 Oct 26;9:224. doi: 10.1186/1471-2180-9-224. PMID: 19857251; PMCID: PMC2771037 describe the classification of Myoviridae bacteriophages using protein sequence similarity, which can readily be used by the skilled person in order to determine whether a bacteriophage is a Myoviridae bacteriophage. An exemplary Myoviridae bacteriophage is the T4 bacteriophage.

According to a particular embodiment, taken alone or according to all possible combinations with embodiments described herein, a bacteriophage of the invention comprises a genomic sequence having at least 90% of identity, in particular at least 99% of identity, with the genomic sequence of vB_KpM_SB4496_mtp5 represented by SEQ ID NO: 11 or the genomic sequence of vB_KpM_SB4975_mtp7 represented by SEQ ID NO: 12, or fragments of these sequences.

By "at least 90% identity" it is meant at least 90%, at least 91%, at least 92%, at least 93%, at least 94%, at least 95%, at least 96%, at least 97%, at least 98%, or at least 99% identity with the respective considered reference sequence and preferably at least 99% identity with the respective considered reference sequence.

By "at least 99% identity" at least 99% identity with the respective considered reference sequence, in particular at least 99.1%, or 99.2%, or 99.3%, or 99.4%, or 99.5%, or 99.6%, or 99.7%, or 99.8%, or 99.9% identity with the respective considered reference sequence.

Identity percentages can be calculated according to the common knowledge of the skilled person in the field, or following the guidance provided later in present description (see above). In a particular embodiment, identity percentages are calculated over the entire length of the compared sequences, i.e., 100% cover or coverage.

By "fragments thereof", it is meant that the bacteriophage comprises a fragment of the genomic sequence represented by SEQ ID NO: 11 or SEQ ID NO: 12 or a sequence having at least 90% of identity with SEQ ID NO: 11 or SEQ ID NO: 12 (or more identity, see above), the fragment having at least 50% of the size of SEQ ID NO: 11 or SEQ ID NO: 12 taken as a reference sequence, or more. The fragment can encompass the nucleotide sequence encoding all or part of the regions described above, according to all combinations and preferably with the sequence corresponding to the tail fiber polypeptide region (e.g., L-shaped tail fiber protein) being present, e.g., the tail fiber polypeptide region (SEQ ID NO: 1 or 2), the hinge connector of long tail fiber distal connector region (SEQ ID NO: 3 or 4), the hinge connector of long tail fiber proximal connector region (SEQ ID NO: 5 or 6), the long tail fiber proximal unit region (SEQ ID NO: 7 or 8), the distal long tail fiber assembly catalyst region (SEQ ID NO: 9 or 10).

According to a particular embodiment, a bacteriophage of the invention has a genomic sequence encoding all or part of the regions described above, e.g., the tail fiber polypeptide region (SEQ ID NO: 1 or 2), the hinge connector of long tail fiber distal connector region (SEQ ID NO: 3 or 4), the hinge connector of long tail fiber proximal connector region (SEQ ID NO: 5 or 6), the long tail fiber proximal unit region (SEQ ID NO: 7 or 8), the distal long tail fiber assembly catalyst region (SEQ ID NO: 9 or 10), with the reminder of its genomic sequence having at least 90% of identity with the genomic sequence of vB_KpM_SB4496_mtp5 represented by SEQ ID NO: 11 or the genomic sequence of vB_KpM_SB4975_mtp7 represented by SEQ ID NO: 12, or fragments of these sequences, as defined above.

According to a particular embodiment, a bacteriophage of the invention comprises or consists essentially of or consists of a genomic sequence having at least 99% of identity with the genomic sequence of vB_KpM_SB4496_mtp5 represented by SEQ ID NO: 11 or the genomic sequence of vB_KpM_SB4975_mtp7 represented by SEQ ID NO: 12.

According to a particular embodiment, taken alone or according to all possible combinations with embodiments described herein, a bacteriophage of the invention is:
a. the bacteriophage strain vB_KpM_SB4496_mtp5 deposited at the French National Collection of Microorganisms at the Institut Pasteur under Accession Number CNCM I-5855 on June 27, 2022 or a variant bacteriophage, in particular wherein the variant has the same phenotypic and/or functional features as the parent bacteriophage, or
b. the bacteriophage strain vB_KpM_SB4975_mtp7 deposited at the French National Collection of Microorganisms at the Institut Pasteur under Accession Number CNCM I-5856 on June 27, 2022 or a variant bacteriophage, in particular wherein the variant has the same phenotypic and/or functional features as the parent bacteriophage.

The address of CNCM is: Collection Nationale de Culture de Microorganismes, Institut Pasteur, 25-28 rue du Docteur Roux, 75724 Paris CEDEX 15, France.

By "variant thereof", in the above context of a variant bacteriophage from a deposited bacteriophage, it is meant that the variant bacteriophage is either a progeny of the parent bacteriophage strain or is a bacteriophage having a genomic sequence with a substantial identity percentage with respect to the parent strain, i.e., at least 95%, or 96%, or 97%, or 98%, or 99% or 100% nucleotide sequence identity, in particular over the whole length of the aligned sequence. Identity percentages can be calculated as indicated above (in particular through a nucleotide blast algorithm).

"Progeny" means a bacteriophage replicate consisting of descendants produced by subculturing a vB_KpM_SB4496_mtp5 strain or vB_KpM_SB4975_mtp7 strain referred to herein, by a method commonly known to a skilled person in the art (and conventionally used).

According to a particular embodiment, in the above context of a variant strain of a vB_KpM_SB4496_mtp5 strain or vB_KpM_SB4975_mtp7 strain, a "variant" has the same phenotypic and/or functional features as the parent strain.

By "functional features" it is for example meant:
- the same specificity against a capsular deficient *Klebsiella pneumoniae* (Kp) strain, as the parent strain, and/or
- the presence of a polypeptide of SEQ ID NO:1 or SEQ ID NO: 2 or a variant thereof having at least 96% identity with SEQ ID NO: 1 or SEQ ID NO: 2, which polypeptide corresponds to a tail fiber protein, and/or
- the capability of the bacteriophage to target and/or infect at least two capsular deficient *Klebsiella pneumoniae* strains of distinct O-types, in particular at least three capsular deficient *Klebsiella pneumoniae* strains of distinct O-types, and/or
- has the same lytic activity, for example the 'lytic infection" capability of the parent strain, according to the definitions provided herein, in particular the capability of inducing a lytic infection that can be observed on a broad range of capsular deficient *Klebsiella pneumoniae* strains, i.e., a range encompassing at least two (and up to at least 10, as described above) capsular deficient *Klebsiella pneumoniae* strains of distinct O-types.

Reference is made to the definition provided above, with regards to the "distinct O-types" feature and thus extent of the range (from at least 2 to at least 10).
- has the same lytic activity, for example as stated above, and the same phenotypic characteristics as said bacteriophage strain.

The capsular *Klebsiella pneumoniae* strain BJ1-GAΔ*wza* described in the experimental section herein can be a target and host strain for vB_KpM_SB4496_mtp5 and vB_KpM_SB4975_mtp7, for the purpose of cultivating them or for titration experiments. Of note, the capsular *Klebsiella pneumoniae* strain BJ1-GAΔ*wza* has been deposited at the French National Collection of Microorganisms at the Institut Pasteur, as an associated deposit to deposits having Accession Numbers CNCM I-5855 and CNCM I-5856 filed on June 27, 2022 referred to above, as the strain used for cultivating the bacteriophages of deposits having Accession Numbers CNCM I-5855 and CNCM I-5856.

Suitable methods for isolating pure bacteriophage strains from a bacteriophage-containing sample such as those referred to above are well known, and such methods may be adapted by the skilled artisan in view of the guidance provided herein. Isolation of active bacteriophage from suitable samples typically proceeds by mixing the sample with nutrient broth, inoculating the broth with a host bacterial strain, and incubating to enrich the mixture with bacteriophage that can infect the host strain. The accessible *Klebsiella pneumoniae* strain BJ1-GAΔ*wza* can be used as the host bacterial strain. After the incubation for enrichment, the mixture is filtered to remove bacteria, leaving the bacteriophage in the filtrate. Serial dilutions of the filtrate can be plated on a lawn of bacteria; active bacteriophages, i.e., lytic bacteriophages according to the definitions provided herein, infect and lyse neighbouring bacteria. The agar limits the physical spread of the bacteriophage throughout the plate, resulting in small visibly clear areas called plaques on the plate where bacteriophage has destroyed the bacteria within the confluent lawn of growth. Since one plaque with a distinct morphology represents one bacteriophage particle that replicated in the bacteria within that area of the bacterial lawn, the purity of a bacteriophage preparation can be ensured by removing the material in that plaque with a pasteur pipette (a "plaque pick") and using this material as the inoculum for further growth cycles of the bacteriophage. The bacteriophage produced in such cycles represents a single strain or "monophage." The purity of bacteriophage preparation (including confirmation that it is a monophage and not a polyvalent phage preparation) can be assessed by a combination of electron microscopy, SDS-PAGE, DNA restriction digest, analytical ultracentrifugation and cross-test against various bacterial strains. In addition, each phage can be uniquely identified by its DNA restriction digest profile, protein composition, and/or genome sequence.

Therefore, quantities of bacteriophage needed according to the invention, in particular for therapeutic uses described below, may be produced by culture on a suitable bacterial host strain in the manner described above for enrichment culture. When performing an enrichment culture to produce bacteriophage for therapeutic use, a host strain can be selected based on its ability to give a maximum yield of bacteriophage, as determined in pilot experiments with several different host. Although not limitative, the *Klebsiella pneumoniae* strain BJ1-GAΔ*wza* can be used as the bacterial host strain. The skilled person can readily and conventionally use another strain as host strain, if necessary after several enrichment culture testing. This matter is conventional in the art.

According to a particular embodiment, taken alone or in combination with any other embodiment described herein, a bacteriophage of the invention is capable of producing a lytic infection in a capsular-deficient *Klebsiella pneumoniae* strain. The bacteriophage that is capable of producing said lytic infection is any bacteriophage disclosed herein, let it be a particular embodiment of a specific bacteriophage, or a variant thereof. In this context, a "variant" means a bacteriophage which has the same functional features as the parent bacteriophage, for example, a variant bacteriophage which is a progeny of a parent bacteriophage but retains the lytic activity of the parent bacteriophage.

According to a particular embodiment, the lytic infection can be observed on a broad range of capsular-deficient *Klebsiella pneumoniae* strains, i.e., a range encompassing at least two (and up to at least 10, as described above) capsular-deficient *Klebsiella pneumoniae* strains of distinct O-types. Reference is made to the definition provided above, with regards to the "distinct O-types" feature and thus extent of the range (from at least 2 to 10 or from 2 to more than 10).

According to a particular embodiment, the said capsular-deficient *Klebsiella pneumoniae* strain is capsular-deficient *Klebsiella pneumoniae* BJ1-GA or a variant thereof (that is still capsular-deficient). By "variant" in this context, it is meant a capsular-deficient *Klebsiella pneumoniae* strain deriving from capsular-deficient *Klebsiella pneumoniae* BJ1-GA, i.e., is a progeny of BJ1-GA but retains the same phenotypic characteristics and/or functional features as the parent strain.

According to a particular embodiment, the said capsular-deficient *Klebsiella pneumoniae* is a *Klebsiella pneumoniae* BJ1-GAΔ*wza* referred to above or a variant thereof (that is still capsular-deficient). By "variant" in this context, it is meant a capsular-deficient *Klebsiella pneumoniae* strain deriving from capsular-deficient *Klebsiella pneumoniae* BJ1-GAΔ*wza*, i.e., is a progeny of BJ1-GAΔ*wza* but retains the same phenotypic characteristics and/or functional features as the parent strain.

By "lytic infection" it is meant that once the bacteriophage has targeted, i.e., recognized and attached to its target Kp, it is capable of infecting it so that lysis can be observed on a population of target Kp bacteria. Examples of experimentation enabling to report the existence of a lysis effect are described herein. According to a more particular aspect, it is possible to calculate an infection titer (examples provided herein) as well known by the skilled artisan. According to another aspect, relative to a Phage efficiency of plating (EOP) test, when used to appreciate the extent of a lysis, it is possible to determine the efficiency of plating (see Examples herein).

Accordingly, "lytic infection" means that lysis of the Kp host occurs once a bacteriophage has recognized and infected its target Kp. Lysis can be assessed on a population of target Kp bacteria by simple experimentation available to the skilled person.

According to a more particular embodiment, "lytic infection" is deemed to be present when an infection titer can be calculated on a population of target Kp bacteria. Titers are calculated using an exponential growing Klebsiella culture. Once a titer can be calculated, the condition is deemed to be met.

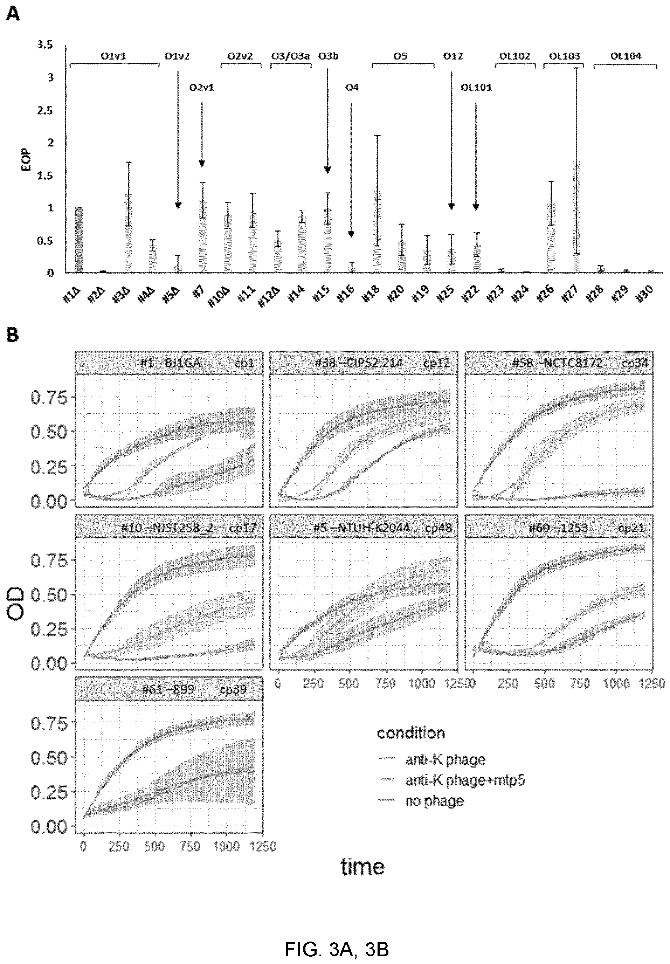

According to a more particular embodiment, "lytic infection" is deemed to be effective when an efficiency of plating can be observed. Similarly to the above, as soon as an efficiency of plating can be observed, the condition is deemed to be met (see Examples herein, Figure 3 in particular).

As described hereafter, a bacteriophage of the present invention is particularly suitable for therapeutical purposes.

Accordingly, the invention also relates to a pharmaceutical composition comprising the bacteriophage according to any embodiment described herein. According to a particular embodiment, such a composition comprises, or consists essentially of, or consists of, at least one bacteriophage of the invention and a pharmaceutically acceptable carrier or delivery vehicle(s). Such a composition may allow the bacteriophage of the invention, according to all embodiments described herein, to be found as a formulation adapted for the intended purpose, with carrier or delivery vehicle(s) chosen to meet the formulation requirements. Such a pharmaceutical composition can be stored as a concentrated aqueous solution or lyophilized powder preparation. A pharmaceutically acceptable carrier or excipient refers to a non-toxic solid, semi-solid or liquid filler, diluent, encapsulating material or formulation auxiliary of any type. Typically, a pharmaceutical composition contains vehicles, which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. Injectable solutions can be prepared by incorporating the bacteriophage of the present invention in the required amount in the appropriate solvent with several of the other ingredients enumerated above, as required, followed by filtered sterilization. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. Bacteriophage may also be formulated for oral administration by resuspending purified phage preparation in aqueous medium, such as deionized water, mineral water, 5% sucrose solution, glycerol, dextran, polyethylene glycol, sorbitol, or such other formulations that maintain phage viability, and are non-toxic to humans. The pharmaceutical composition may contain other components so long as the other components do not reduce the effectiveness (infectivity) of the bacteriophage so much that the therapy is negated. Pharmaceutically acceptable carriers are well known, and one skilled in the pharmaceutical art can easily select carriers suitable for particular routes of administration (Remington's Pharmaceutical Sciences, Mack Publishing Co., Easton, Pa., 1985).

Pharmaceutical compositions may be administered by parenteral (subcutaneously, intramuscularly, intravenously, intraperitoneally, intrapleurally, intravesicularly or intrathecally), topical, oral, rectal, inhalation, ocular, auricular, or nasal route, as necessitated by the situation, which can be appreciated by the skilled person.

Injection of specific lytic bacteriophages directly into the bloodstream can eliminate or significantly reduce the number of targeted bacteria in the blood. If, after either oral or local administration, bacteriophages get and persist into the bloodstream in sufficient numbers to eliminate bacteria from the bloodstream, septicemia may be treated by administering bacteriophages of the invention orally (or locally). If the bacteriophages do not get or do not persist into the bloodstream in sufficient numbers to eliminate bacteria from the bloodstream, the route of direct i.v. injection of bacteriophages for treating septic infections can be used to target the causative agent(s), i.e., bacteria, of infection, and therefore treat the infection arising in the bloodstream, if necessary by acting on both capsular-deficient Kp and other pathogenic bacteria (e.g., when a composition/cocktail as defined herein is used) and can provide an urgently needed means for dealing with currently untreatable septicemic infections. The phage may be administered orally in, for example, mineral water, optionally with 2.0 grams of sodium bicarbonate added to reduce stomach acidity. Alternatively, sodium bicarbonate may be administered separately to the patient just prior to dosing with the bacteriophage. Bacteriophages also may be formulated in a tablet or a capsule which will enable the transfer of bacteriophages through the stomach with no reduction of bacteriophage viability due to gastric acidity, and release of fully active bacteriophages in the small intestine. For non-oral administration, the composition of the present invention may be formulated into injections for subcutaneous, intravenous, or intramuscular routes, suppositories, or sprays inhalable via the respiratory tract, such as aerosols. Injection preparations may be obtained by dissolving or suspending the composition of the present invention, together with a stabilizer or a buffer, in water and packaging the solution or suspension in ampules or vial units. For sprays, such as aerosol, a propellant for spraying a water-dispersed concentrate or wetting powder may be used in combination with an additive.

As used herein, the term "effective amount" refers to a quantity sufficient to achieve a therapeutic effect (e.g. treating a infection). In the context of therapeutic or prophylactic applications, the amount of a composition administered to the subject will depend on the type and severity of the disease and on the characteristics of the individual, such as general health, age, sex, body weight and tolerance to drugs. It will also depend on the degree, severity and type of disease. The skilled artisan will be able to determine appropriate dosages depending on these and other factors. The dose of the bacteriophage and duration of therapy for a particular human patient or animal can be determined by the skilled clinician using standard pharmacological approaches in view of the above factors. The response to treatment may be monitored by, analysis of blood or body fluid levels of a targeted bacteria in relevant tissues or monitoring disease state in the patient or animal. The skilled clinician will adjust the dose and duration of therapy based on the response to treatment revealed by these measurements. Based on previous human experience, a dose of phage between 10⁶ and 10¹¹ PFU, in particular between 10⁶ and 10⁹ PFU, more particularly between 10⁷ and 10⁹ PFU, will be suitable in most instances for administration to a human patient (see Paul, K.; Merabishvili, M.; Hazan, R.; Christner, M.; Herden, U.; Gelman, D.; Khalifa, L.; Yerushalmy, O.; Coppenhagen-Glazer, S.; Harbauer, T.; Schulz-Jürgensen, S.; Rohde, H.; Fischer, L.; Aslam, S.; Rohde, C.; Nir-Paz, R.; Pirnay, J.-P.; Singer, D.; Muntau, A.C. Bacteriophage Rescue Therapy of a Vancomycin-Resistant Enterococcus faecium Infection in a One-Year-Old Child following a Third Liver Transplantation. Viruses 2021, 13, 1785. https:// doi.org/10.3390/v13091785, or Lebeaux, D.; Merabishvili, M.; Caudron, E.; Lannoy, D.; Van Simaey, L.; Duyvejonck, H.; Guillemain, R.; Thumerelle, C.; Podglajen, I.; Compain, F.; Kassis, N.; Mainardi, J.-L.; Wittmann, J.; Rohde, C.; Pirnay, J.-P.; Dufour, N.; Vermeulen, S.; Gansemans, Y.; Van Nieuwerburgh, F.; Vaneechoutte, M. A Case of Phage Therapy against Pandrug-Resistant Achromobacter xylosoxidans in a 12-Year-Old Lung-Transplanted Cystic Fibrosis Patient. Viruses 2021, 13, 60. https: //doi.org/10.3390/v13010060, or Little, J.S., Dedrick, R.M., Freeman, K.G. et al. Bacteriophage treatment of disseminated cutaneous Mycobacterium chelonae infection. Nat Commun 13, 2313 (2022). https:// doi.org/10.1038/s41467-022-29689-4).

The invention also relates to a nucleic acid molecule having at least 90%, in particular at least 99%, of identity with the genomic sequence of vB_KpM_SB4496_mtp5 represented by SEQ ID NO: 11 or the genomic sequence of vB_KpM_SB4975_mtp7 represented by SEQ ID NO: 12, or fragments of these sequences.

By "at least 90% identity" it is meant at least 90%, at least 91%, at least 92%, at least 93%, at least 94%, at least 95%, at least 96%, at least 97%, at least 98%, or at least 99% identity with the respective considered reference sequence and preferably at least 99% identity with the respective considered reference sequence.

By "at least 99% identity" it is meant at least 99% identity with the respective considered reference sequence, in particular at least 99.1%, or 99.2%, or 99.3%, or 99.4%, or 99.5%, or 99.6%, or 99.7%, or 99.8%, or 99.9% identity with the respective considered reference sequence. Identity percentages can be calculated according to the common knowledge of the skilled person in the field, or following the guidance provided later in present description (see above). In a particular embodiment, identity percentages are calculated over the entire length of the compared sequences, i.e., 100% cover or coverage.

By "fragments thereof", it is meant that the nucleic acid molecule is a fragment of the sequence represented by SEQ ID NO: 11 or SEQ ID NO: 12 or a sequence having at least 90% of identity with SEQ ID NO: 11 or SEQ ID NO: 12 (or more identity, see above). According to a particular embodiment, the fragment has at least 5.5%, or 5.6%, or 5.7%, or 5.8%, or 5.9% or 6.0% of the size of SEQ ID NO: 11 or SEQ ID NO: 12 taken as a reference sequence, or more. Such a size corresponds to the size of a nucleic acid molecule, which would be coherent with a capacity to encode all the CDS of the tail fiber polypeptides depicted in figure 12 herein, and documented in the present description. According to another particular embodiment, fragment has between 0.1% and 2.5%, and up to 3%, 4%, 5% or 6% of the size of SEQ ID NO: 11 or SEQ ID NO: 12 taken as a reference sequence. According to more specific embodiments, the length range from such a fragment is between from 0.1%, 0.2%, 0.3%, 0.4%, 0.5%, 0.6%, 0.7%, 0.8%, 0.9%, 1%, 1.1%, 1.2%, 1.3%, 1.4%, 1.5%, 1.6%, 1.7%, 1.8%, 1.9%, 2%, 2.1%, 2.2%, 2.3%, 2.4% and 2.5% and up to 1%, 1.1%, 1.2%, 1.3%, 1.4%, 1.5%, 1.6%, 1.7%, 1.8%, 1.9%, 2%, 2.1%, 2.2%, 2.3%, 2.4%, 2.5%, 3%, 3.5%, 4%, 4.5%, 5%, 5.5% and 6.0% of the size of SEQ ID NO: 11 or SEQ ID NO: 12 taken as a reference sequence, with all possible combinations of outer boundaries. Such a size corresponds to the size of a nucleic acid molecule, which would be coherent with a capacity to encode a particular CDS of any one of the tail fiber polypeptides depicted in figure 12 herein, and documented in the present description and sequence listing. In particular embodiments, the fragment can encompass the nucleotide sequence encoding all or part of the regions described above, e.g., the tail fiber polypeptide region (SEQ ID NO: 1 or 2), the hinge connector of long tail fiber distal connector region (SEQ ID NO: 3 or 4), the hinge connector of long tail fiber proximal connector region (SEQ ID NO: 5 or 6), the long tail fiber proximal unit region (SEQ ID NO: 7 or 8), the distal long tail fiber assembly catalyst region (SEQ ID NO: 9 or 10), in particular as disclosed above with regards to proteins. Of note, the nucleotide sequences encoding the tail fibers of SEQ ID NO: 1 and 2 are provided herein under SEQ ID NO: 13 and 14, respectively. The skilled person can readily determine the regions of SEQ ID NO: 11 or SEQ ID NO: 12, which correspond to the encoded proteins, using the nucleotide positions provided in the attached sequence listing, or on the basis of his knowledge, through translation of the considered protein sequences into nucleotide sequences.

According to a particular embodiment, a nucleic acid molecule has a sequence encoding all or part of the regions described above, e.g., the tail fiber polypeptide region (SEQ ID NO: 1 or 2), the hinge connector of long tail fiber distal connector region (SEQ ID NO: 3 or 4), the hinge connector of long tail fiber proximal connector region (SEQ ID NO: 5 or 6), the long tail fiber proximal unit region (SEQ ID NO: 7 or 8), the distal long tail fiber assembly catalyst region (SEQ ID NO: 9 or 10). According to a particular embodiment, a nucleic acid molecule has a sequence encoding all or part of the regions described above, e.g., the tail fiber polypeptide region (SEQ ID NO: 1 or 3), the hinge connector of long tail fiber distal connector region (SEQ ID NO: 3 or 4), the hinge connector of long tail fiber proximal connector region (SEQ ID NO: 5 or 6), the long tail fiber proximal unit region (SEQ ID NO: 7 or 8), the distal long tail fiber assembly catalyst region (SEQ ID NO: 9 or 10), with the remainder of the nucleic acid molecule sequence having at least 90% of identity with the genomic sequence of vB_KpM_SB4496_mtp5 represented by SEQ ID NO: 11 or the genomic sequence of vB_KpM_SB4975_mtp7 represented by SEQ ID NO: 12, or fragments of these sequences, as defined above.

According to a particular embodiment, a nucleic acid molecule of the invention comprises or consists essentially of or consists of a genomic sequence having at least 99% of identity with the genomic sequence of vB_KpM_SB4496_mtp5 represented by SEQ ID NO: 11 or the genomic sequence of vB_KpM_SB4975_mtp7 represented by SEQ ID NO: 12.

According to a particular embodiment, nucleic acid molecule of the invention is a recombinant nucleic acid molecule, and/or is an isolated nucleic acid molecule, and/or is a nucleic acid molecule that differs from known nucleic acid molecule, especially natural nucleic acid molecule, by at least 1, 2 or 3 nucleotide substitutions or deletions within the nucleic acid molecule, in particular differ from SEQ ID NO: 11 or SEQ ID NO: 12, by at least 1, 2 or 3 nucleotide substitutions or deletions within the nucleic acid molecule.

The invention also relates to a composition of at least one bacteriophage of the invention according to any embodiment described herein, or of at least one of pharmaceutical composition of the invention according to any embodiment described herein, comprising the same, on one hand (the first active ingredient), and at least another bacteriophage or a pharmaceutical composition comprising it on the second hand (the second and possibly further active ingredient(s) depending upon the number of added bacteriophage(s)). Examples of "compositions" including when separate administration are contemplated, are provided in the Experimental section herein.

By "another bacteriophage" in this context, it is meant any bacteriophage especially a bacteriophage that is highly specific for capsular *Klebsiella pneumoniae* types as discussed herein and is accordingly referred to as "anti-K phage" in present description. Accordingly, such a bacteriophage may be, in a non-limitative manner, a bacteriophage targeting a capsulated Kp.

Examples are provided in length in the present disclosure, and the effects of such compositions (also referred to as "cocktails" in the Experimental section and herein) are exemplified herein. As described herein, such compositions can have an increased killing effect on *Klebsiella pneumoniae* bacteria wherein the bacteria include non-capsular *Klebsiella pneumoniae* and capsular *Klebsiella pneumoniae,* with a slow regrowth of escape mutants. It is plausible that such effects can be attained because in *vitro* lysis tests normally show that the use of cocktails (mix of more than one phage) increases the efficiency of the phage "treatment", see https:// doi. org/10.1128/mBio.02530-19 or Yang Y, Shen W, Zhong Q, Chen Q, He X, Baker JL, Xiong K, Jin X, Wang J, Hu F and Le S (2020) Development of a Bacteriophage Cocktail to Constrain the Emergence of Phage-Resistant Pseudomonas aeruginosa. Front. Microbiol. 11:327. doi: 10.3389/fmicb.2020.00327 or Abedon ST, Danis-Wlodarczyk KM, Wozniak DJ. Phage Cocktail Development for Bacteriophage Therapy: Toward Improving Spectrum of Activity Breadth and Depth. Pharmaceuticals (Basel). 2021 Oct 3;14(10):1019. doi: 10.3390/ph14101019. PMID: 34681243; PMCID: PMC8541335.

Accordingly, a "composition" can either consist of the association/combination of the bacteriophages as active principles referred to above (first, second and possibly further active ingredients) as separate ingredients (i.e. a kit of active ingredients of admixture containing them), or consists of a composition comprising the active principles referred to above in a single composition. One or several active ingredients of compositions containing them may still be found within separate compositions.

According to another aspect, the bacteriophages described herein, the composition, in particular the pharmaceutical compositions described herein, according to all embodiments and combinations thereof, are for use as a medicament.

Within the present description, the "for use" feature will be used in order to refer to the use intended for a method of treatment of a patient in need thereof, comprising administering to the patient a therapeutically effective amount of the bacteriophages or the compositions of the present invention.

According to a particular embodiment, the said medicament is against an infection caused by at least one *Klebsiella pneumoniae* (Kp) strain, in particular where the infection is a nosocomial infection and in particular is an hospital-acquired nosocomial infection.

The *Klebsiella pneumoniae* (Kp) strain can be a capsulated or a non-capsulated strain. According to a particular embodiment, the *Klebsiella pneumoniae* (Kp) strain is a capsulated strain. According to a particular embodiment, the *Klebsiella pneumoniae* (Kp) strain is a non-capsulated strain (K^{d}). Of note, the *Klebsiella pneumoniae* are naturally capsulated *in vitro,* but it may not systematically be the case *in vivo,* or at least not under all circumstances.

According to a particular embodiment, the bacteriophages or the compositions comprising the bacteriophages for use in the treatment of a bacterial infection in a patient or an animal is administered in the early steps of the infection, typically when non-capsulated *Klebsiella pneumoniae* are known or are likely to represent a minority of the population of infectious bacteria. This may prevent the development of capsule-deficient *Klebsiella pneumoniae* in the population of bacteria.

According to another embodiment, at the early stage of the infection, a composition comprising a combination of the bacteriophages of the invention (anti-K^{d} bacteriophages) and anti-K bacteriophages can alternatively be administered to target every type of *Klebsiella pneumoniae.*

According to a particular embodiment, the infection caused by the said at least one *Klebsiella pneumoniae* (Kp) strain is selected from the group consisting of: cystic fibrosis, otitis media, keratitis, endophthalmitis, bacteremia, burn wound infection, pulmonary infection, pneumoniae, meningitis, peritonitis, urinary tract infection, blood infection or sepsis, more preferably pulmonary, pneumoniae, urinary tract infections, meningitis, peritonitis, blood infection or sepsis, and most preferably pulmonary, pneumonia, urinary tract infection, blood infection or sepsis.

According to a particular embodiment, the infection affects a patient selected among the group consisting of: immunocompromised patients and/or seriously ill patients in hospitals, intensive care units, or organ transplant centers. Typically, the present invention can be suited for the treatment of nosocomial infections and in particular, hospital-acquired nosocomial infections.

According to a particular embodiment, "treating an infection" or "therapeutic treatment" refer to both preventive treatment, defined as a decolonization of an asymptomatic patient before an infection that can be clinically observed expands (e.g., use of bacteriophages to decolonize a patient before a surgical operation or the like) as well as a curative or disease/infection modifying treatment, including treatment of subjects at risk of contracting an infection (i.e., asymptomatically colonized patient) or suspected to have contracted an infection as well as subjects who are ill or have been diagnosed as suffering from an infection.

The literature comprehensively provides guidance regarding preferred administration modes, or dosages, or protocols, to which the skilled person can refer. See for instance European regulatory aspects of phage therapy: magistral phage preparations, Gilbert Verbeken, Jean-Paul Pirnay, Current Opinion in Virology, Volume 52, 2022, Pages 24-29, ISSN 1879-6257, https:// doi.org/10.1016/j.coviro.2021.11.005.

The best phage administration routes are the ones that bring the phage in contact with the bacteria as fastest as possible. So for local bone, muscle infections, the route generally used is directly on the cavity(ies), directly in contact with the bone and surrounding tissues, see Gibb BP, Hadjiargyrou M. Bacteriophage therapy for bone and joint infections. Bone Joint J. 2021 Feb;103-B(2):234-244. doi: 10.1302/0301-620X.103B2.BJJ-2020-0452.R2. PMID: 33517726; PMCID: PMC7954149 or Ferry T, Boucher F, Fevre C, Perpoint T, Chateau J, Petitjean C, Josse J, Chidiac C, L'hostis G, Leboucher G, Laurent F; Lyon Bone and Joint Infection Study Group. Innovations for the treatment of a complex bone and joint infection due to XDR Pseudomonas aeruginosa including local application of a selected cocktail of bacteriophages. J Antimicrob Chemother. 2018 Oct 1;73(10):2901-2903. doi: 10.1093/jac/dky263. PMID: 30060002. For difficult access infections the phage composition, in particular under the form of a purified solution, needs to be delivered intravenously, see LaVergne S, Hamilton T, Biswas B, Kumaraswamy M, Schooley RT, Wooten D. Phage Therapy for a Multidrug-Resistant Acinetobacter baumannii Craniectomy Site Infection. Open Forum Infect Dis. 2018 Mar 23;5(4):ofy064. doi: 10.1093/ofid/ofy064. PMID: 29687015; PMCID: PMC5905571. To target gut colonization the administration routes are oral or rectal phage delivery, see Mario Corbellino, Nicolas Kieffer, Mzia Kutateladze, Nana Balarjishvili, Lika Leshkasheli, Lia Askilashvili, George Tsertsvadze, Sara Giordana Rimoldi, Deia Nizharadze, Naomi Hoyle, Lia Nadareishvili, Spinello Antinori, Cristina Pagani, Daniele Giuseppe Scorza, Ai Ling Loredana Romanò, Sandro Ardizzone, Piergiorgio Danelli, Maria Rita Gismondo, Massimo Galli, Patrice Nordmann, Laurent Poirel, Eradication of a Multidrug-Resistant, Carbapenemase-Producing Klebsiella pneumoniae Isolate Following Oral and Intra-rectal Therapy With a Custom Made, Lytic Bacteriophage Preparation, Clinical Infectious Diseases, Volume 70, Issue 9, 1 May 2020, Pages 1998-2001, https:// doi.org/10.1093/cid/ciz782.

According to a particular embodiment, it is also meant by "treating an infection" or "therapeutic treatment", protecting the subject from more severe consequences, on its health status, of the treated infection, compared to the consequences that would arise in the absence of treatment. This includes eliminating or lowering or alleviating the symptoms associated with the infection.

According to a particular embodiment, it is meant by "treating an infection" or "therapeutic treatment", the fact of abolishing, or preventing, or decreasing the mortality associated with the infection(s) by above-mentioned bacteria, in an extent that the odds of survival to the infection(s) are increased. According to a particular embodiment, a treatment according to the invention comes with 40%, or 50%, or 60%, or 70% reduction of the mortality rate for the treated subject.

All features described herein, in any embodiment of the present description, with respect to "compositions" of active ingredients also apply and can be relied upon in the context of therapeutic treatment, i.e., for example dosages, formulation, administration regimen, non-exhaustively.

Said differently, the invention also relates to a combination of active ingredients comprising at least one bacteriophage of the invention according to any embodiment described herein (first active ingredient) and at least another bacteriophage on the second hand (the second and possibly further active ingredient(s) depending upon the number of added bacteriophage(s)), for use in treating an infection as described herein (kit-of-parts),
wherein the active ingredients are administered as a single composition or as separate active ingredients, in particular in the same administration step, and
wherein optionally the active ingredients are, within their respective compositions if any, either free of or associated with pharmaceutically acceptable vehicle(s) or carrier(s).

The invention also relates to a method for the treatment of an infection as defined herein, and/or for the treatment or lowering of symptoms thereof and/or complications associated therewith, as defined herein, in a subject in need thereof, as defined herein, the method comprising administering to a subject active ingredient(s) as defined herein, especially a therapeutically effective amount of the same, according to any one of the embodiments disclosed herein, and all possible combinations thereof. Instant description makes use of the "for use" wording for defining therapeutic applications. Throughout the description, a wording using the expression "method of" can be alternatively used without the intended meaning being different.

The invention also relates to the use of an active ingredient according to any one of the embodiments described herein, for the preparation of a medicament having the therapeutic purpose(s) or effect(s) described herein. Reference is made to any one of the embodiments described herein, and all combinations thereof, with respect to purpose(s) of said medicament.

The invention also relates to a method for decreasing the bacterial load of at least one *Klebsiella pneumoniae* (Kp) strain in the environment, comprising putting the environment in contact with an effective amount of a bacteriophage of the invention according to any embodiment described herein, or a composition as described in any embodiment directed to a pharmaceutical composition described herein, or a composition or combination as described in any embodiment of the invention described herein.

According to particular embodiment, "environment" indicated above and herein excludes the human and animal body(ies). The environment can be food or non-food contact equipment, surfaces, etc. in food processing plants and other food establishments, not only hospitals.

Indeed, the active ingredient(s) of the invention as described herein is(are) also particularly suitable for environmental applications. For example, environmental applications of bacteriophages in health care institutions are useful for equipment such as endoscopes and environments such as Intensive Care Units which may be potential sources of nosocomial infection by Kp strains, especially resistant ones, while being difficult or impossible to disinfect. Bacteriophages or compositions (cocktails) would be particularly useful in treating equipment or environments inhabited by Kp strain clones which may become resistant to commonly used disinfectants. Bacteriophage compositions used to disinfect inanimate objects, or the environment may be sprayed, painted, or poured, onto such objects or surfaces in aqueous solutions with bacteriophage titers ranging between 10⁷-10¹¹ PFU/ml. Alternatively, bacteriophages may be applied by aerosolizing agents that might include dry dispersants which would facilitate distribution of the bacteriophages into the environment. Finally, objects may be immersed in a solution containing bacteriophages of the invention. The optimal numbers and timing of applications of bacteriophage compositions remains to be determined and would be predicated by the exact usage of such products. The bacteriophages of the present invention can also be used for decontaminating food products. Accordingly, the invention also concerns the use of bacteriophages or compositions as described in any embodiment herein, for decontaminating food products (see http:// www.intralytix.com/index.php?page=prod). By decontaminating food products, it is meant that upon contacting bacteriophages or compositions as described herein with food products, control or elimination or significant reduction of the contamination of the food product by the target strain(s), is achieved.

Other examples and features of the invention will be apparent when reading the examples and the figures, which illustrate the experiments conducted by the inventors, in complement to the features and definitions given in the present description.

### Legend of the Figures

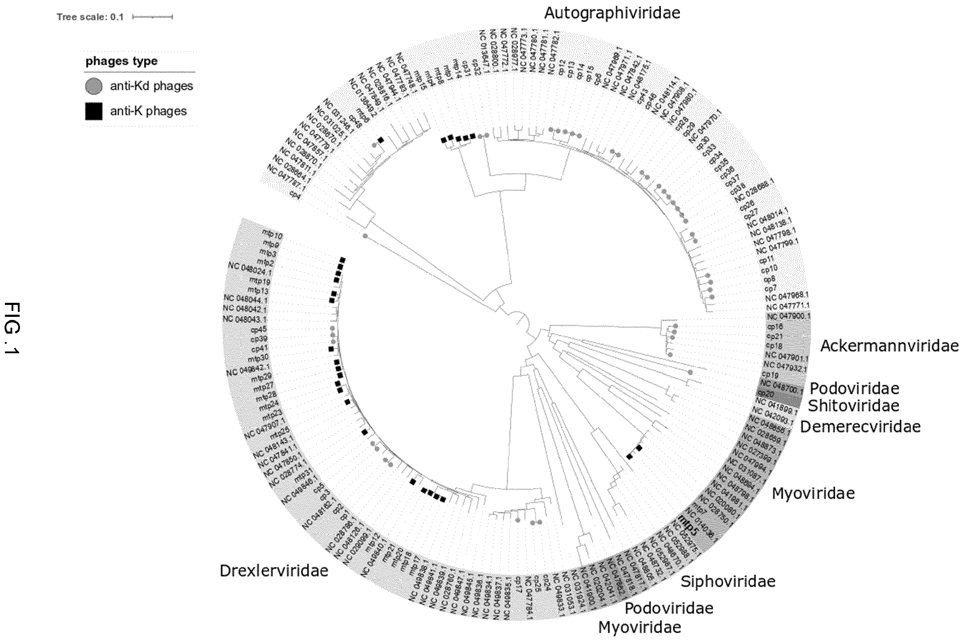

**Figure 1****.** Phylogenetic tree of phage genomes isolated and sequenced herein, and of 98 other anti-K. *pneumoniae* phage genomes (NCBI, March 2021). Shades overlaid on the name tags represent the phage families and the grey circles and black squares represent the phages isolated for this work: circles: anti-K^{d} phages, Squares: anti-K phages.

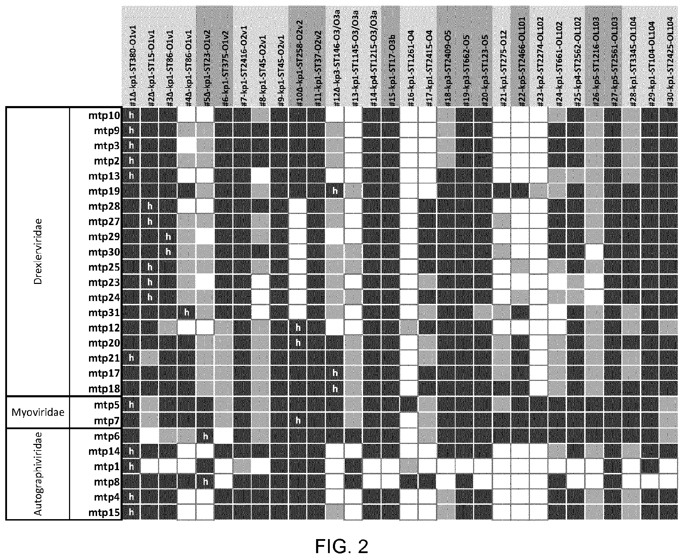

**Figure 2****.** Host range of anti-K^{d} phages. The phages were tested against capsule-deficient strains (n=7, *wza* or *wcaJ* mutants, represented by a Δ) and spontaneous non-mucoid culture-derived strains (n=23). Black: complete lysis; grey: intermediate lysis; white: absence of lysis. "h": initial host used for phage isolation.
**Figure 3****.** Efficacy of phage mtp5 on non-mucoid spontaneous mutants or in combination with anti-K phages. A. Efficiency of plating of phage mtp5 on the non-mucoid spontaneous mutants. Bacterial strains are ordered by O-type and the EOP was calculated has a ratio of phage titers relative to the host strain, using 1 as the reference value of the host. In darker orange is represented the strain used for mtp5 phage isolation. Only 24 out of 30 non-capsulated strains are represented. For the remaining six, we were not able to calculate the infection titers, although we could still see lysis on the highest phage concentrations. **B.** Association of mtp5 with anti-K phages in cocktails against capsulated strains. Growth curves for Kp strains with anti-K phages in the absence (blue) or presence (orange) of mtp5 (orange; equal proportions of each) (n=3 to 5 replicates for each condition). Phages were added at t=0 at MOI of 1 × 10-2. Grey curves are controls (no phage).

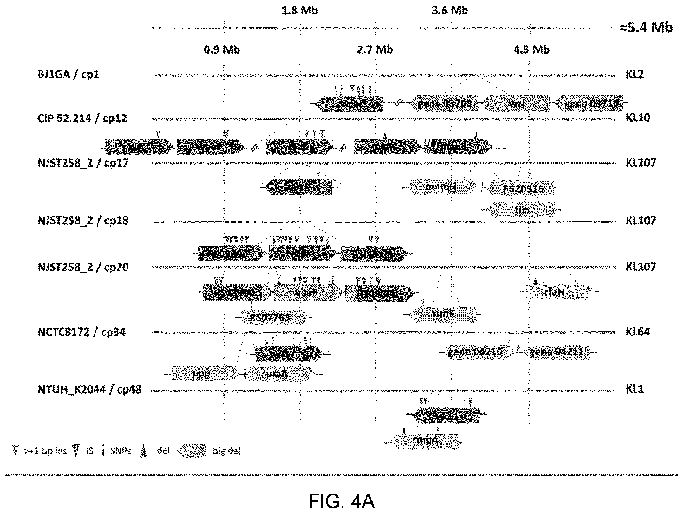

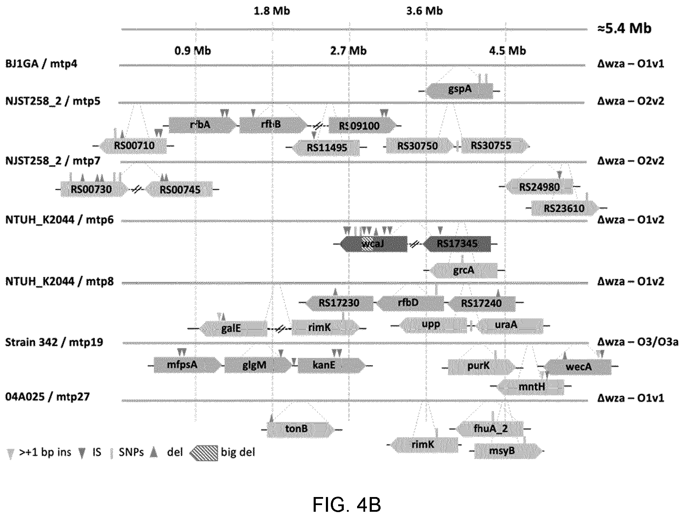

**Figure 4****.** Mutations observed in populations exposed to phages. A) Wild-type (WT) strains resistant to anti-K phages; B) capsule-deficient strains resistant to anti-K^{d} phages. Genes representation colors: dark=capsule locus or O-antigen locus or LPS locus and light: others.

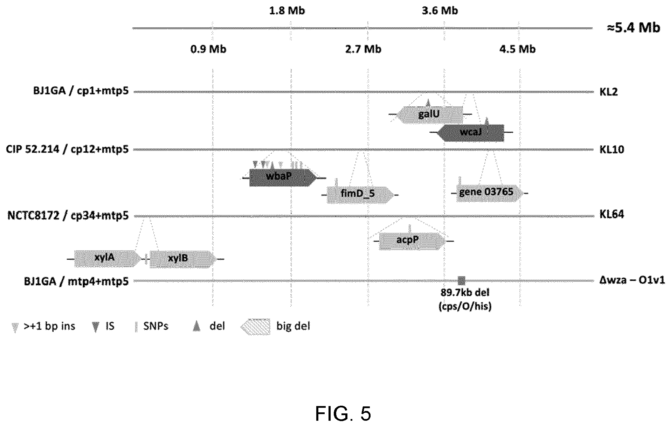

**Figure 5****.** Anti-K+mtp5 phage cocktails resistance mutations. Genes representation colors: dark=capsule locus or O-antigen locus or LPS locus and light: others.

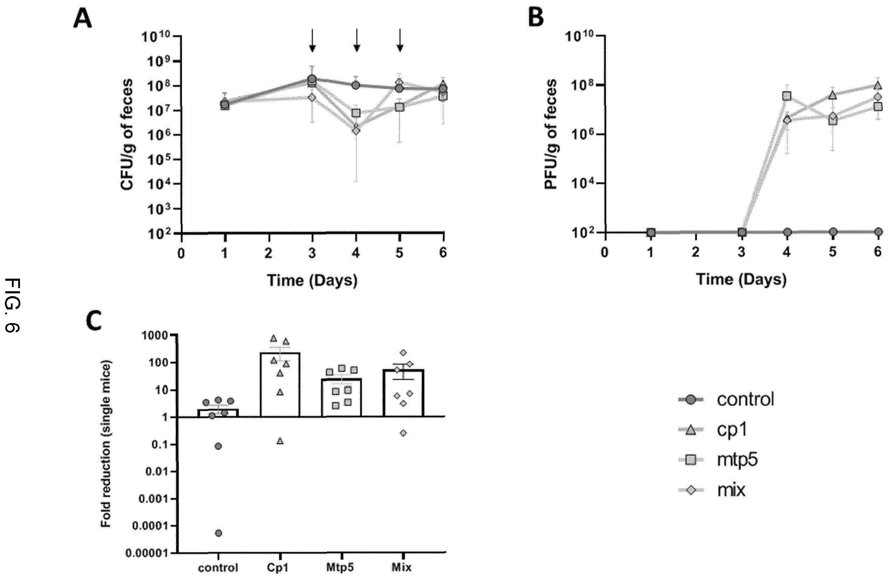

**Figure 6****.. Phage replication and coexistence with hypervirulent *K. pneumoniae* in the gut.** *K. pneumoniae* BJ1GA-colonized OMM12 mice (n=28) received by oral gavage at day 3, 4 and 5 either PBS (circles, n=7) or phages cp1 and mtp5, together (mix; diamond, n=7; 6×10⁷ pfu per dose made of the same amount of each phage) or individually (cp1: triangles, n=7; mtp5: squares, n=7). **A.** Levels of K. *pneumoniae* BJ1-GA in the feces (arrows represent the days phage was given to the mice). **B.** Phage titers from the fecal samples reported in panel A. **C.** Fold-reduction of the bacterial population levels one day after first treatment.

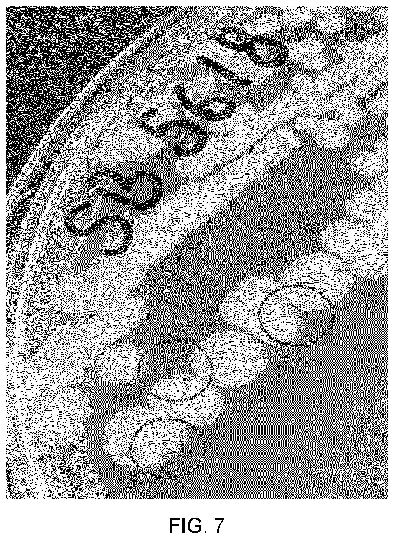

**Figure 7****.** Example of non-mucoid (NM) sectors
   Capsulated wild-type K. *pneumoniae* strains were streaked on TSA (Tryptic Soy Agar) and incubated for 24 h at 37°C. After incubation the TSA plate was scanned for non-mucoid (NM) sectors here depicted on the plate, and if nothing was detected, the plates were kept at 25°C until NM sectors can be observed (Chiarelli 2017).

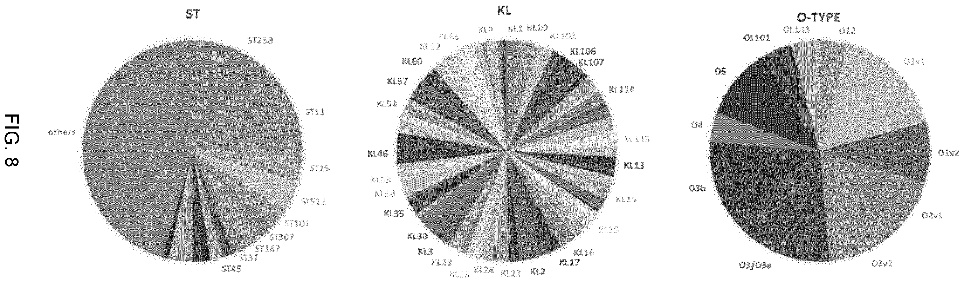

**Figure 8****.** Frequency of ST on the data set of 7388 genomes, KL- and O-antigen types in the deduplicated / non-redundant dataset of 7388 genomes.
   Analysis of the distribution of sequence-types (STs), capsule locus (KL) types and O-antigen types on a collection of 7,388 high quality *K. pneumoniae* species complex genomes publicly available. Based on the MLST, the most prevalent allelic profiles present in the dataset were ST258, ST11, ST15, ST512, ST101, ST307 and ST147. To eliminate bias due to recent clonal expansions or outbreaks, we analysed the prevalence of the KL and the O-antigen on a deduplicated sample where only one strain per ST was included.
**Figure 9****.** Small genomes have less hypothetical proteins.
   CDS number per phage with green representing proteins with known functions and red hypothetical proteins.
**Figure 10****.** Anti-K phages host-range
   Anti-K phages were tested against wild-type strains and the 7 capsule-deficient (Δ*wza* or Δ*wcaJ*) mutants. The filled squares represent complete lysis, shaded squares represent intermediate lysis and empty squares represent absence of lysis. "h" stand for the strain used as host for phage isolation.

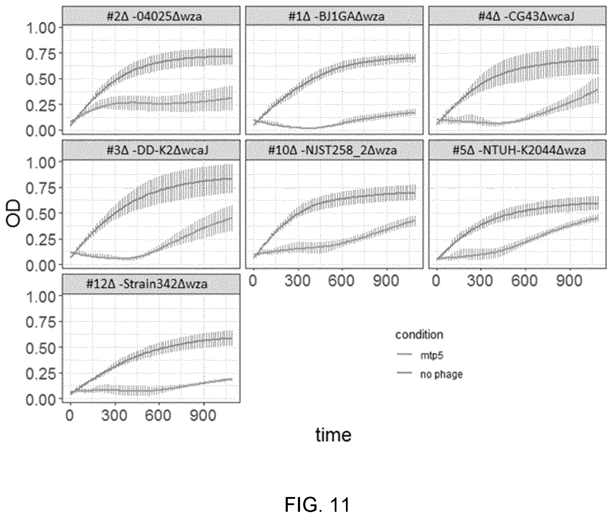

**Figure 11****.** Lysis kinetics of phage mtp5 on the 7 capsule-deficient mutant (Δ*wza* or Δ*wcaJ*) strains. Growth curves for different capsule-deficient K. *pneumoniae* species complex strains (n = 3 or more for each condition) measured via OD600nm reading in liquid broth in the absence (grey) or presence of phage mtp5 (orange) at t = 0 at multiplicity of infection (MOI) of 10² (error bars represent standard error of the mean, SEM).
**Figure 12****.** Genetic structure of anti-K^{d} phages mtp5 and mtp7 tail fibers genes and the closest anti-Klebsiella phages available in public databases.
   Mtp5 genome alignment with mtp7 the closest phage on our collection and the five closest phages previously described on the NCBI database (Kp15, Kp27, Matisse, Miro and PMBT1).

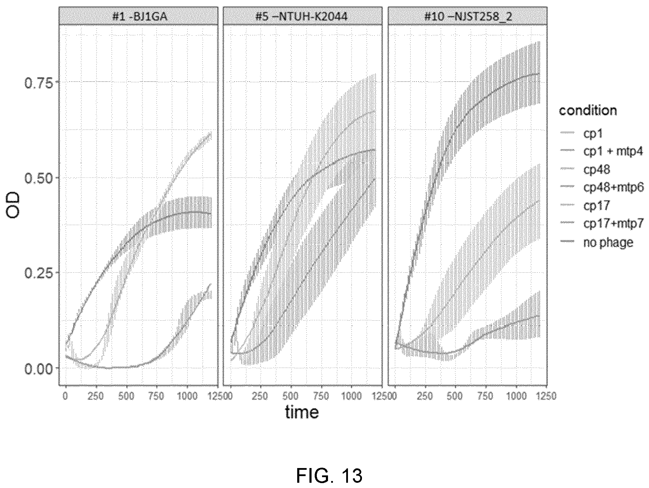

**Figure 13****.** Phage cocktails not including mtp5
   Growth curves (n = 3 or more for each condition) measured via OD600nm reading in liquid broth for 3 different strains in the present of three different phage cocktails respectively. Cocktails (orange) containing anti-K phage (blue) and anti-K^{d} phages otherthan mtp5 tested: (i) anti-K phage cp1 and anti-K^{d} phage mtp4 was tested on strain BJ1-GA; (ii) anti-K phage cp17 and anti-K^{d} phage mtp7 tested on strain NJST258_2; and (iii) anti-K phage cp48 and anti-K^{d} phage mtp6 tested against NTUH-K2044. Error bars represent standard error of the mean (SEM).

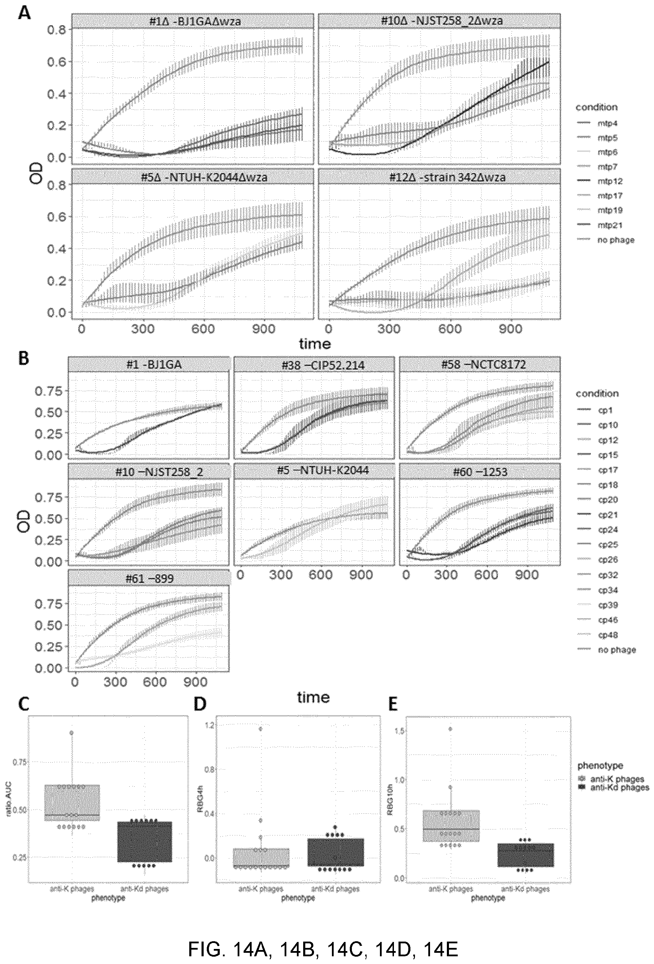

**Figure 14****.** Subpopulations of bacteria non-susceptible to phages emerge at different speeds depending on the presence/absence of capsule
   A. Growth curves for 3 Kp strains with or without capsule (n = 3 or more for each condition) measured via OD600nm reading in liquid broth in the absence (orange) or presence of A. anti-K^{d} phages and B. anti-K phages at t = 0 at multiplicity of infection (MOI) of 10² (error bars represent standard error of the mean, SEM). **C.** Area under the curve (AUC) and D. Relative bacterial growth (RBG) at 4h, **E.** 10h, graph with boxplots of the time of regrowth or relative bacterial growth on cultures targeted by anti-K phages vs anti-K^{d} phages (**P-value ≤ 0.01;***P-value ≤ 0.001, Mann-Whitney test)

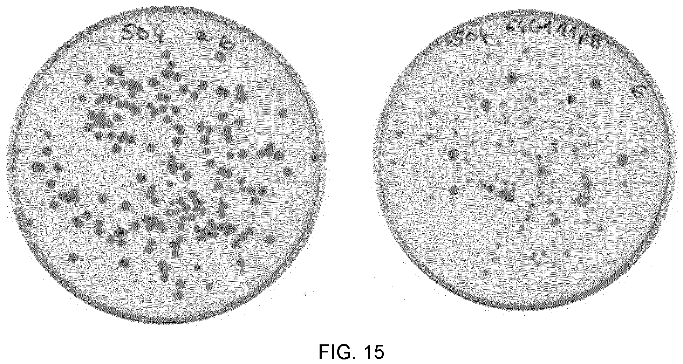

**Figure 15****.** Resistance to phage leads to capsule loss
   Example of inoculation of a control and a "non-susceptible culture of strain SB504 (against phage cp34).

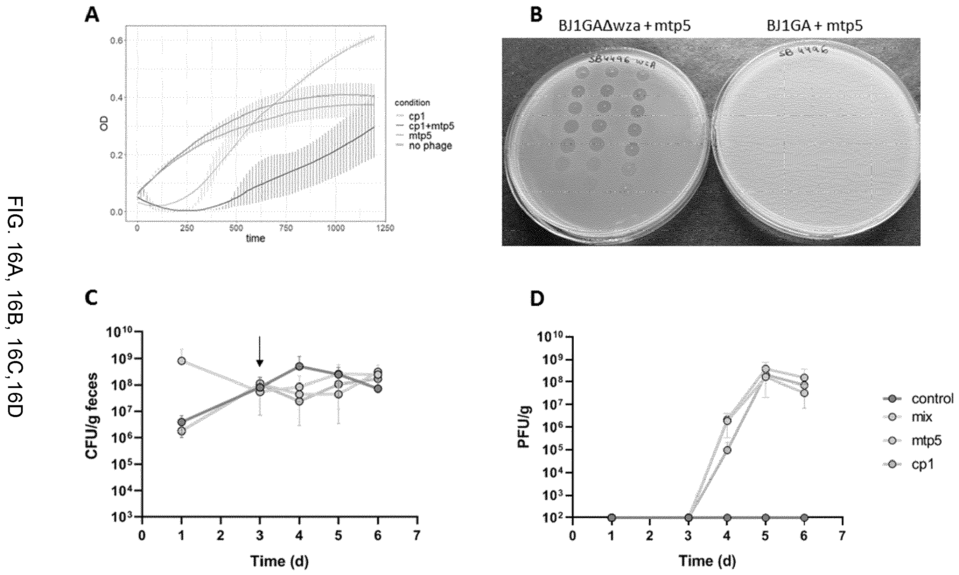

**Figure 16****.** A: Growth curves measured via OD600 nm reading in liquid broth for strain BJ1GA in the presence of phage cp1 or mtp5. **B:** Phage mtp5 does not infect strain SB4496 in *in vitro* solid media conditions. **C and D.** K. *pneumoniae-colonized* OMM12 mice (n=13) received at day3 PBS (pink, n=4), or the two phages cp1 and mtp5 together named mix (green, n=3; 6×10⁷ pfu per dose made of the same amount of each phage) or the individual phages cp1 (blue, n=3) and mtp5 (orange, n=3) by oral gavage. **C.** Levels of K. *pneumoniae* BJ1GA strain in the feces (arrow on the day phage is given to the mice). **D.** Phage titers from the fecal samples reported in panel C.

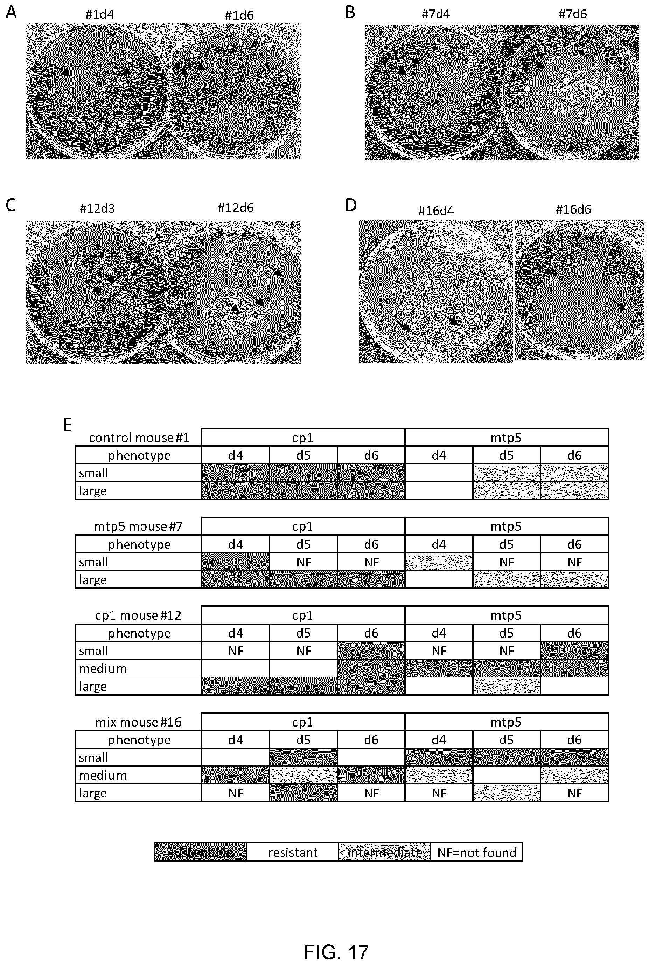

**Figure 17****.** A-D. Example of the phenotypes isolated from the feces of mice after K. pneumoniae colonization and phage treatment. A. Two K. pneumoniae phenotypes (small and large colonies) observed on SCAI medium agar from mouse #1 of the control group. B. example of the two phenotypes isolated at day 4 (first day with phage) from feces of mouse #7 treated with phage mtp5 (the small phenotype wasnot observed the following days, where only the large phenotype was observed . C. example of the three phenotypes isolated from feces of mouse #12 treated with phage cp1 (large, medium and small colony variants)). D. example of two of the three the phenotypes isolated from feces of mouse #16 treated with cocktail of cp1+mtp5. E. All clones were tested for resistance against the two original phages showing different susceptibility patterns.

### Material and methods

### Bacterial strains and phages

Bacterial strains used for phage isolation and host-range assays are readily available (7388 genomes total) . Strains were routinely cultured in lysogeny broth (LB), or on LB agar or Simmons Citrate agar with Inositol (SCAI) plates, at 37°C.
Phages isolated in this study are described in **Table 3.** Phages were amplified in exponential growing cultures of the respective host strain for approximately 4 to 5 hours (h). Cell lysate supernatants containing amplified phages were after 0.22 µm filter sterilized and stored at 4°C.

### Genomic background and surface structures analysis of publicly available K. pneumoniae species complex genomes for phage host strains selection

The *K. pneumoniae* species complex (KpSC) includes five species distributed among seven phylogroups: *K. pneumoniae* subsp. *pneumoniae* (Kp1), *K. quasipneumoniae* subsp. *quasipneumoniae* (Kp2), K. *variicola* subsp. *variicola* (Kp3), *K. quasipneumoniae* subsp. *similipneumoniae* (Kp4), K. *variicola* subsp. *tropica* (Kp5), *'K*. *quasivariicola'* (Kp6) and K. *africana* (Kp7) (Rodrigues et al. 2019). In total, we have previously collected 7,388 KpSC genomes from NCBI assembly database (GenBank) on March 2019 (Hennart et al. 2021). Comparative genomic analysis were performed using Kleborate v1.0 (Lam, Wick, Watts, et al. 2021), a tool designed for the genotyping of KpSC. Kleborate can extract from the genomes relevant information such as species assignation, the classical 7-gene MLST sequence type (ST), several chromosomal and plasmid associated virulence loci, antimicrobial resistance genes/mutations and predict (with a confidence score) the capsule type (KL) and O antigen (LPS) serotype (Lam, Wick, Watts, et al. 2021).

### Phage isolation

Based on the genotyping information collected, we selected a total of fourteen KpSC strains for phage isolation **(Table 1).** These included 7 wild-type (wt) strains (all Kp1) and 7 capsule-deficient mutants (6 Kp1 and 1 Kp3) (de Sousa et al. 2020) as hosts for isolation of anti-K phages and anti-K^{d} phages, respectively. Phage isolation was performed using water from the river Seine and from sewage, as previously described (Lourenço et al. 2020).

**Table 1. Host strains selected for phage isolation**

| **ID in this study** | **Strain ID** | **SB ID** | **Species (PhG)** | **Phenot ype** | **ST** | **KL-type** | **O-type** |
|---|---|---|---|---|---|---|---|
| **#1Δ** | BJ1-GA | SB4496 | *Klebsiella pneumoniae* (Kp1) | *Δwza* | ST380 | mutant (KL2) | O1v1 |
| **#2Δ** | 04A025 | SB20 | *Klebsiella pneumoniae* (Kp1) | *Δwza* | ST15 | mutant (KL24) | O1v1 |
| **#3Δ** | SA1 | SB4021 | *Klebsiella pneumoniae* (Kp1) | *ΔwcaJ* | ST86 | mutant (KL2) | O1v1 |
| **#4Δ** | CG43 | SB4454 | *Klebsiella pneumoniae* (Kp1) | *ΔwcaJ* | ST86 | mutant (KL2) | O1v1 |
| **#5Δ** | NTUH_K 2044 | SB3928 | *Klebsiella pneumoniae* (Kp1) | *Δwza* | ST23 | mutant (KL1) | O1v2 |
| **#10Δ** | NJST258 _2 | SB4975 | *Klebsiella pneumoniae* (Kp1) | *Δwza* | ST258 | mutant (KL107) | O2v2 |
| **#12Δ** | 342 | SB579 | *Klebsiella variicola* subsp. *variicola* (Kp3) | *Δwza* | ST146 | mutant (KL30) | O3/O3a |
| **#5** | NTUH_K 2044 | SB3928 | *Klebsiella pneumoniae* (Kp1) | wt | ST23 | KL1 | O1v2 |
| **#10** | NJST258 _2 | SB4975 | *Klebsiella pneumoniae* (Kp1) | wt | ST258 | KL107 | O2v2 |
| **#31** | CIP 52.145 | SB3341 | *Klebsiella pneumoniae* (Kp1) | wt | ST66 | KL2 | O1v2 |
| **#36** | CIP 52.214 | SB3245 | *Klebsiella pneumoniae* (Kp1) | wt | ST297 | KL10 | O1v1 |
| **#54** | NCTC81 72 | SB504 | *Klebsiella pneumoniae* (Kp1) | wt | ST505 | KL64 | O1v1 |
| **#56** | 1253 | SB5521 | *Klebsiella pneumoniae* (Kp1) | wt | ST307 | KL102 | O2v2 |
| **#57** | 899 | SB5442 | *Klebsiella pneumoniae* (Kp1) | wt | ST101 | KL106 | O1v2 |

| | | | | | | | |
|---|---|---|---|---|---|---|---|
| ID, identification; SB, strain bank; PhG, phylogroup; ST, Sequence-Type; KL, capsular locus; O, O-antigen locus; Δ, mutants; wt, wild-type. | | | | | | | |

### Phage host range tests

Host range tests were performed as follows: 3µL of phosphate-buffered saline (PBS)-diluted phage solutions (0.22 µm filter sterilized crude lysates adjusted to 10⁷ PFU/mL) were deposited side by side on the lawn of each tested bacterium on square LB agar plates. Plates were incubated at 37°C overnight (≈18 h).

Isolated phages were tested against 50 different KpSC strains, including 43 WT strains representing 32 different KL-types and the 7 capsule-deficient mutants (de Sousa et al. 2020); and against 16 strains from other *Enterobacteriaceae* species (9 *Escherichia coli,* 6 *Salmonella* spp. and 1 *Citrobacter*spp.). Additionally, the anti-K^{d} phages were also tested against 23 KpSC capsule-deficient clones (generated as explained in methods below) (Chiarelli et al. 2020).

### Generation of non-mucoid K. pneumoniae strains (capsule-deficient clones)

Following Chiarelli *et al* (Chiarelli et al. 2020), 23 WT KpSC strains were streaked on Tryptic Soy Agar (TSA) and incubated at 37°C for 24 h. Then, the TSA plate was scanned for non-mucoid (NM) sectors **(****Figure 7****),** and if nothing was detected, the plates were kept at 25°C until NM sectors can be observed. The sectors were then isolated, plated and kept at 37°C for other 24h, and then left at 25°C to verify if all colonies were homogenous. If homogenous, two other steps of purification were performed before storage at -80°C.

### Phage efficiency of plating (EOP) test

The efficiency of plating (EOP) was calculated (ratio of the number of plaques formed by the phage on each strain tested and the number of plaques formed on the specific host strain) for each phage. Three/four independent replicates were performed using bacterial cultures grown to an OD of approximately 0.2 at 600nm and spread on LB plates onto which phage dilutions were spotted. Plates were incubated at 37°C overnight.

### Phage genome sequencing and analysis

Sterile phage lysates [obtained by a 5 h phage amplification, followed by centrifugation (10 min, 5000 rpm) and 0.22 µm filter sterilization], were treated with DNase (120 U) and RNase (240 mg/mL) and incubated for 30 min at 37°C before adding EDTA (20 mM). Lysates were then treated with proteinase K (100 mg/mL) and SDS (0.5%) and incubated at 55°C for 30 min. DNA was extracted by a phenol-chloroform protocol modified from Pickard 2009 (Pickard, 2009). Genomic DNA libraries were prepared with TruSeq DNA PCR-Free sample preparation kit (Illumina Inc., San Diego, USA) and 2×150 paired-end sequencing was performed using the NextSeq 500/550 Illumina technology (Illumina, San Diego, USA). The quality of the reads was checked with fastqc v0.8.5 (Cokelaer et al. 2017), and reads were cleaned using fqcleaner pipeline from Galaxy-Institut Pasteur (https://gitlab.pasteur.fr/GIPhy/fqCleanER). *De novo* assembly was performed using Spades (3.11.0) (Prjibelski et al. 2020), or a workflow implemented in Galaxy-Institut Pasteur using clc_assembler v4.4.2 and clc_mapper v4.4.2 (CLC Bio, Qiagen) when necessary. Phage termini were determined by PhageTerm 2.0.1 (Garneau et al. 2017) and annotations performed using PATRIC RASTtk (Brettin et al. 2015; Aziz et al. 2008). Phage lifestyles were accessed using BACPHLIP, a python library for predicting phage lifestyle based on genome sequence (Hockenberry and Wilke 2021).

Phylogenetic trees were generated using JolyTree v2.0 (MASH based; parameters: sketch size of 100000, probability of observing a random k-mer of 0.00001 and k-mer size of 15) (Criscuolo 2019), and visualized using iTol (Letunic and Bork 2019). In order to understand the distribution of the phages isolated in our study in the global population of phages isolated against Kp, we performed a phylogenetic analysis including our phage genomes and 98 publicly available genomes deposited on the refseq NCBI database, with Kp (taxid: 573) stated as host (March 2021).

### Depolymerases analysis

To detect the different polymerases present on our newly isolated phages we performed an initial HMMER (v3.3) comparative analysis using 14 HMM profiles associated with bacteriophage-encoded depolymerases (de Sousa et al. 2020) (filtered by the e-value of the best domain maximum 10⁻³). A Blastp (v2.6.0, default parameters filtering by e-value (maximum 10⁻⁵) and identity (30%)) search was also performed for specific protein sequences described and validated for broad-range Klebsiella phages described in the literature (Pan et al. 2017).

### Growth curves/lysis kinetics analysis

To record phage replication and bacterial lysis, an overnight culture of the respective KpSC strain was diluted in LB Lennox media and grown to an OD at 600 nm of ≈0.2, from which 140 µL were distributed on a 96- well plate (Microtest 96 plates, Falcon). Afterwards were added 10 µL of sterile phage lysates, previously diluted in PBS, to obtain a multiplicity of infection (MOI) of 1 × 10⁻² in each well. Plates were incubated in a microplate reader at 37°C, with a shaking step of 30 pmidbefore the automatic recording of OD at 600 nm every 15 min over 18h-20 h (Tecan microplate reader). The Area Under the Curve (AUC) was calculated in R using the function trapz from the pracma package (v2.3.3). The Relative Bacterial Growth (RBG) was calculated using the following equation: RBG = [Abs600 (t = 4 h) -Abs600 (t = 0 h)]bp/[Abs600 (t = 4 h) - Abs600 (t = 0 h)]b, in which Abs600 stands for the absorbance at 600 nm of bacterial cultures, b for bacteria only (control cultures), bp for bacteria with phage (infected cultures) and t for time in hours (h). The RBG was performed for 6 WT strains and 4 capsule-deficient mutants exposed to 3 individual phages (exception of SB3928 that was only exposed to 2 phages). RBG was calculated at 4, 6, 8 and 10 hours (Majkowska-Skrobek et al. 2021). Mann-Whitney-Wilcoxon test was performed for AUC and RBG results comparisons using R.

### Isolation and test of possible phage resistant clones

After 20 h kinetics, the cultures were plated in LB Lennox agar to select possible phage resistant clones and uncover the specific receptors of some of our phages, and simultaneously study the function of the mutated genes and their relevance for Kp infection, colonization or even antibiotic resistance. After ≈18 h at 37°C, colonies were counted and checked for phenotypic differences. In total, 24 colonies from the control cultures and 50 colonies from the infected cultures were isolated and grown in 96-well plates with LB Lennox plus 16% glycerol for 24 h at 37°C and stored at -80°C. Afterwards a fresh overnight culture of each clone (ie each isolated colony) was grown at 37°C and refreshed in LB liquid media the following day and grown at 37°C to an OD of approximately 0.5 (600nm) and a double spot test (6 µL bacterial culture + 3 µL phage) was performed in order to test for resistance.

Later, a fresh culture of 1 mL of each clone (ie each isolated colony) was grown for 24 h at 37°C and then all cultures were pooled together and centrifuged. This pelleted population was then diluted in 1 mL of PBS. 500 µL of the sample were used for DNA extraction using the Maxwell Cell tissue kit (Promega) and subject to Illumina sequencing, as described above.

For some cultures, the detection of a very small colony variant phenotype led to problems in isolation. In such cases, DNA extraction was performed directly from colonies collected from the plate without a previous resistance test.

Initial phenotypic observations (colonies on LB plates) regarding the anti-K phages appear to show a loss of capsule within the population 20 h post-infection (colonies were tested from samples in the presence and absence of phage). Colonies with the different phenotypes were counted and percentages were taken into consideration before isolation for the resistance assay, and subsequently DNA extraction and sequencing.

### Murine model of K. pneumoniae colonization

*In vivo* assays were performed using the OligoMM¹² mice model. This mice model harbours a consortium of 12 different bacterial strains in the gut: *Acutalibacter muris, Akkermansia muciniphila, Bacteroides caecimuris, Bifidobacterium longum subsp animalis, Blautia coccoides, Clostridium clostridioforme, Clostridium innocuum, Enterococcus faecalis, Flavonifractor plautii, Lactobacillus reuteri, Muribaculum intestinale and Turicimonas muris* (Brugiroux et al. 2016). Two independent experiments with repeated phage administration were performed with n= 3-4 mice per group. At day 0, mice were orally colonized with Kp1 BJ1-GA (SB4496). Initial inoculum was prepared by culturing bacteria overnight at 37°C in LB broth with 25 mg/mL chloramphenicol. Subsequently, the culture was diluted 1:25 in fresh LB medium, and subcultured for 4 h at 37°C. Bacteria were resuspended in 10 mL phosphate-buffered saline (PBS) and adjusted to 5×10⁸ CFUs/ml. Mice were orally inoculated with 200 µL of bacterial suspension. At day 3,4 and 5 mice received the two phages alone (adjusted to 10⁸ PFU), or in combination (1:1 mixture of each phage in 200 µL of PBS) once daily by oral gavage.

The level of phages was assessed from serial dilutions in PBS spotted on LB plates overlaid with strain BJ1-GA. Weight of the mice was monitored during the course of the experiment, and feces were collected at different time points after colonization (day 1,3, 4,5 and 6).

Subsequently, fecal samples were diluted in 1 mL PBS and homogenized by bead-beating with 1 mm zirconia/silica beads for two times 25 s using a Mini-Beadbeater-96 (BioSpec). To determine CFUs, serial dilutions of homogenized samples were plated on LB plates with 25 mg/mL chloramphenicol. To determine PFUs, serial dilution of homogenized samples were plated on LB plates overlaid with strain BJ1-GA. Plates were cultured at 37°C over night before counting. CFUs of Kp1 BJ1-GA and PFUs of the phages were calculated after normalization to the weight of feces.

### Animals and ethics

Oligo-MM12 C57BL/6NTac mice were bred in isolators (Getinge) in the germfree facility at the HZI. Animals used in experiments were gender and age matched. Female and male mice with an age of 8-12 weeks were used. Sterilized food and water ad libitum was provided. Mice were kept under strict 12-hour light cycle (lights on at 7:00 am and off at 7:00 pm) and housed in groups of 3-4 mice per cage. All mice were euthanized by asphyxiation with CO2 and cervical dislocation. All animal experiments have been performed in agreement with the guidelines of the Helmholtz Center for InfectionResearch, Braunschweig, Germany; the National Animal Protection Law [Tierschutzgesetz (TierSchG) and Animal Experiment Regulations (Tierschutz-Versuchstierverordnung (TierSchVersV)], and the recommendations ofthe Federation of European Laboratory Animal Science Association (FELASA). The study was approved by the Lower Saxony State Office for Nature, Environment and Consumer Protection (LAVES), Oldenburg, Lower Saxony, Germany; permit No. 33.8-42502-04-20/3564.

### Statistical Analysis

For the growth curves of the different strains exposed or not to phage (n=>3 for each condition) error bars represent standard error of the mean (SEM). Regarding Area under the curve (AUC) and relative bacterial growth (RBG) a Mann-Whitney test was performed (**P-value ≤ 0.01;***P-value ≤ 0.001).

Statistical analysis from *in vivo* mice experiments bacterial levels, were carried out using the Ime4, ImerTest and car packages of R (Bates et al. 2014; Fox and Weisberg 2019; Kuznetsova, Brockhoff, and Christensen 2017). CFU numbers were log10-transformed prior to analysis. In each experiment, four groups of mice were considered, three groups exposed to the different phages (cp1, mtp5, mix) and a group unexposed control group. The impact of phages could be assessed based on the abundance of phages (log-PFU). Given the non-linearity of responses, the day at which a measure was performed was considered as a categorical variable. Linear mixed-models were used to account for random experimental effects (i.e., individuals and experiments effects). Overall effects were assessed with Analysis of Variance (ANOVA) and post-hoc Tukey's comparisons and were performed using the Ismeans R package (Lenth 2016). Only p< 0.05 was considered statistically significant.

### Additional tables

**Table 5. Depolymerases**

| Sequence | Description | Cut-offs | Reference |
|---|---|---|---|
| YP_009226010.1 | Tail fiber protein | e-value maximum le-5, minimum identity 30% | (Majkowska-Skrobek et al 2016) |
| BAP15746.1 | Hypothetical protein | | (Lin et al 2014) |
| BAQ02780.1 | Tail fiber | | (Pan et al 2015) |
| S1-1 (YP_002003830.1) | Tail spike (gp17) | | (Pan et al 2017)PMID: 28077636 |
| S1-2 (YP_008532048.1) | tail spike protein head-binding protein | | |
| S1-3 (AGF88658.1) | tailspike protein | | |
| S2-1 (YP_007010682.1) | putative tail fiber protein | | |
| S2-2 (WP_020326882.1) | phage T7 tail fiber protein | | |
| S2-3 (YP_654147.1) | K5 lyase | | |
| S2-4 (YP_007003187.1) | tail fiber protein | | |
| S2-5 (YP_398994.1) | putative tail fiber | | |
| S2-6 (WP_020801644.1) | right-handed parallel beta-helix repeat-containing protein | | |
| S2-8 (YP_006987359.1) | putative tail fiber protein | | |

**Table 6. Phage cocktails tested**

| **Strain ID** | **anti-K phage** | **anti-Kd phage** |
|---|---|---|
| SB4496 | cp1 | mtp5 |
| SB5521 | cp21 | mtp5 |
| SB3245 | cp12 | mtp5 |
| SB3928 | cp48 | mtp5 |
| SB504 | cp34 | mtp5 |
| SB4975 | cp17 | mtp5 |
| SB5442 | cp39 | mtp5 |
| SB4496Δwza | - | mtp5+mtp4 |
| SB4975 | cp17 | mtp7 |
| SB3928 | cp48 | mtp6 |
| SB4496 | cp1 | mtp4 |

**Table 7. Statistics-1day phage**

| ANOVA | | | |
|---|---|---|---|
| day: testing difference between the different days | | | |
| phage: effect of one day phage exposure | | | |
| Analysis of Deviance Table (Type II Wald chisquare tests) | | | |
| Response: scale(log10(CFU.g + 1)) | | | |
| | Chisq | Df | Pr(>Chisq) |
| day | 36,9354 | 4 | 1,86E-07 |
| phage | 0,5408 | 3 | 0,90985 |
| day:phage | 20,1356 | 12 | 0,06456 |

| | | | |
|---|---|---|---|
| Post-hoc tests (tukey) Comparison between "phage" and "no phage" at the different time points Degrees-of-freedom method: kenward-roger Results are given on the scale(6.62, 1.07) (not the response) scale. Confidence level used: 0.95 | | | |

### $contrasts

**day = 1:**

| contrast | estimate | SE | df | t.ratio | p.value |
|---|---|---|---|---|---|
| control - cp1 | -0,6579 | 0,519 | 78,4 | -1,268 | 0,5857 |
| control - mix | -0,5375 | 0,519 | 78,4 | -1,036 | 0,7288 |
| control - mtp5 | -0,3465 | 0,519 | 78,4 | -0,668 | 0,9089 |
| cp1 - mix | 0,1204 | 0,498 | 78,4 | 0,242 | 0,995 |
| cp1 - mtp5 | 0,3115 | 0,498 | 78,4 | 0,625 | 0,9238 |
| mix - mtp5 | 0,191 | 0,498 | 78,4 | 0,383 | 0,9807 |

**day = 3:**

| contrast | estimate | SE | df | t.ratio | p.value |
|---|---|---|---|---|---|
| control - cp1 | -0,4827 | 0,519 | 78,4 | -0,931 | 0,7886 |
| control - mix | 0,1673 | 0,519 | 78,4 | 0,322 | 0,9883 |
| control - mtp5 | -0,4251 | 0,519 | 78,4 | -0,819 | 0,8451 |
| cp1 - mix | 0,65 | 0,498 | 78,4 | 1,304 | 0,563 |
| cp1 - mtp5 | 0,0576 | 0,498 | 78,4 | 0,116 | 0,9994 |
| mix - mtp5 | -0,5924 | 0,498 | 78,4 | -1,189 | 0,6358 |

**day = 4:**

| contrast | estimate | SE | df | t.ratio | p.value |
|---|---|---|---|---|---|
| control - cp1 | 0,8902 | 0,519 | 78,4 | 1,716 | 0,3224 |
| control - mix | 1,0232 | 0,519 | 78,4 | 1,973 | 0,2072 |
| control - mtp5 | 0,4888 | 0,519 | 78,4 | 0,942 | 0,7822 |
| cp1 - mix | 0,1331 | 0,498 | 78,4 | 0,267 | 0,9933 |
| cp1 - mtp5 | -0,4013 | 0,498 | 78,4 | -0,805 | 0,8518 |
| mix - mtp5 | -0,5344 | 0,498 | 78,4 | -1,072 | 0,7073 |

**day = 5:**

| contrast | estimate | SE | df | t.ratio | p.value |
|---|---|---|---|---|---|
| control - cp1 | 0,548 | 0,519 | 78,4 | 1,056 | 0,7169 |
| control - mix | -0,1369 | 0,519 | 78,4 | -0,264 | 0,9935 |
| control - mtp5 | 0,5297 | 0,519 | 78,4 | 1,021 | 0,7377 |
| cp1 - mix | -0,6849 | 0,498 | 78,4 | -1,374 | 0,5191 |
| cp1 - mtp5 | -0,0183 | 0,498 | 78,4 | -0,037 | 1 |
| mix - mtp5 | 0,6666 | 0,498 | 78,4 | 1,337 | 0,5421 |

**day = 6:**

| contrast | estimate | SE | df | t.ratio | p.value |
|---|---|---|---|---|---|
| control - cp1 | 0,2577 | 0,54 | 84,1 | 0,477 | 0,9639 |
| control - mix | 0,8215 | 0,54 | 84,1 | 1,521 | 0,4297 |
| control - mtp5 | 0,5811 | 0,54 | 84,1 | 1,076 | 0,7052 |
| cp1 - mix | 0,5638 | 0,498 | 78,4 | 1,131 | 0,6715 |
| cp1 - mtp5 | 0,3233 | 0,498 | 78,4 | 0,649 | 0,9157 |
| mix - mtp5 | -0,2405 | 0,498 | 78,4 | -0,482 | 0,9628 |

| | | | | | |
|---|---|---|---|---|---|
| Note: contrasts are still on the scale(6.62, 1.07) scale Degrees-of-freedom method: kenward-roger P value adjustment: tukey method for comparing a family of 4 estimates | | | | | |

### Bacterial abundance (CFU) as a function of time (day) and exposure to phages.

Random effects include individual IDs and the two experiments performed. Overall analysis of variance (ANOVA) reveals significant effects of day (p=0.0000001857) but no significant effect was shown for phage (p=0.90985) and the interaction day:phage (p=0.06456).

The post-hoc Tukey comparisons displayed above were performed between mice exposed to the different phages (cpl, mtp5 and mix) and not exposed within each day.

**Table 8. Statistics-3day phage**

| ANOVA | | | |
|---|---|---|---|
| day: testing difference between the different days | | | |
| phage: effect of the continued phage exposure | | | |
| Analysis of Deviance Table (Type II Wald chisquare tests) | | | |
| Response: scale(log10(CFU.g + 1)) | | | |
| | Chisq | Df | Pr(>Chisq) |
| day | 26,3799 | 4 | 2,65E-05 |
| phage | 0,4244 | 3 | 0,93517 |
| day:phage | 23,4388 | 12 | 0,02422 |

| | | | |
|---|---|---|---|
| Post-hoc tests (tukey) Comparison between "phage" and "no phage" at the different time points Degrees-of-freedom method: kenward-roger Results are given on the scale(6.62, 1.1) (not the response) scale. Confidence level used: 0.95 | | | |

**day = 1:**

| contrast | estimate | SE | df | t.ratio | p.value |
|---|---|---|---|---|---|
| control - cp1 | -0,5369 | 0,503 | 85,6 | -1,068 | 0,7098 |
| control - mix | -0,4188 | 0,503 | 85,6 | -0,833 | 0,8386 |
| control - mtp5 | -0,2312 | 0,503 | 85,6 | -0,46 | 0,9675 |
| cp1 - mix | 0,1182 | 0,503 | 85,6 | 0,235 | 0,9954 |
| cp1 - mtp5 | 0,3057 | 0,503 | 85,6 | 0,608 | 0,9292 |
| mix - mtp5 | 0,1876 | 0,503 | 85,6 | 0,373 | 0,9822 |

**day = 3:**

| contrast | estimate | SE | df | t.ratio | p.value |
|---|---|---|---|---|---|
| control - cp1 | -0,8665 | 0,503 | 85,6 | -1,724 | 0,3179 |
| control - mix | -0,2288 | 0,503 | 85,6 | -0,455 | 0,9685 |
| control - mtp5 | -0,8104 | 0,503 | 85,6 | -1,612 | 0,3774 |
| cp1 - mix | 0,6377 | 0,503 | 85,6 | 1,268 | 0,5854 |
| cp1 - mtp5 | 0,0561 | 0,503 | 85,6 | 0,112 | 0,9995 |
| mix - mtp5 | -0,5816 | 0,503 | 85,6 | -1,157 | 0,6555 |

**day = 4:**

| contrast | estimate | SE | df | t.ratio | p.value |
|---|---|---|---|---|---|
| control - cp1 | 1,0569 | 0,503 | 85,6 | 2,102 | 0,1606 |
| control - mix | 1,1875 | 0,503 | 85,6 | 2,362 | 0,0923 |
| control - mtp5 | 0,6636 | 0,503 | 85,6 | 1,32 | 0,5529 |
| cp1 - mix | 0,1306 | 0,503 | 85,6 | 0,26 | 0,9938 |
| cp1 - mtp5 | -0,3933 | 0,503 | 85,6 | -0,782 | 0,8622 |
| mix - mtp5 | -0,524 | 0,503 | 85,6 | -1,042 | 0,7252 |

**day = 5:**

| contrast | estimate | SE | df | t.ratio | p.value |
|---|---|---|---|---|---|
| control - cp1 | 0,5194 | 0,521 | 89,8 | 0,998 | 0,7511 |
| control - mix | -0,1527 | 0,521 | 89,8 | -0,293 | 0,9912 |
| control - mtp5 | 0,5012 | 0,521 | 89,8 | 0,963 | 0,7709 |
| cp1 - mix | -0,6721 | 0,503 | 85,6 | -1,337 | 0,5423 |
| cp1 - mtp5 | -0,0182 | 0,503 | 85,6 | -0,036 | 1 |
| mix - mtp5 | 0,6539 | 0,503 | 85,61 | 1,301 | 0,5651 |

**day = 6:**

| contrast | estimate | SE | df | t.ratio | p.value |
|---|---|---|---|---|---|
| control - cp1 | 0,2286 | 0,544 | 95,2 | 0,42 | 0,975 |
| control - mix | 0,7815 | 0,544 | 95,2 | 1,435 | 0,4806 |
| control - mtp5 | 0,5458 | 0,544 | 95,2 | 1,002 | 0,7483 |
| cp1 - mix | 0,553 | 0,503 | 85,6 | 1,1 | 0,6905 |
| cp1 - mtp5 | 0,3173 | 0,503 | 85,6 | 0,631 | 0,9218 |
| mix - mtp5 | -0,2357 | 0,503 | 85,6 | -0,469 | 0,9657 |

| | | | | | |
|---|---|---|---|---|---|
| Note: contrasts are still on the scale(6.62, 1.1) scale Degrees-of-freedom method: kenward-roger P value adjustment: tukey method for comparing a family of 4 estimates | | | | | |

### Bacterial abundance (CFU) as a function of time (day) and exposure to phages.

Random effects include individual IDs and the two experiments performed. Overall analysis of variance (ANOVA) reveals significant effects of day (p=0.00002652) and the interaction day:phage (p=0.02422) but no significant effect was shown for phage (p=0.93517).

The post-hoc Tukey comparisons displayed above were performed between mice exposed to the different phages (cpl, mtp5 and mix) and not exposed within each day.

**Table 9. Percentage of resistant clones**

| | | infected | resistance | %resistance | #(incomplete resistance) | notes |
|---|---|---|---|---|---|---|
| SB3928 | control | 21 | 3 | 12,5 | | |
| | cp48 | 0 | 50 | 100 | | |
| | cp48+mtp6 | 0 | 50 | 100 | | |
| | cp48+mtp5 | 0 | 50 | 100 | | |
| | wza control | 24 | 0 | 0 | | |
| | mtp6 | 0 | 50 | 100 | | |
| | mtp8 | 0 | 50 | 100 | | |
| SB4975 | control | 24 | 0 | 0 | | |
| | cp18 | 0 | 50 | 100 | | |
| | cp20 | 0 | 50 | 100 | | |
| | cp17 | 2 | 48 | 96 | | |
| | cp17+mtp7 | 9/5 | 41/45 | 82/90 | | |
| | wza control | 24 | 0 | 0 | | |
| | mtp7 | 0 | 50 | 100 | | |
| | mtp5 | 0 | 50 | 100 | | |
| SB4496 | control | 24 | 0 | 0 | | |
| | cp1 | 0 | 50 | 100 | | |
| | cp1+mtp5 | 0 | 50 | 100 | | |
| | cp1+mtp4 | * | * | * | | too small and diverse colonies |
| | wza control | 24 | 0 | 0 | | |
| | mtp5 | * | * | * | | colonies too small |
| | mtp4 | * | * | * | | colonies too small and diverse |
| | mtp5+mtp4 | * | * | * | | colonies too small and diverse |
| SB20 wza | wza control | 24 | 0 | 0 | | |
| | mtp27 | 0 | 50 | 100 | 13 | |
| SB579 wza | wza control | 24 | 0 | 0 | | |
| | mtp19 | 0 | 50 | 100 | | |
| SB3245 | control | 24 | 0 | 0 | | |
| | cp12 | 0 | 50 | 100 | | |
| | cp12+mtp5 | 0 | 50 | 100 | | |
| SB504 | control | 24 | 0 | 0 | | |
| | cp34 | 5 | 45 | 90 | | |
| | cp34+mtp5 | * | * | * | | colonies too small |

| | | | | | | |
|---|---|---|---|---|---|---|
| ^{∗} colonies phenotype made it difficult of isolation so population was sequence directly from plate | | | | | | |

### Results

### Selection of wild-type and capsule-deficient mutant strains for isolation of anti-K and anti-Kd phages

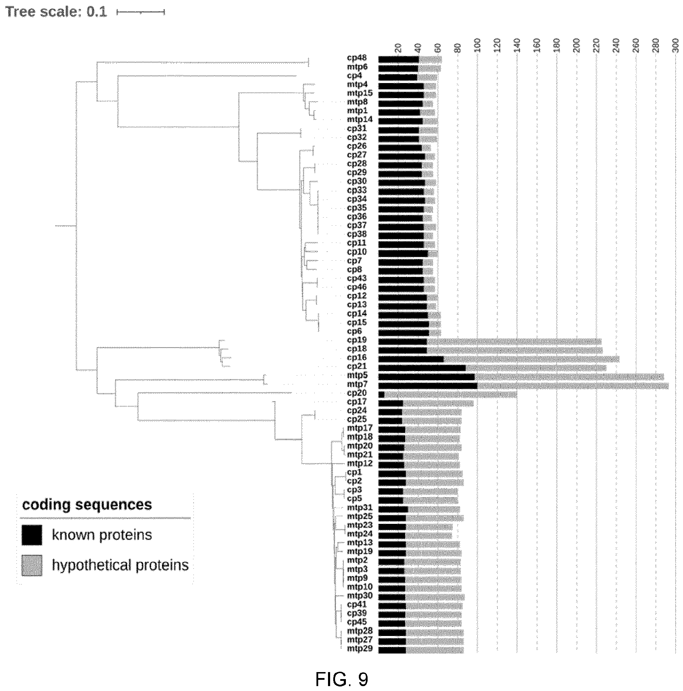

Genotyping of 7,388 K. pneumoniae species complex (henceforth, K. pneumoniae) public genomes revealed a high genetic diversity, with more than 1,193 STs. Some STs (7-gene MLST sequence type) were predominant (ST258, ST11, ST15, ST512, ST101, ST307 and ST147), together representing 55% of all genomes (Figure 9C). To eliminate bias due to recent clonal expansions or outbreaks, only one strain per ST was used to analyse the distribution of K locus types (KL) and the O-antigen locus types (OL). KL1, KL64, KL2, KL10, KL30, KL102, KL106 and KL107 were the most prevalent K-types, whereas OL1, OL2 and OL3/O3a were the predominant O-types (Figure 9, consistent with recent literature (Lam, Wick, Watts, et al. 2021). We thus selected as hosts for phage isolation, 7 WT Kp1 strains (Table 1) representative of these major K- and O-types. Additionally, 7 Kp capsule-deficient strains (de Sousa et al. 2020) harbouring the three most prevalent O-types (Figure 7) were also included (Table 1).

Based on the population genomics analysis (Figure 9), we selected (Table1) from our strain bank (SB) collection, 7 K. pneumoniae (Kp1) strains as hosts for phage isolation:
K. pneumoniae SB4975 (NJST258_2), a representative of the ST258-KL107, one of the most widespread lineages in clinical settings and linked to nosocomial infections and outbreaks (Deleo et al. 2014). K. pneumoniae SB504 (NCTC8172), a ST505 harbouring the cps locus (KL) structure type 64, one of the most disseminated KL-types and which has been extensively associated with two widespread carbapenemase-producing ST's, ST147 and ST11 (Rodrigues et al. 2022; Dong et al. 2018). Two other selected hosts were strains 899 SB5442 (899) and SB5521 (1253), representing two successful MDR ST-KL combinations in clinical settings, ST101-KL106 and ST307-KL102, respectively (Huynh et al. 2020; Wyres et al. 2019). K. pneumoniae strain SB3245 (CIP52.214), a ST297-KL10, was selected as it represented one of the most prevalent KL-types. To finish, we selected two hypervirulent (Hv) K. pneumoniae strains, SB3341 (CIP52.145) ST66-KL2 and SB3928 (NTUH_K2044) ST23-KL1, representing K1 and K2 capsule types associated with severe invasive infections (Wu et al. 2009; Lery et al. 2014). ST23 is the most frequent sublineage isolated in community-acquired liver abscesses, which is a prevalent infection in Asian countries (Wu et al. 2009; Turton et al. 2007; Merlet et al. 2012).

The seven capsule-deficient strains used for anti-Kd phage included 6 K. pneumoniae and 1 K. variicola subsp. variicola (Kp3): Kp1 SB20Δwza ST15/O1v1 (04A025), Kp1 SB3928Δwza ST23/O1v2 (NTUH_K2044), Kp1 SB4021ΔwcaJ (SA1) and SB4454ΔwcaJ (CG43) ST86/O1v1; Kp1 SB4496Δwza ST380/O1v1 (BJ1-GA), Kp1 SB4975Δwza ST258/O2v2 (NJST258_2) and Kp3 SB579Δwza ST146-O3/O3a (342) (de Sousa et al. 2020). Together, these O types represented 50.7% of non-redundant strains in the genomic database.

### Isolation and genomic characterization of 68 new Kp phages

We isolated and sequenced 41 anti-K phages and 27 anti-Kd phages (Table 3), all categorized as virulent phages (BACPHLIP) (Hockenberry and Wilke 2021; Mavrich and Hatfull 2017). No genes encoding for putative virulence factors or antibiotic resistance were found in any of these phages genome.

A phylogenetic analysis of the isolated phages was performed together with 98 publicly available complete anti-Klebsiella phage genomes. Our phages were distributed into five phage families: Autographiviridae, Drexlerviridae, Myoviridae, Ackermannviridae and Schitoviridae (Figure 1). The majority anti-K phages were associated with the Autographiviridae family (26/41), whereas 19 out of 27 of anti-Kd phages belonged to Drexlerviridae (Figure 1, Table 3). Several virulent phages of Klebsiella are known to have depolymerase functional domains in their tail fibers, giving them the ability to digest specific exopolysaccharides. Protein analysis and comparison with already described depolymerases detected on the anti-K phages detected between 0 and 5 depolymerase domains, whereas anti-Kd phages had only 0 or 1 domains (Tables 4 and 5).

**Table 2 Bacterial Collection**

| **ID in this study** | **Strain ID** | **SB ID** | **PhG** | **ST** | **KL** | **O** | **Origin** | **Source details** | **Year** | **Country** | **Accession number** |
|---|---|---|---|---|---|---|---|---|---|---|---|
| **#1/#1A** | BJ1-GA | SB4496 | Kp1 | ST380 | KL2/Δwza | O1v1 | Human | Clinical | 2011 | France | NZ_HG518479.1 |
| **#2/#2Δ** | 04A025 | SB20 | Kp1 | ST15 | KL24/Δwza | O1v1 | Human | Clinical | 1997 | France | GCA_900977905.1 |
| **#3/#3Δ** | SA1 | SB4021 | Kp1 | ST86 | KL2/ΔwcaJ | O1v1 | Human | Clinical | 2008 | France | GCF_020525885.1 |
| **#4/#4Δ** | CG43 | SB4454 | Kp1 | ST86 | KL2/ΔwcaJ | O1v1 | Human | Clinical | NA | NA | NC_022566, NC_005249 |
| **#5/#5Δ** | NTUH_K2044 | SB3928 | Kp1 | ST23 | KL1/Δwza | O1v2 | Human | Clinical | NA | Taiwan | NC_012731, NC_006625 |
| #**6** | T69 | SB4536 | Kp1 | ST375 | KL2/NM | O1v2 | Human | Clinical | 2010 | France | GCF_000529775.1 |
| **#7** | 35285 | SB5097 | Kp1 | ST2416 | NM | O2v1 | Animal (bovine) | Carriage | 2015 | France | NA |
| **#8** | 1170 | SB5462 | Kp1 | ST45 | KL24/NM | O2v1 | Human (pregnant woman) | Carriage | 2016 | Madagascar | ERR3416005 |
| **#9** | SB1170 | SB1170 | Kp1 | ST45 | KL24/NM | O2v1 | Human | Carriage | 2002 | The Netherlands | NZ_CP084856.1 |
| **#10/#10Δ** | NJST258_2 | SB4975 | Kp1 | ST258 | KL107/Δwza | O2v2 | Human | Clinical | 2010 | USA | NZ_CP006923-928 |
| **#11** | SB1139 | SB1139 | Kp1 | ST37 | KL8/NM | O2v2 | Human | Carriage | 2002 | The Netherlands | GCA 900977655.1 |
| **#12/#12Δ** | 342 | SB579 | Kp3 | ST146 | KL30/Δwza | O3/O3a | Food | Maize endophytic diazotroph | NA | USA | NC_011281, NC_011282, NC_011283 |
| **#13** | K293M | SB5655 | Kp1 | ST1145 | NM | O3/O3a | Human (pregnant woman) | Carriage | 2016 | Cambodia | ERR3416182 |
| **#14** | 07A044 | SB30 | Kp4 | ST1215 | KL53/NM | O3/O3a | Human | Clinical | 1997 | Germany | GCA_000613225.1 |
| **#15** | 38120 | SB5235 | Kp1 | ST17 | NM | O3b | Animal (poultry) | Carriage | 2015 | France | NA |
| **#16** | MVK-03E048 | SB6163 | Kp1 | ST1261 | NM | O4 | Environment | Water | 2018 | France | NA |
| **#17** | 35284 | SB5096 | Kp1 | ST2415 | NM | O4 | Animal (bovine) | Carriage | 2015 | France | NA |
| **#18** | 34842 | SB5084 | Kp3 | ST2409 | NM | O5 | Animal (bovine) | Carriage | 2015 | France | NA |
| **#19** | MVK-03E089 | SB6438 | Kp4 | ST662 | NM | O5 | Environment | Roots | 2018 | France | NA |
| **#20** | MVK-03E195 | SB6629 | Kp3 | ST123 | NM | O5 | Environment | Soil | 2018 | France | NA |
| **#21** | 1398 | SB5623 | Kp1 | ST275 | NM | 012 | Human (pregnant woman) | Carriage | 2016 | Madagascar | ERR3416151 |
| **#22** | 814 | SB5387 | Kp5 | ST2466 | NM | OL101 | Human (pregna nt woman) | Carriage | 2015 | Madagascar | CAAHGL010000000 |
| **#23** | SB1124 | SB1124 | Kp2 | ST2274 | KL138/NM | OL102 | Environment | Water | 2002 | The Netherlands | GCA_900977675.1 |
| **#24** | 35649 | SB5137 | Kp1 | ST661 | NM | OL102 | Animal (bovine) | Carriage | 2015 | France | NA |
| **#25** | 1268 | SB5533 | Kp4 | ST2562 | NM | 012 | Human (pregnant woman) | Carriage | 2016 | Madagascar | ERR3416066 |
| **#26** | CDC 4241-71 Brenner | SB94 | Kp5 | ST1216 | KL23/NM | OL103 | Environment | NA | NA | NA | GCF_900978435.1 |
| **#27** | 1266 | SB5531 | Kp5 | ST2561 | NM | OL103 | Human (pregnant woman) | Carriage | 2016 | Madagascar | GCF_900978675.1 |
| **#28** | MVK-03E175 | SB6492 | Kp1 | ST3345 | NM | OL104 | Environment | roots | 2018 | France | NA |
| **#29** | MVK-03F084 | SB6606 | Kp2 | ST104 | NM | OL104 | Environment | water | 2019 | France | NA |
| **#30** | MVK-03E190 | SB6506 | Kp1 | ST2425 | NM | OL104 | Environment | soil | 2018 | France | NA |
| **#31** | CIP 52.145 | SB3341 | Kp1 | ST66 | KL2 | O1v2 | Human | Clinical | <1935 | Indonesia | F0834904-06 |
| **#32** | SB4-2 | SB1067 | Kp1 | ST17 | KL2 | O2v2 | Human | Carriage | 2002 | The Netherlands | GCA_900977535.1 |
| **#33** | cur15505 | SB2390 | Kp1 | ST14 | KL2 | O1v1 | Human | Clinical | NA | Curaçao | NZ_CP084852.1 |
| **#34** | ATCC43816 KPPR1 | SB4358 | Kp1 | ST493 | KL2 | O1v1 | NA | NA | 2010 | USA | GCF_000742755.1 |
| **#35** | ATCC 13883 (DSM 30104) | SB132 | Kp2 | ST3 | KL3 | O1v1 | Human | Clinical | <1990 | NA | GCF_000742135.1 |
| **#36** | Klebsiella 919 (CIP 52.214) | SB3245 | Kp1 | ST297 | KL10 | O1v1 | NA | KL10 ref | NA | NA | NA |
| **#37** | Mich. 61 (CIP 52.219) | SB187 | Kp1 | ST502 | KL15 | - | NA | KL15 ref | NA | NA | NA |
| **#38** | 2069-49 (CIP 52.220) | SB3252 | Kp1 | ST561 | KL16 | - | NA | KL16 ref | NA | NA | NA |
| **#39** | F2R9 | SB48 | Kp3 | ST2263 | KL16 | - | Food | Banana | NA | Mexico | NZ_CP072130.1 |
| **#40** | 2005/49 | SB189 | Kp1 | ST322 | KL17 | - | NA | KL17 ref | NA | NA | NA |
| **#41** | PS 4783 | SB4983 | Kp1 | ST35 | KL22 | O1v1 | Human | Clinical | 2015 | Belgium | NA |
| | | | | | | | | | | | |
| | 140109.305 | | | | | | | | | | |
| **#42** | 1171 | SB5462 | Kp1 | ST45 | KL24 | O2v1 | Human (pregnant woman) | Carriage | 2016 | Madagascar | ERR3416005 |
| **#43** | 2002/49 (CIP 52.230) | SB197 | Kp1 | ST47 | KL25 | - | NA | KL25 ref | NA | NA | NA |
| **#44** | 7824 | SB318 | Kp1 | ST43 | KL30 | - | NA | NA | NA | NA | NA |
| **#45** | 01A065 | SB1 | Kp3 | ST2273 | KL31 | O3/O3a | Human | Clinical | 1997 | Austria | NZ_CP084766.1 |
| **#46** | 06A093 | SB28 | Kp1 | ST10 | KL35 | - | Human | Clinical | 1997 | France | NA |
| **#47** | 01A030 | SB11 | Kp2 | ST1528 | KL35 | O3/O3a | Human | Clinical | 1997 | Austria | GCA_000751755.1 |
| **#48** | 08A119 | SB33 | Kp6 | ST1214 | KL51 | O1v2 | Huma | Clinical | 1997 | Germany | NZ_CP084768.1 |
| **#49** | MGH78578 | SB107 | Kp1 | ST38 | KL52 | OL101 | Human | Clinical | 1994 | USA | GCF_000016305.1 |
| **#50** | CIP110288 | SB4697 | Kp4 | ST2275 | KL57 | - | Environment | Soil | 2010 | China | CP084778.1 |
| **#51** | 12A476 | SB203 | Kp4 | ST384 | KL61 | O3/O3a | Human | Clinical | 1998 | The Netherlands | GCA_900977875.1 |
| **#52** | 1405 | SB5961 | Kp1 | ST48 | KL62 | O1v1 | Human (pregnant woman) | Carriage | 2016 | Madagascar | ERR3416378 |
| **#53** | 38037 | SB5171 | Kp1 | ST147 | KL64 | O2v1 | Animal (poultry) | Carriage | 2015 | France | NA |
| **#54** | KLSP64 (NCTC8172) | SB504 | Kp1 | ST505 | KL64 | - | NA | KL64 ref | NA | NA | UGKZ01000002.1 |
| **#55** | U41 | SB2110 | Kp2 | ST526 | KL72 | - | Environment | Environment | 1990 | Germany | GCA_900978005.1 |
| **#56** | 1253 | SB5521 | Kp1 | ST307 | KL102 | O2v2 | Human (pregnant woman) | Carriage | 2016 | Madagascar | ERR3416055 |
| **#57** | 899 | SB5442 | Kp1 | ST101 | KL106 | NP | Human (pregnant woman) | Carriage | 2016 | Madagascar | ERR3415970 |
| **#58** | 09A323 | SB164 | Kp4 | ST414 | KL123 | - | Human | Clinical | 1997 | Greece | GCA_900977715.1 |
| **#59** | Kleb Ali 0320584 | SB98 | Kp2 | ST622 | KL125 | O3/O3a | Environment | NA | NA | NA | GCA_900978495.1 |
| **#60** | At-22 | SB4767 | Kp3 | ST1220 | KL134 | - | Environment | Atta cephalotes fungus garden | NA | NA | NC_013850 |
| **#61** | 1285 | SB5546 | Kp1 | ST29 | KL19 | O1v2 | Human (pregnant woman) | Carriage | 2016 | Madagascar | ERR3416076 |

| | | | | | | | | | | | |
|---|---|---|---|---|---|---|---|---|---|---|---|
| ID, identification; SB, strain bank; PhG, phylogroup; ST, Sequence-Type; KL, capsular locus; O, O-antigen locus; NA, not available | | | | | | | | | | | |

**Table 3 Isolated Phages**

| **Phage-type** | **Complete name** | **Short name** | **Genome size (bp)** | **no. of ORF** | **% hypothetical proteins** | **Family** | **Subfamily** | **Genus** | **Host strain** | | | | |
|---|---|---|---|---|---|---|---|---|---|---|---|---|---|
| | | | | | | | | | **Strain ID** | **SB ID** | **ST** | **KL-type** | **O-type** |
| **anti-K^{d}** | **vB_KpP_SB4 496_mtpl** | mtp1 | 38885 | 57 | 33,3 | *Autographi viridae* | *Studiervirinae* | *Teetrevirus* | **BJ1-GA** | SB4496Δwza | ST380 | mutant | O1v1 |
| | **vB_KpS_SB4 496_mtp2** | mtp2 | 49122 | 83 | 71,1 | *Drexlervirid ae* | - | *Webervirus* | **BJ1-GA** | SB4496Δwza | ST380 | mutant | O1v1 |
| | **vB_KpS_SB4 496_mtp3** | mtp3 | 49122 | 83 | 71,1 | *Drexlervirid ae* | - | *Webervirus* | **BJ1-GA** | SB4496Δwza | ST380 | mutat | O1v1 |
| | **vB_KpP_SB4 496_mtp4** | mtp4 | 40132 | 58 | 32,8 | *Autographi viridae* | *Studiervirinae* | *Teetrevirus* | **BJ1-GA** | SB4496Δwza | ST380 | mutant | O1v1 |
| | **vB_KpM_SB4 496_mtp5** | mtp5 | **175100** | 289 | 66,8 | ***Myoviridae*** | ***Tevenvirinae*** | ***Slopekvirus*** | **BJ1-GA** | **SB4496Δwza** | **ST380** | **mutant** | O1v1 |
| | **vB_KpP_SB3 928_mtp6** | mtp6 | 43492 | 63 | 57,1 | *Autographi viridae* | *Slopekvirinae* | *Drulisvirus* | **NTUH_K 2044** | SB3928Δwza | ST23 | mutant | O1v2 |
| | **vB_KpM_SB4 975_mtp7** | mtp7 | 177726 | 293 | 66,6 | *Myoviridae* | *Tevenvirinae* | *Slopekvirus* | **NJST258 _2** | SB4975Δwza | ST258 | mutant | O2v2 |
| | **vB_KpP_SB3 928_mtp8** | mtp8 | 39208 | 55 | 27,3 | *Autographi viridae* | *Studiervirinae* | *Teetrevirus* | **NTUH_K 2044** | SB3928Δwza | ST23 | mutant | O1v2 |
| | **vB_KpS_SB4 496_mtp9** | mtp9 | 49199 | 84 | 70,2 | *Drexlervirid ae* | - | *Webervirus* | **BJ1-GA** | SB4496Δwza | ST380 | mutant | O1v1 |
| | **vB_KpS_SB4 496_mtp10** | mtp10 | 49198 | 84 | 70,2 | *Drexlervirid ae* | - | *Webervirus* | **BJ1-GA** | SB4496Δwza | ST380 | mutant | O1v1 |
| | **vB_KpS_SB4 975_mtp12** | mtp12 | 49712 | 82 | 70,7 | *Drexlervirid ae* | - | *Webervirus* | **NJST258 _2** | SB4975Δwza | ST258 | mutant | O2v2 |
| | **vB_KpS_SB4 496_mtp13** | mtp13 | 48961 | 82 | 68,3 | *Drexlervirid ae* | - | *Webervirus* | **BJ1-GA** | SB4496Δwza | ST380 | mutant | O1v1 |
| | **vB_KpP_SB4 496_mtpl4** | mtp14 | 40202 | 60 | 33,3 | *Autographi viridae* | *Studiervirinae* | *Teetrevirus* | **BJ1-GA** | SB4496Δwza | ST380 | mutant | O1v1 |
| | **vB_KpP_SB4 496_mtp15** | mtp15 | 39756 | 58 | 31,0 | *Autographi viridae* | *Studiervirinae* | *Teetrevirus* | **BJ1-GA** | SB4496Δwza | ST380 | mutant | O1v1 |
| | **vB_KpS_SB5 79_mtp17** | mtp17 | 49806 | 83 | 69,9 | *Drexlervirid ae* | - | *Webervirus* | **Strain 342** | SB579Δwza | ST146 | mutant | O3/O3a |
| | **vB_KpS_SB5 79_mtp18** | mtp18 | 49806 | 82 | 69,5 | *Drexlervirid ae* | - | *Webervirus* | **Strain 342** | SB579Δwza | ST146 | mutant | O3/O3a |
| | **vB_KpS_SB5 79_mtp19** | mtp19 | 49290 | 84 | 67,9 | *Drexlervirid ae* | - | *Webervirus* | **Strain 342** | SB579Δwza | ST146 | mutant | O3/O3a |
| | **vB_KpS_SB4 975_mtp20** | mtp20 | 49672 | 84 | 70,2 | *Drexlervirid ae* | - | *Webervirus* | **NJST258 _2** | SB4975Δwza | ST258 | mutant | O2v2 |
| | **vB_KpS_SB4 496_mtp21** | mtp21 | 49504 | 81 | 70,4 | *Drexlervirid ae* | - | *Webervirus* | **BJ1-GA** | SB4496Δwza | ST380 | mutant | O1v1 |
| | **vB_KpS_SB2 0_mtp23** | mtp23 | 47319 | 75 | 65,3 | *Drexlervirid ae* | - | *Webervirus* | **04A025** | SB20Δwza | ST15 | mutant | O1v1 |
| | **vB_KpS_SB2 0_mtp24** | mtp24 | 47299 | 74 | 66,2 | *Drexlervirid ae* | - | *Webervirus* | **04A025** | SB20Δwza | ST15 | mutant | O1v1 |
| | **vB_KpS_SB2 0_mtp25** | mtp25 | 50132 | 86 | 68,6 | *Drexlervirid ae* | - | *Webervirus* | **04A025** | SB20Δwza | ST15 | mutant | O1v1 |
| | **vB_KpS_SB2 0_mtp27** | mtp27 | 49035 | 86 | 69,8 | *Drexlervirid ae* | - | *Webervirus* | **04A025** | SB20Δwza | ST15 | mutant | O1v1 |
| | **vB_KpS_SB2 0_mtp28** | mtp28 | 49036 | 86 | 69,8 | *Drexlervirid ae* | - | *Webervirus* | **04A025** | SB20Δwza | ST15 | mutant | O1v1 |
| | **vB_KpS_SB4 021_mtp29** | mtp29 | 49114 | 86 | 69,8 | *Drexlervirid ae* | - | *Webervirus* | **SA1** | SB4021Δwca J | ST86 | mutant | O1v1 |
| | **vB_KpS_SB4 021_mtp30** | mtp30 | 49926 | 87 | 70,1 | *Drexlervirid ae* | - | *Webervirus* | **SA1** | SB4021Δwca J | ST86 | mutant | O1v1 |
| | **vB_KpS_SB4 454_mtp31** | mtp31 | 48467 | 82 | 64,6 | *Drexlervirid ae* | - | *Webervirus* | **CG43** | SB4454Δwca J | ST86 | mutant | O1v1 |
| anti-K | **vB_KpS_SB3 341_cp1** | cp1 | 49492 | 85 | 68,2 | *Drexlervirid ae* | - | *Webervirus* | **CIP 52.145** | SB3341 | ST66 | KL2 | O1v2 |
| | **vB_KpS_SB3 341_cp2** | cp2 | 49514 | 86 | 68,6 | *Drexlervirid ae* | - | *Webervirus* | **CIP 52.145** | SB3341 | ST66 | KL2 | O1v2 |
| | **vB_KpS_SB3 341_cp3** | cp3 | 49289 | 80 | 70,0 | *Drexlervirid ae* | - | *Webervirus* | **CIP 52.145** | SB3341 | ST66 | KL2 | O1v2 |
| | **vB_KpP_SB3 341_cp4** | cp4 | 43903 | 59 | 64,4 | *Autographi viridae* | *Molineuxvirina e* | - | **CIP 52.145** | SB3341 | ST66 | KL2 | O1v2 |
| | **vB_KpS_SB3 341_cp5** | cp5 | 49366 | 80 | 70,0 | *Drexlervirid ae* | - | *Webervirus* | **CIP 52.145** | SB3341 | ST66 | KL2 | O1v2 |
| | **vB_KpP_SB3 341_cp6** | cp6 | 40819 | 63 | 33,3 | *Autographi viridae* | *Studiervirinae* | *Przondovirus* | **CIP 52.145** | SB3341 | ST66 | KL2 | O1v2 |
| | **vB_KpP_SB3 341_cp7** | cp7 | 40519 | 55 | 32,7 | *Autographi viridae* | *Studiervirinae* | *Przondovirus* | **CIP 52.145** | SB3341 | ST66 | KL2 | O1v2 |
| | **vB_KpP_SB3 245_cp8** | cp8 | 40866 | 55 | 32,7 | *Autographi viridae* | *Studiervirinae* | *Przondovirus* | **CIP 52.214** | SB3245 | ST297 | KL10 | O1v1 |
| | **vB_KpP_SB3 245_cp10** | cp10 | 41076 | 60 | 33,3 | *Autographi viridae* | *Studiervirinae* | *Przondovirus* | **CIP 52.214** | SB3245 | ST297 | KL10 | O1v1 |
| | **vB_KpP_SB3 245-cp11** | cp11 | 40632 | 57 | 33,3 | *Autographi viridae* | *Studiervirinae* | *Przondovirus* | **CIP 52.214** | SB3245 | ST297 | KL10 | O1v1 |
| | **vB_KpP_SB3 245_cp12** | cp12 | 41242 | 60 | 35,0 | *Autographi viridae* | *Studiervirinae* | *Przondovirus* | **CIP 52.214** | SB3245 | ST297 | KL10 | O1v1 |
| | **vB_KpP_SB3 245_cp13** | cp13 | 41187 | 58 | 32,8 | *Autographi viridae* | *Studiervirinae* | *Przondovirus* | **CIP 52.214** | SB3245 | ST297 | KL10 | O1v1 |
| | **vB_KpP_SB3 245_cp14** | cp14 | 40327 | 63 | 33,3 | *Autographi viridae* | *Studiervirinae* | *Przondovirus* | **CIP 52.214** | SB3245 | ST297 | KL10 | O1v1 |
| | **vB_KpP_SB3 245_cp15** | cp15 | 40764 | 63 | 33,3 | *Autographi viridae* | *Studiervirinae* | *Przondovirus* | **CIP 52.214** | SB3245 | ST297 | KL10 | O1v1 |
| | **vB_KpM_SB4 975_cp16** | cp16 | 159143 | 248 | 81,0 | *Ackermann viridae* | - | *Taipeivirus* | **NJST258 _2** | SB4975 | ST258 | KL107 | O2v2 |
| | **vB_KpS_SB4 975_cp17** | cp17 | 52028 | 96 | 74,0 | *Drexlervirid ae* | - | *Webervirus* | **NJST258 _2** | SB4975 | ST258 | KL107 | O2v2 |
| | **vB_KpM_SB4 975_cp18** | cp18 | 156221 | 230 | 80,4 | *Ackermann viridae* | - | *Taipeivirus* | **NJST258 _2** | SB4975 | ST258 | KL107 | O2v2 |
| | **vB_KpM_SB4 975_cp19** | cp19 | 157059 | 230 | 85,7 | *Ackermann viridae* | - | *Taipeivirus* | **NJST258 _2** | SB4975 | ST258 | KL107 | O2v2 |
| | **vB_Kp_SB49 75_cp20** | cp20 | 73745 | 145 | 93,8 | *Schitovirida* e | - | - | **NJST258 _2** | SB4975 | ST258 | KL107 | O2v2 |
| | **vB_KpM_SB5 521_cp21** | cp21 | 160505 | 236 | 75,8 | *Ackermann viridae* | - | *Taipeivirus* | **1253** | SB5521 | ST307 | KL102 | O2v2 |
| | **vB_KpS_SB5 521_cp24** | cp24 | 50485 | 84 | 72,6 | *Drexlervirid ae* | - | *Webervirus* | **1253** | SB5521 | ST307 | KL102 | O2v2 |
| | **vB_KpS_SB5 521_cp25** | cp25 | 50507 | 84 | 72,6 | *Drexlervirid ae* | - | *Webervirus* | **1253** | SB5521 | ST307 | KL102 | O2v2 |
| | **vB_KpP_SB5 04_cp26** | cp26 | 39916 | 53 | 30,2 | *Autographi viridae* | *Studiervirinae* | *Przondovirus* | **NCTC817** 2 | SB504 | ST505 | KL64 | O1v1 |
| | **vB_KpP_SB5 04_cp27** | cp27 | 40764 | 57 | 33,3 | *Autographi viridae* | *Studiervirinae* | *Przondovirus* | **NCTC817** 2 | SB504 | ST505 | KL64 | O1v1 |
| | **vB_KpP_SB5 04_cp28** | cp28 | 39661 | 55 | 36,4 | *Autographi viridae* | *Studiervirinae* | *Przondovirus* | **NCTC817** 2 | SB504 | ST505 | KL64 | O1v1 |
| | **vB_KpP_SB5 04_cp29** | cp29 | 39662 | 55 | 36,4 | *Autographi viridae* | *Studiervirinae* | *Przondovirus* | **NCTC817** 2 | SB504 | ST505 | KL64 | O1v1 |
| | **vB_KpP_SB5 04_cp30** | cp30 | 40881 | 58 | 32,8 | *Autographi viridae* | *Studiervirinae* | *Przondovirus* | **NCTC817** 2 | SB504 | ST505 | KL64 | O1v1 |
| | **vB_KpP_SB5 04_cp31** | cp31 | 40303 | 60 | 45,0 | *Autographi viridae* | *Studiervirinae* | *Przondovirus* | **NCTC817 2** | SB504 | ST505 | KL64 | O1v1 |
| | **vB_KpP_SB5 04_cp32** | cp32 | 40381 | 59 | 44,1 | *Autographi viridae* | *Studiervirinae* | *Przondovirus* | **NCTC817** 2 | SB504 | ST505 | KL64 | O1v1 |
| | **vB_KpP_SB5 04_cp33** | cp33 | 40350 | 56 | 33,9 | *Autographi viridae* | *Studiervirinae* | *Przondovirus* | **NCTC817** 2 | SB504 | ST505 | KL64 | O1v1 |
| | **vB_KpP_SB5 04_cp34** | cp34 | 40427 | 57 | 33,3 | *Autographi viridae* | *Studiervirinae* | *Przondovirus* | **NCTC817** 2 | SB504 | ST505 | KL64 | O1v1 |
| | **vB_KpP_SB5 04_cp35** | cp35 | 40368 | 55 | 32,7 | *Autographi viridae* | *Studiervirinae* | *Przondovirus* | **NCTC817** 2 | SB504 | ST505 | KL64 | O1v1 |
| | **vB_KpP_SB5 04_cp36** | cp36 | 40366 | 54 | 33,3 | *Autographi viridae* | *Studiervirinae* | *Przondovirus* | **NCTC817** 2 | SB504 | ST505 | KL64 | O1v1 |
| | **vB_KpP_SB5 04_cp37** | cp37 | 40427 | 58 | 36,2 | *Autographi viridae* | *Studiervirinae* | *Przondovirus* | **NCTC817** 2 | SB504 | ST505 | KL64 | O1v1 |
| | **vB_KpP_SB5 04_cp38** | cp38 | 40350 | 55 | 32,7 | *Autographi viridae* | *Studiervirinae* | *Przondovirus* | **NCTC817** 2 | SB504 | ST505 | KL64 | O1v1 |
| | **vB_KpP_SB5 04_cp39** | cp39 | 49251 | 84 | 69,0 | *Drexlervirid ae* | - | *Webervirus* | 899 | SB5442 | ST101 | KL106 | O1v2 |
| | **vB_KpS_SB5 442_cp41** | cp41 | 49193 | 85 | 68,2 | *Drexlervirid ae* | - | *Webervirus* | 899 | SB5442 | ST101 | KL106 | O1v2 |
| | **vB_KpP_SB5 442_cp43** | cp43 | 39694 | 57 | 33,3 | *Autographi viridae* | *Studiervirinae* | *Przondovirus* | 899 | SB5442 | ST101 | KL106 | O1v2 |
| | **vB_KpS_SB5 442_cp45** | cp45 | 49251 | 84 | 69,0 | *Drexlervirid ae* | - | *Webervirus* | 899 | SB5442 | ST101 | KL106 | O1v2 |
| | **vB_KpP_SB5 442_cp46** | cp46 | 39694 | 57 | 33,3 | *Autographi viridae* | *Studiervirinae* | *Przondovirus* | 899 | SB5442 | ST101 | KL106 | O1v2 |
| | **vB_KpP_SB3 928_cp48** | cp48 | 43569 | 64 | 56,3 | *Autographi viridae* | *Slopekvirinae* | *Drulisvirus* | **NTUH_K** 2044 | SB3928 | ST23 | KL1 | O1v2 |

| | | | | | | | | | | | | | |
|---|---|---|---|---|---|---|---|---|---|---|---|---|---|
| ORF, open-reading frames; ID, identification; SB, strain bank; ST, Sequence-Type; KL, capsular locus; O, O-antigen locus; Δ, mutants; wt, wild-type. | | | | | | | | | | | | | |

**Table 4 Depolymerases**

| phage | qseqid - pK64 | pident | evalue | hmm analysis | e-value | capsule | ST | O | host-range |
|---|---|---|---|---|---|---|---|---|---|
| cp1 | YP_008532048.1 | 47,872 | 2,13E-20 | - | - | KL2 | ST66 | O1v2 | KL2 (3strains) |
| | YP_398994.1 | 51,282 | 3,73E-32 | - | - | | | | |
| cp2 | YP_008532048.1 | 47,872 | 2,14E-20 | - | - | KL2 | ST66 | O1v2 | KL2 (3strains) |
| | YP_398994.1 | 51,282 | 3,75E-32 | - | - | | | | |
| cp3 | YP_008532048.1 | 47,872 | 3,13E-20 | - | - | KL2 | ST66 | O1v2 | KL2 (3strains) |
| | YP_398994.1 | 51,282 | 2,79E-32 | - | - | | | | |
| cp4 | YP_007010682.1 | 47,478 | 1,51E-101 | - | - | KL2 | ST66 | O1v2 | KL2(only3341) |
| | YP_008532048.1 | 47,872 | 3,13E-20 | - | - | KL2 | ST66 | O1v2 | KL2 (3strains) |
| | YP_398994.1 | 51,282 | 2,79E-32 | - | - | | | | |
| | YP_002003830.1 | 70,588 | 9,07E-147 | - | - | | | | |
| cp6 | YP_007003187.1 | 44,928 | 2,08E-12 | - | - | KL2 | ST66 | O1v2 | |
| | YP_007003187.1 | 34,286 | 3,01E-11 | - | - | | | | |
| cp7 | YP_002003830.1 | 77,816 | 1,43E-157 | - | - | KL2 | ST66 | O1v2 | KL2 (2strains) & KL22 |
| | YP_007003187.1 | 39 | 3,13E-13 | - | - | | | | |
| | YP_002003830.1 | 76,109 | 3,59E-154 | - | - | | | | |
| cp8 | WP_020326882.1 | 36,905 | 7,70E-68 | - | - | KL10 | ST297 | O1v1 | KL10 & KL25 |
| | YP_007003187.1 | 40,278 | 1,97E-12 | - | - | | | | |
| | YP_002003830.1 | 75,768 | 4,04E-152 | - | - | | | | |
| cp10 | WP_020326882.1 | 36,926 | 8,76E-67 | - | - | KL10 | ST297 | O1v1 | KL10 & KL25 |
| | YP_007003187.1 | 40,278 | 2,09E-12 | - | - | | | | |
| | YP_002003830.1 | 75,768 | 9,03E-154 | - | - | | | | |
| cp11 | WP_020326882.1 | 36,706 | 3,85E-66 | - | - | KL10 | ST297 | O1v1 | KL10 & KL25 |
| | YP_007003187.1 | 40,278 | 2,23E-12 | - | - | | | | |
| | YP_002003830.1 | 74,061 | 8,07E-150 | - | - | | | | |
| cp12 | WP_020326882.1 | 36,128 | 1,20E-67 | - | - | KL10 | ST297 | O1v1 | KL10 & KL25 |
| | YP_007003187.1 | 41,667 | 1,91E-12 | - | - | | | | |
| | YP_002003830.1 | 74,061 | 8,06E-150 | - | - | | | | |
| cp13 | WP_020326882.1 | 36,128 | 1,20E-67 | - | - | KL10 | ST297 | O1v1 | KL10 & KL25 |
| | YP_007003187.1 | 41,667 | 1,90E-12 | - | - | | | | |
| | YP_002003830.1 | 70,588 | 8,96E-147 | - | - | | | | |
| cp14 | YP_007003187.1 | 44,928 | 2,06E-12 | - | - | KL10 | ST297 | O1v1 | KL10 |
| | YP_007003187.1 | 34,286 | 2,98E-11 | - | - | | | | |
| | YP_002003830.1 | 70,588 | 9,07E-147 | - | - | | | | |
| cp15 | YP_007003187.1 | 44,928 | 2,08E-12 | - | - | KL10 | ST297 | O1v1 | KL10 |
| | YP_007003187.1 | 34,286 | 3,01E-11 | - | - | | | | |
| | YP_008532048.1 | 48,421 | 8,28E-44 | - | - | | | | |
| cp16 | AGF88658.1 | 49,254 | 1,44E-12 | - | - | KL107 | ST258 | O2v2 | KL107 & KL35 |
| | WP_020801644.1 | 30,899 | 4,37E-12 | - | - | | | | |
| cp17 | YP_398994.1 | 49,219 | 6,52E-35 | - | - | KL107 | ST258 | O2v2 | KL107 |
| | YP_008532048.1 | 47,895 | 2,79E-43 | Peptidase_S74 | 0,000000028 | | | | |
| | YP_008532048.1 | 52,874 | 1,22E-24 | - | - | | | | |
| cp18 | AGF88658.1 | 50,746 | 1,39E-13 | - | - | KL107 | ST258 | O2v2 | KL107 |
| | WP_020326882.1 | 36,346 | 4,06E-88 | - | - | | | | |
| | YP_654147.1 | 32,24 | 1,35E-20 | - | - | | | | |
| | WP_020801644.1 | 30,899 | 4,46E-12 | - | - | | | | |
| | YP_008532048.1 | 47,895 | 2,00E-43 | Pectate_lyase_3 | 0,000065 | | | | |
| | YP_008532048.1 | 44,318 | 3,45E-40 | - | - | | | | |
| cp19 | AGF88658.1 | 52,239 | 4,68E-14 | - | - | KL107 | ST258 | O2v2 | KL107 |
| | AGF88658.1 | 44,643 | 1,82E-11 | - | - | | | | |
| | WP_020801644.1 | 42,241 | 7,85E-16 | - | - | | | | |
| | WP_020801644.1 | 30,899 | 4,29E-12 | - | - | | | | |
| cp20 | - | - | - | - | - | KL107 | ST307 | O2v2 | KL107 |
| | YP_008532048.1 | 57,658 | 7,23E-32 | Peptidase_S74 | 0,000071 | | | | |
| cp21 | YP_008532048.1 | 44,444 | 4,64E-25 | - | - | KL102 | ST307 | O2v2 | KL102 |
| | AGF88658.1 | 41,86 | 7,59E-15 | - | - | | | | |
| | YP_654147.1 | 27,477 | 1,39E-16 | - | - | | | | |
| cp24 | YP_398994.1 | 51,181 | 8,10E-35 | - | - | KL102 | ST307 | O2v2 | KL102 |
| cp25 | YP_398994.1 | 51,181 | 8,10E-35 | - | - | KL102 | ST307 | O2v2 | KL102 |
| cp26 | YP_002003830.1 | 43,987 | 2,23E-58 | - | - | KL64 | ST505 | O1v1 | KL64 (1strain) |
| cp27 | YP_002003830.1 | 43,987 | 2,16E-58 | - | - | KL64 | ST505 | O1v1 | KL64 (1strain) |
| cp28 | YP_002003830.1 | 43,671 | 1,54E-57 | - | - | KL64 | ST505 | O1v1 | KL64 (1strain) |
| cp29 | YP_002003830.1 | 43,671 | 1,44E-57 | - | - | KL64 | ST505 | O1v1 | KL64 (1strain) |
| cp30 | YP_002003830.1 | 45,704 | 2,62E-57 | - | - | KL64 | ST505 | O1v1 | KL64 (2strains) |
| cp31 | YP_002003830.1 | 43,262 | 3,02E-30 | - | - | KL64 | ST505 | O1v1 | KL64 (1strain) |
| cp32 | YP_002003830.1 | 43,262 | 4,86E-30 | - | - | KL64 | ST505 | O1v1 | KL64 (2strains) |
| cp33 | YP_002003830.1 | 43,987 | 1,93E-58 | - | - | KL64 | ST505 | O1v1 | KL64 (1strain) |
| cp34 | YP_002003830.1 | 43,987 | 1,90E-58 | - | - | KL64 | ST505 | O1v1 | KL64 (1strain) & KL102 |
| cp35 | YP_002003830.1 | 43,987 | 1,78E-58 | - | - | KL64 | ST505 | O1v1 | KL64 (1strain) |
| cp36 | YP_002003830.1 | 43,987 | 1,46E-58 | - | - | KL64 | ST505 | O1v1 | KL64 (1strain) |
| cp37 | YP_002003830.1 | 43,987 | 1,78E-58 | - | - | KL64 | ST505 | O1v1 | KL64 (1strain) |
| cp38 | YP_002003830.1 | 43,987 | 1,78E-58 | - | - | KL64 | ST505 | O1v1 | KL64 (1strain) |
| cp39 | YP_398994.1 | 50,427 | 3,60E-31 | Peptidase_S74 | 0,00031 | KL106 | ST101 | O1v2 | KL106 & 5mutants |
| cp41 | YP_398994.1 | 41,615 | 3,99E-34 | Peptidase_S74 | 0,0002 | KL106 | ST101 | O1v2 | KL106 & 5mutants |
| | YP_002003830.1 | 76,451 | 3,22E-155 | - | - | | | | |
| cp43 | WP_020326882.1 | 30,303 | 3,07E-40 | - | - | KL106 | ST101 | O1v2 | KL106 & KL22 |
| | YP_007003187.1 | 40 | 7,14E-14 | - | - | | | | |
| cp45 | YP_398994.1 | 50,427 | 3,60E-31 | Peptidase_S74 | 0,00031 | KL106 | ST101 | O1v2 | KL106 & 5mutants |
| | YP_002003830.1 | 76,451 | 3,22E-155 | - | - | | | | |
| cp46 | WP_020326882.1 | 30,303 | 3,07E-40 | - | - | KL106 | ST101 | O1v2 | KL106 |
| | YP_007003187.1 | 40 | 7,14E-14 | - | - | | | | |
| cp48 | - | - | - | - | - | KL1 | ST23 | O1v2 | KL1 |
| | | | | | | | | | |

| phage | qseqid - pK64 | pident | evalue | hmm analysis | e-value | capsule | ST | O | host-range |
|---|---|---|---|---|---|---|---|---|---|
| mtp1 | YP_002003830.1 | 49,677 | 1,89E-40 | - | - | Δwza | ST380 | O1v1 | |
| mtp2 | YP_398994.1 | 51,282 | 8,21E-32 | Peptidase_S74 | 0,00026 | Δwza | ST380 | O1v1 | |
| mtp4 | YP_002003830.1 | 49,677 | 3,98E-40 | - | - | Δwza | ST380 | O1v1 | |
| mtp5 | - | - | - | Peptidase_S74+ | 1,7E-15 | Δwza | ST380 | O1v1 | |
| | - | - | - | Hyaluronidase_1 | 0,00046 | | | | |
| mtp6 | - | - | - | - | - | Δwza | ST23 | O1v2 | |
| mtp7 | - | - | - | - | - | Δwza | ST258 | O2v2 | |
| mtp8 | YP_002003830.1 | 49,032 | 1,02E-39 | - | - | Δwza | ST23 | O1v2 | |
| mtp9 | YP_398994.1 | 42,236 | 3,74E-34 | Peptidase_S74 | 0,00015 | Δwza | ST380 | O1v1 | |
| mtp10 | YP_398994.1 | 42,236 | 3,74E-34 | Peptidase_S74 | 0,00015 | Δwza | ST380 | O1v1 | |
| mtp12 | YP_398994.1 | 48,837 | 8,76E-36 | Peptidase_S74 | 2,3E-10 | Δwza | ST258 | O2v2 | |
| mtp13 | YP_398994.1 | 50,427 | 2,94E-31 | Peptidase_S74 | 0,00024 | Δwza | ST380 | O1v1 | |
| mtp14 | YP_002003830.1 | 49,677 | 1,58E-40 | - | - | Δwza | ST380 | O1v1 | |
| mtp15 | YP_002003830.1 | 49,032 | 5,95E-39 | - | - | Δwza | ST380 | O1v1 | |
| mtpl7 | YP_398994.1 | 49,606 | 6,80E-34 | Peptidase_S74 | 0,000018 | Δwza | ST146 | O3/O3a | |
| mtpl8 | YP_398994.1 | 49,573 | 9,99E-31 | Peptidase_S74 | 0,000017 | Δwza | ST146 | O3/O3a | |
| mtpl9 | YP_398994.1 | 42,5 | 8,67E-35 | Peptidase_S74 | 0,0002 | Δwza | ST146 | O3/O3a | |
| mtp20 | YP_398994.1 | 54,331 | 1,01E-37 | Peptidase_S74 | 0,000018 | Δwza | ST258 | O2v2 | |
| mtp21 | YP_398994.1 | 54,331 | 1,00E-37 | Peptidase_S74 | 0,000017 | Δwza | ST380 | O1v1 | |
| mtp23 | YP_398994.1 | 41,615 | 2,32E-34 | Peptidase_S74 | 0,00018 | Δwza | ST15 | O1v1 | |
| mtp24 | YP_398994.1 | 50,427 | 3,66E-31 | Peptidase_S74 | 0,00027 | Δwza | ST15 | O1v1 | |
| mtp25 | YP_398994.1 | 51,282 | 1,11E-31 | | - | Δwza | ST15 | O1v1 | |
| mtp27 | YP_398994.1 | 51,282 | 6,76E-32 | Peptidase_S74 | 0,000017 | Δwza | ST15 | O1v1 | |
| mtp28 | YP_398994.1 | 51,282 | 5,91E-32 | Peptidase_S74 | 0,000018 | Δwza | ST15 | O1v1 | |
| mtp29 | YP_398994.1 | 51,282 | 1,47E-31 | Peptidase_S74 | 0,00025 | AwcaJ | ST86 | O1v1 | |
| mtp30 | YP_398994.1 | 51,282 | 9,39E-32 | Peptidase_S74 | 0,00029 | AwcaJ | ST86 | O1v1 | |
| mtp31 | YP_398994.1 | 50,427 | 4,88E-31 | Peptidase_S74 | 0,00027 | AwcaJ | ST86 | O1v1 | |

### Anti-K^{d} phages have a broader host range in comparison with anti-K phages

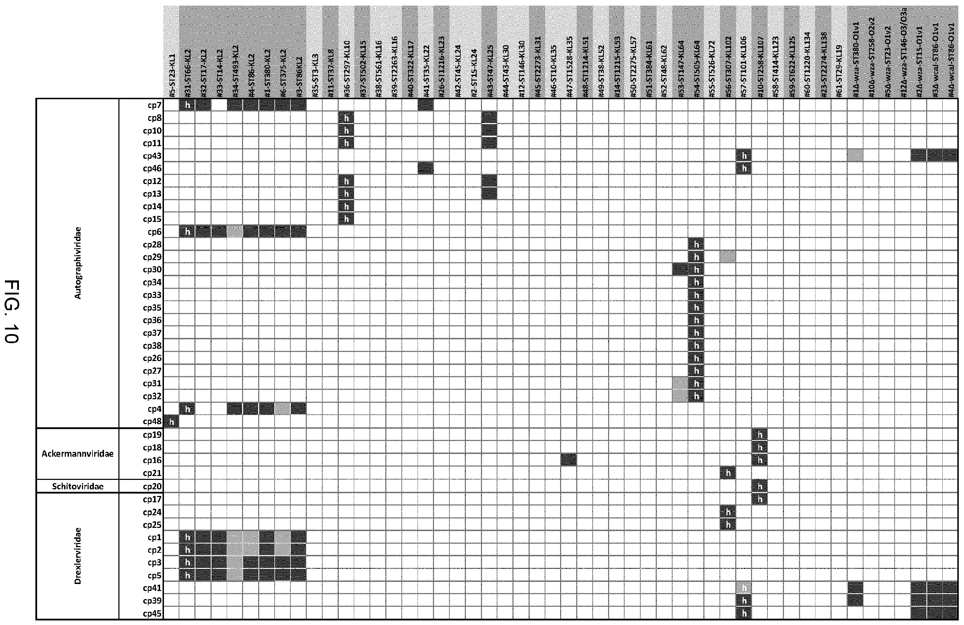

Hereafter, the prefix cp and mtp will be used for the names of anti-K phages and anti-Kd phages, respectively. Consistent with literature, anti-K phages mainly targeted WT strains of a unique K-type (Figure 10) when tested against 50 different strains (31 KL-types) (Table 2). Exceptions included (i) phages cp8-cp13 that were able to target KL10 and KL25 strains; (ii) phage cp34, which infected KL64 and KL102 strains; and (iii) phage cp16, which targeted KL35 and KL107 strains. Additionally, phages cp39, cp41 and cp45, which infected KL106 WT strains, were also capable to target five out of seven capsule-deficient mutant strains (Figure 10).

In sharp contrast, anti-Kd phages infected a broader range of strains, with all infecting at least 3 different O-types on the 7 capsule deficient mutant strains tested (Figure 2, Figure 9). As expected, most of anti-Kd phages were not active against the WT (capsulated) strains.

To investigate further the host-range breadth of anti-Kd phages, we generated 23 spontaneous non-mucoid Kp clones belonging to 22 different STs and representing 10 different O-types out of the 11 currently known (exception of O8, due to its low prevalence, representing only 0.03% of the 7,388 genomes analysed; Table 2) (Follador et al. 2016; Wick et al. 2018). With the exceptions of mtp1 and mtp8, which only targeted 10 and 16 strains, respectively, anti-Kd phages infected 20 to 28 strains out of the 30 capsule-deficient or non-mucoid strains (Figure 2). Among these, the broadest host-range phages were: (i) mtp5, which infected the 30 strains; (ii) mtp7, mtp17, mtp18, mtp20 and mtp21 (28 strains each); and (iii) mtp6, mtp19 and mtp25 (27 strains each; Figure 2).

### Mtp5 and mtp7, phages with broad host range against non-capsulated K. pneumoniae

Phage mtp5 was isolated against strain BJ1-GAΔwza and was able to target all 30 tested non-capsulated strains **(****Figure 2****).** This phage presented a 175.1 kb genome composed of 288 CDSs and 1 tRNA gene and belongs to the Myoviridae family (Tevenvirinae subfamily and Slopekvirus genus). Its genomic structure was closely related (ID ranging from 98.43% to 98.98%, and coverage of 94 to 97%) to phage mtp7 and other previously described anti-K. *pneumoniae* phages Matisse, Kp27, Kp15, Miro and PMBT1 (Maciejewska et al. 2017; Provasek et al. 2015; Mijalis et al. 2015; Koberg et al. 2017), with the main genomic difference being found in one of the two tail fiber genes **(****Figure 12****).** Phage mtp7 was isolated against strain NJST258_2Δwza and was able to target 28 of the 30 tested non-capsulated strains. This phage has a genome of 177.7 kb, composed by 293 CDS and 1 tRNA gene and also belongs to the Myoviridae family (Tevenvirinae subfamily and Slopekvirus genus).

Efficiency of plating (EOP) assays confirmed the ability of mtp5 to replicate in all non-capsulated strains **(****Figure 3A****)** belonging to at least 23 different STs and 10 different O-types. Further, when tested against WT (capsulated) KpSC strains in combination with anti-capsule phages **(****Figure 3B****),** phage mtp5 improved the lysis effect for all tested strains with the single exception of strain 899 (ST101-KL106-O1v2; **Figure 3B****).** Phage mtp7 was also tested in combination with anti-capsule phage cp17 and showed to improve the lysis effect **(****Figure 13****).**

### Resistance against anti-K^{d} phages emerges more slowly than against anti-K phages

The emergence of subpopulations of bacteria that escape phage predation is typically observed in *in vitro* bacterial cultures with phages (Hernandez and Koskella 2019; Lourenço et al. 2020). We compared the timing of this phenomenon for anti-K^{d} phages and anti-K phages **(****Figure 14****).** With both categories of phages, the impact of infection becomes visible 45 to 60 min post-infection. However, resurgence of growth was significantly delayed in bacterial cultures infected with anti-K^{d} phages when compared to those infected with anti-K phages (AUC ratios: 0.2 to 0.4 vs 0.4 to 0.6, respectively; p-value = 0.006, Mann-Whitney test; **Figure 14C****).** At the same time, the relative bacterial growth (RBG) estimations at 4 h from both cultures with anti-K or anti-K^{d} phages were very low (from <0 to 0.2, p-value=0.4323) for the majority of strains, indicating the high efficiency of the phages in controlling the bacterial population. Still, after 6 h, 8 h and 10 h, the RBG of the non-susceptible populations was significantly lower for non-capsulated strains than for capsulated strains (p-value= 1.119x10⁻⁰⁶, 2.204x10⁻⁰⁴ and 3.332x10⁻⁰⁴, respectively) **(****Figure 14****, panels D and E).** These results highlight that the emergence of phage escape mutants was slower for anti-K^{d} phages.

### Differences in genomic changes associated with anti-K and anti-Kd phages resistance

We next characterized and compared the bacterial genetic changes associated with anti-K and anti-Kd phage resistance. For this purpose, we first used seven WT strains/anti-K phage pairs and observed that the majority of mutations that were detected in the non-susceptible population occurred in the cps cluster genes, mainly in wcaJ or wbaP (Figure 4A, Figure 15, Table 6). These mutations included single-nucleotide polymorphisms (SNPs) and disruptions by insertion sequences (IS); and the relative frequency of these two events varied depending on the strains/phage combination. Interestingly, in one case wbaP was disrupted by an IS91 family element, which harboured by one of the plasmids of the strain (Figure 4A, Table 6).

In contrast, resistance against anti-Kd phages was associated with more variable events (Figure 4B, Table 6). We detected mutations in the ferrichrome-iron receptor fhuA and in the tonB transport system (04A025/mtp27), in LPS synthesis genes (NJST258_2Δwza/mtp7) or more particularly in the O-antigen locus; for example, in gspA (BJ1GAΔwza/mtp4), or in rfb or galE (NTUH_K2044Δwza/mtp8) and wbaP (NTUH_K2044Δwza/mtp6 - more information in supplementary text 2). We also detected mutations on mfpsA (encoding a mannosylfructose-phosphate synthase) and mntH (encoding a divalent metal cation transporter) (342Δwza/mtp19 pair). Overall, mutations in 22 distinct genes were found among anti-Kd resistant populations. These results indicate that a variety of surface structures were disrupted in the phage resistant mutants, possibly corresponding to a diversity of receptors used by the anti-Kd phages. Next we tested several cocktails using both anti-K and anti-Kd phages. These cocktails including also phage mtp5 are discussed on the paragraph below.

### Mutations associated with resistance against phages mtp5 and mtp7

Mtp5-resistant populations of BJ1GAΔwza (its original host) emerged after around 6.5 h, but no mutations were detected (as assessed at 18 h of infection). In contrast, mtp5-resistant populations of NJST258_2Δwza showed mutations in several genes related to membrane biosynthesis, including in gene RS00710, encoding a glycosyltransferase involved in core LPS biosynthesis **(****Figure 4B****).** For the NJST258_2Δwza/mtp7 pair, we found mutations in gene RS00730, coding for a polysaccharide deacetylase family protein located in the LPS locus **(****Figure 4B****).**

We next searched for resistance mutations when using two-phage cocktails of mtp5 against capsulated strains **(****Figure 5****).** We observed a single widespread mutation in *gaIU* (BJ1GA/cp1+mtp5)(also detected in BJ1GA/cp1+mtp4) or a non-synonymous SNP mutation in gene *acpP* (NCTC8172/cp34+mtp5), coding for an acyl carrier protein involved in fatty acid biosynthesis. No mutations were found in *wcaJ* or *wbaP cps* genes both in BJ1GA and NCTC8172, suggesting mtp5 resistance mutations can be sufficient to prevent infection by the two phages **(****Figure 5****).** These results show that resistance to mtp5 is linked to the production of the initiation building-blocks of the membrane biosynthesis.

We also tested phage mtp5 in cocktail with a second anti-K^{d} phage, mtp4, against the capsule-deficient mutant strain BJ1GAΔ*wza*. The resistant population showed an 89 kb deletion, comprising 72 different genes encoding for capsule and O-antigen synthesis, and some genes of the core LPS biosynthesis. Additionally, this big deletion also removed the histidine operon and disrupted the *pksJ* gene from the colibactin operon as well as the chaperone *dnaK* gene. Other anti-K^{d} phages, mtp4, mtp6 and mtp7 were also tested and resistance accessed in conjugation with anti-K phages (Figure 13).

Using the combination NJST258_2 with cp17+mtp7 the non-susceptible population the most significant mutations presented mutations were on a LPS O-locus sugar glycosyltransferase and on a tonB-dependent receptor.

### Phage mtp5 can target K. pneumoniae within the mammalian gut

To investigate the ability of phage mtp5, alone and in combination with anti-K phage cp1, to target K *pneumoniae in vivo,* we used strain BJ1-GA, the original mtp5 host. Mtp5 showed not to infect BJ1GA WT in *in vitro* settings and cp1 the contrary, could infect the WT but not the capsule deficient strain **(****Figure 16A and B****).**

Both phages were used alone and in combination, to target K. pneumoniae strain BJ1-GA (the original host of both phages) colonising the mice gut. After a single dose of phage, results showed a small but not significant (Figure 15C) decrease of the Kp strain numbers in the feces on the subsequent days. However, we observed stable phage numbers, pointing to replication of both phages mtp5 and cp1, when used either alone or in combination in the mice gut (Figure 15D). When extending the phage administration for 2 more days, a decrease of the bacterial loads was again observed, but only on the first day after treatment (Figure 6AC), followed by a stabilisation of the bacterial and phage (Figure 6B) populations. Although 10-to-100-fold decrease was observed on the numbers of the host strain in the feces (no statistical significance was obtained possibly due to high variability of the samples, Figure 6C, Tables 7 and 8), we could again detect similar levels of phage and bacterial populations in the mice with the three consecutive phage treatments. These observations indicate that phage infection and replication of both phages took place in the mice gut.

From the different fecal samples collected at days 3, 4, 5 and 6, we could distinguish different colony phenotypes from the different groups of mice. We could observe the hyper-capsulated WT phenotype (called here large), medium colonies and small colony variants. For the control mice from days 4 to 6 we were able to identify the presence of the large and medium colony phenotypes but when testing for resistance, both colony phenotypes appeared susceptible to cp1 throughout the experiment but resistance to mtp5 at day 4 but and with an intermediate susceptibility (as seen before, with lysis at high concentration but no plaques at lower dilutions) at days 5 and 6 (Figure 17A and E). A similar pattern was observed for mice receiving phage mtp5 (Figure 17B and E). On the other hand for phage cp1 mice all phenotypes were detected after phage treatment, showing different susceptibility to the phages (Figure 17C and E). Surprisingly the clone tested for one of the phenotypes (small) revealed to be susceptible to both phages. Similar phenotypic patterns were observed on the mouse treated with both phages (Figure 17D and E). This diversity of phenotypes suggests that, first while the mucoid phenotype is expressed, subpopulations that have either lost hyper-capsule production, or lost capsule production at all, co-exist and second that resistant clones may be emerging. Non-capsulated bacteria could give access to the anti-Kd phage, with consequent infection and replication. For certain plates we were also able to detect very small colony variants, which showed to be enterococcus faecalis by maldi-tof analysis.

### Discussion

Capsular structure diversity in Klebsiella is a major hurdle for anti-Klebsiella phage therapy. Recent global population studies (Lam, Wick, Watts, et al. 2021) have shown that cocktails of > 20 capsular (K)-types or 3 O-types would be needed to cover 80% of K. pneumoniae population involved in human infections. An exception to the major constraint posed by K-type diversity in K. pneumoniae is the success of the capsule specialist phages for liver abscess, a localized infection which is mainly associated with K1 and K2 (Lin et al. 2014; Hung et al. 2011).

Despite the above, so far most phages targeting K. pneumoniae have been isolated from wild-type and thus capsulated strains. Klebsiella capsule is normally abundant and is thus the structure that acts as receptor for the majority of anti-Klebsiella phages described. The capsular host range is indeed generally restricted to the K-type of the host from which the phage was isolated (Rieger-Hug and Stirm 1981; Lin et al. 2014; Fang et al. 2022; Eckstein et al. 2021; Chen et al. 2022; Fang and Zong 2022). Consistently, a marked host specificity was observed for anti-K phages isolated in this study, which in general were only able to target a unique K-type. One exception was observed with some of the phages (cp8, cp10-cp13) isolated from a KL10 host strain, which were also able to infect KL25 strains, possibly explained by the presence of different depolymerase domains in this phage (See Tables 4 and 5). Additionally, our isolation of phages (cp41, cp45 and cp39) which infected KL106 WT strains but also some capsule-deficient ones, is consistent with previous findings suggesting that in the widespread ST258 lineage that exhibits this KL type, capsule production is limited to low levels (Castronovo et al. 2017); the isolated phages may therefore have non-capsular structures as receptors. Despite the dominant picture of capsule specificity of anti-K phages, notable rare exceptions have been described, with phages carrying multiple depolymerase domains. However, the most broad-range phage was able to infect only up to 11 different KL-types (Pan et al. 2017; Šimoliu̅nas et al. 2013; Townsend et al. 2021), which remains limited in face of the approximately 200 described or genomics-inferred K types.

Recent in vitro studies have shown that K. pneumoniae capsulated strains typically evolve phage resistance through capsule production inactivation (Hesse et al. 2020; D. Tan et al. 2020; Cai et al. 2019; Majkowska-Skrobek et al. 2021). Consistently, we found that emergence of resistance against anti-K phages is rapid and revolves mainly around mutations or disruptions mediated by ISs on wcaJ (encoding an undecaprenyl-phosphate glucose-1-phosphate transferase) or wbaP (encoding an undecaprenyl phosphate galactose transferase). These glycosyltransferases are responsible for one of the first steps of capsule production and are major targets for capsule loss (Haudiquet et al. 2021) and phage resistance in K. pneumoniae (Hesse et al. 2020; D. Tan et al. 2020; Cai et al. 2019). In one case, wbaP disruption was due to insertion by an IS9 initially present on a plasmid, highlighting their possible role on phage resistance. Moreover, the co-occurrence of different mutations in wcaJ and wbaP genes in the same population, reveals that parallel resistance evolution is occurring. Taken together, these observations point out the importance of finding alternative receptor targets for the prospect of phage therapy to tackle K. pneumoniae infections (Rodriguez-Valera et al. 2009).

Here, we explored a strategy where phages against capsule-deficient (anti-Kd phages) K. pneumoniae could be isolated, and subsequently used either alone or combined with anti-K phages. Consistent with our initial hypothesis, phylogenetically diverse anti-Kd phages were isolated and turned out to infect a broad range of non-capsulated strains. The strongest illustration of the breadth of possible host ranges of anti-Kd phages, was provided by phage mtp5. This phage targeted the majority of non-capsulated K. pneumoniae strains, either against capsule-deficient strains or against WT K. pneumoniae when in combination with anti-K phages. However, for some of the strains infected with mtp5, we were not able to access the efficiency of plating due to the absence of phage plaques on lower dilutions. This phenomenon was observed for strains belonging to O-types OL1, OL2, OL3/O3a, OL4 and OL12, yet we were able to access phage numbers on other strains with the same O-types. These conditions might correspond to "lysis from without" in which high phage concentrations lead to bacterial lysis without actual infection and phage production (Abedon 2011).

In addition to their broad host range, anti-Kd phages had better resilience against emergence of resistance. Indeed, we observed extended time before regrowth of bacterial populations, and even more so when used in cocktail, in addition to a reduced relative bacterial growth, compared to anti-K phages. We explored the genomic changes behind anti-Kd phage resistance and observed that depending on the anti-Kd phages, resistance is the result of a diversity of mutations, suggesting that the LPS core plays a role in anti-Kd phages attachment consistent with broader host range exhibited by these phages. In line with these observations, resistance to mtp5 master phage, alone or in cocktail with anti-K phages, did not rely on mutation of a specific gene, operon or step of the LPS pathway, but rather disruption of genes that play an important role in the process of membrane biosynthesis. The evolution by loss of particular membrane proteins or components of the core LPS, may delay the development of anti-Kd phage resistance by requesting specific mutations and by necessitating physiological adjustments against important changes to the cell membrane composition.

An important question for the prospect of phage therapy is the efficacy of phage *in vivo.* Our mouse colonization model showed replication of anti-K and anti-Kd phages in the gut, with a small reduction on the bacterial loads on the day after the first treatment but leading to a coexistence of both populations on the following days independently of one or three-day treatment. These dynamics are reminiscent of other studies that suggested a coexistence of bacterial and virulent phages populations in the mice gut (Maura et al. 2012; Lourenço et al. 2020; B. B. Hsu et al. 2019; Mirzaei and Maurice 2017; Kirsch et al. 2021).

Interestingly, our data suggest a capacity of phage mtp5 to target the WT strain in the gut. This was implied by the decrease in bacterial load and populations coexistence observed when this phage was used individually. This observation raises an interesting hypothesis that capsule expression may not be universal in the gut, either due to non-uniform expression along the gastrointestinal tract, or over time, or through other variation patterns even in the absence of selection by other phage. Further analysis of the bacterial populations of the mice exposed or not to the phages allowed us to depict the presence of several different colony phenotypes (Figure 16). Spot assays showed the emergence of different resistant profiles and its coexistence within the gut either with or without capsule after phage exposure. In mice exposed to phage mtp5 we could not see major changes, with the dominance of the capsulated phenotype. On the other hand mice exposed to phage cp1 or the cocktail showed the presence of different colony sizes, big (hyper-capsulated,) medium (capsulated) or small all showing different resistance profiles including susceptibility to both phages. These results suggest that despite the presence of low-producing capsule clones in the gut, that lead to mtp5 replication, the loss of capsule production may be more costly than its down-regulation. The complexity of the environment and the presence of multiple organisms in the gut leads to multiple ecological interactions, and capsule expression may incur variable fitness costs (Y. H. Tan et al. 2020; Lourenço et al. 2022; Koskella et al. 2012). Different subpopulations of less capsulated/non-capsulated or capsulated Kp may occupy different niches in the gut environment. Understanding the expression of Kp capsule in different sites *in vivo* will be important to define the potential of anti-Kd phages.

In summary, we show that anti-Kd phages, i.e. anti-Klebsiella phages isolated from capsule deficient strains, combine interesting properties for the prospect of phage therapy: broad host range, slower emergence of resistance, and synergy with anti-K phages. Some anti-Kd phages such as mtp5, had a nearly complete broad host range, suggesting that the targeting of conserved structures (possibly conserved beyond the single Kp species) located beneath the capsule is a realistic avenue to circumvent the narrow spectrum conundrum of anti-capsule phages. Our *in vivo* experiments showed that such phages may replicate in the mouse gut even in WT strains. More work is needed on the question of the expression of the capsule *in vivo* given its implications for anti-Klebsiella phage therapy.

### References

Abedon, Stephen T. 2011. 'Lysis from Without'. Bacteriophage 1 (1): 46-49. https://doi.org/10.4161/bact.1.1.13980.
Alfano, C., and R. McMacken. 1989. 'Heat Shock Protein-Mediated Disassembly of Nucleoprotein Structures Is Required for the Initiation of Bacteriophage Lambda DNA Replication'. The Journal of Biological Chemistry 264 (18): 10709-18.
Antimicrobial Resistance Collaborators. 2022. 'Global Burden of Bacterial Antimicrobial Resistance in 2019: A Systematic Analysis'. Lancet (London, England) 399 (10325): 629-55. https://doi.org/10. 1016/S0140-6736(21)02724-0.
Aziz, Ramy K., Daniela Bartels, Aaron A. Best, Matthew DeJongh, Terrence Disz, Robert A. Edwards, Kevin Formsma, et al. 2008. 'The RAST Server: Rapid Annotations Using Subsystems Technology'. BMC Genomics 9 (February): 75. https://doi.org/10. 1186/1471-2164-9-75.
Bates, Douglas, Martin Mächler, Ben Bolker, and Steve Walker. 2014. 'Fitting Linear Mixed-Effects Models Using Lme4'. ArXiv.-1406.5823[Stat], June. http://arxiv.org/abs/1406.5823.
Bengoechea, Jose A., and Joana Sa Pessoa. 2019. 'Klebsiella Pneumoniae Infection Biology: Living to Counteract Host Defences'. FEMS Microbiology Reviews 43 (2): 123-44. https://doi.org/10.1 093/femsre/fuy043.
Bonilla, Estrada, Ana Rita Costa, Daan F. van den Berg, Teunke van Rossum, Stefan Hagedoorn, Hielke Walinga, Minfeng Xiao, et al. 2021. 'Genomic Characterization of Four Novel Bacteriophages Infecting the Clinical Pathogen Klebsiella Pneumoniae'. DNA Research: An International Journal for Rapid Publication of Reports on Genes and Genomes 28 (4): dsab013. https://doi.org/10. 1093/dnares/dsab013.
Bowers, Jolene R., Brandon Kitchel, Elizabeth M. Driebe, Duncan R. MacCannell, Chandler Roe, Darrin Lemmer, Tom de Man, et al. 2015. 'Genomic Analysis of the Emergence and Rapid Global Dissemination of the Clonal Group 258 Klebsiella Pneumoniae Pandemic'. PloS One 10 (7): e0133727. https://doi.org/10.1371/journal.pone.0133727.
Brettin, Thomas, James J. Davis, Terry Disz, Robert A. Edwards, Svetlana Gerdes, Gary J. Olsen, Robert Olson, et al. 2015. 'RASTtk: A Modular and Extensible Implementation of the RAST Algorithm for Building Custom Annotation Pipelines and Annotating Batches of Genomes'. Scientific Reports 5 (February): 8365. https://doi.org/10.1038/srep08365.
Brugiroux, Sandrine, Markus Beutler, Carina Pfann, Debora Garzetti, Hans-Joachim Ruscheweyh, Diana Ring, Manuel Diehl, et al. 2016. 'Genome-Guided Design of a Defined Mouse Microbiota That Confers Colonization Resistance against Salmonella Enterica Serovar Typhimurium'. Nature Microbiology 2 (November): 16215. https://doi.org/10.1038/nmicrobiol.2016.215.
Cai, Ruopeng, Gang Wang, Shuai Le, Mei Wu, Mengjun Cheng, Zhimin Guo, Yalu Ji, et al. 2019. 'Three Capsular Polysaccharide Synthesis-Related Glucosyltransferases, GT-1, GT-2 and WcaJ, Are Associated With Virulence and Phage Sensitivity of Klebsiella Pneumoniae'. Frontiers in Microbiology 10: 1189. https://doi.org/10.3389/fmicb.2019.01189 .
Castronovo, Giuseppe, Ann Maria Clemente, Alberto Antonelli, Marco Maria D'Andrea, Michele Tanturli, Eloisa Perissi, Sara Paccosi, et al. 2017. 'Differences in Inflammatory Response Induced by Two Representatives of Clades of the Pandemic ST258 Klebsiella Pneumoniae Clonal Lineage Producing KPC-Type Carbapenemases'. PloS One 12 (1): e0170125. https://doi.org/10.1371/journal.pone.0170125.
Chen, Xinyi, Qi Tang, Xue Li, Xiangkuan Zheng, Pei Li, Min Li, Fei Wu, Zhengjun Xu, Renfei Lu, and Wei Zhang. 2022. 'Isolation, Characterization, and Genome Analysis of Bacteriophage P929 That Could Specifically Lyase the KL19 Capsular Type of Klebsiella Pneumoniae'. Virus Research, March, 198750. https://doi.org/10.1016/j.virusres.2022.198750.
Chiarelli, Adriana, Nicolas Cabanel, Isabelle Rosinski-Chupin, Pengdbamba Dieudonne Zongo, Thierry Naas, Rémy A. Bonnin, and Philippe Glaser. 2020. 'Diversity of Mucoid to Non-Mucoid Switch among Carbapenemase-Producing Klebsiella Pneumoniae'. BMC Microbiology 20 (1): 325. https://doi.org/10.1186/s12866-020-02007-y.
Cokelaer, Thomas, Dimitri Desvillechabrol, Rachel Legendre, and Melissa Cardon. 2017. ‴Sequana": A Set of Snakemake NGS Pipelines'. The Journal of Open Source Software 2 (16): 352. https://doi.org/10.21105/joss.00352 .
Criscuolo, Alexis. 2019. 'A Fast Alignment-Free Bioinformatics Procedure to Infer Accurate Distance-Based Phylogenetic Trees from Genome Assemblies'. Research Ideas and Outcomes 5 (June): e36178. https://doi.org/10.3897/rio.5.e36178 .
Eckstein, Simone, Jana Stender, Sonia Mzoughi, Kilian Vogele, Jana Kühn, Daniela Friese, Christina Bugert, et al. 2021. 'Isolation and Characterization of Lytic Phage TUN1 Specific for Klebsiella Pneumoniae K64 Clinical Isolates from Tunisia'. BMC Microbiology 21 (1): 186. https://doi.org/10.1186/s12866-021-02251-w.
Fang, Qingqing, Yu Feng, Alan McNally, and Zhiyong Zong. 2022. 'Characterization of Phage Resistance and Phages Capable of Intestinal Decolonization of Carbapenem-Resistant Klebsiella Pneumoniae in Mice'. Communications Biology 5 (1): 48. https://doi.org/10.1038/s42003-022-03001-y.
Fang, Qingqing, and Zhiyong Zong. 2022. 'Lytic Phages against ST11 K47 Carbapenem-Resistant Klebsiella Pneumoniae and the Corresponding Phage Resistance Mechanisms'. MSphere, March, e0008022. https://doi.org/10.1128/msphere.00080-22 .
Follador, Rainer, Eva Heinz, Kelly L. Wyres, Matthew J. Ellington, Michael Kowarik, Kathryn E. Holt, and Nicholas R. Thomson. 2016. 'The Diversity of Klebsiella Pneumoniae Surface Polysaccharides'. Microbial Genomics 2 (8): e000073. https://doi.org/101099/mgen0000073
Fox, John, and Sanford Weisberg. 2019. An R Companion to Applied Regression. Third edition. Los Angeles: SAGE.
Garneau, Julian R., Florence Depardieu, Louis-Charles Fortier, David Bikard, and Marc Monot. 2017. 'PhageTerm: A Tool for Fast and Accurate Determination of Phage Termini and Packaging Mechanism Using next-Generation Sequencing Data'. Scientific Reports 7 (1): 8292. https://doi.org/10.1038/s41598-017-07910-5.
Gorodnichev, Roman B., Nikolay V. Volozhantsev, Valentina M. Krasilnikova, Ivan N. Bodoev, Maria A. Kornienko, Nikita S. Kuptsov, Anastasia V. Popova, et al. 2021. 'Novel Klebsiella Pneumoniae K23-Specific Bacteriophages From Different Families: Similarity of Depolymerases and Their Therapeutic Potential'. Frontiers in Microbiology 12: 669618. https://doi.org/10.3389/fmicb.2021.669618.
Hao, Guijuan, Rundong Shu, Liqin Ding, Xia Chen, Yonghao Miao, Jiaqi Wu, Haijian Zhou, and Hui Wang. 2021. 'Bacteriophage SRD2021 Recognizing Capsular Polysaccharide Shows Therapeutic Potential in Serotype K47 Klebsiella Pneumoniae Infections'. Antibiotics (Basel, Switzerland) 10 (8): 894. https://doi.org/10.3390/antibiotics10080894.
Haudiquet, Matthieu, Amandine Buffet, Olaya Rendueles, and Eduardo P. C. Rocha. 2021. 'Interplay between the Cell Envelope and Mobile Genetic Elements Shapes Gene Flow in Populations of the Nosocomial Pathogen Klebsiella Pneumoniae'. PLoS Biology 19 (7): e3001276. https://doi.org/10.1371/journal.pbio.3001276.
Hennart, Melanie, Julien Guglielmini, Martin C.J. Maiden, Keith A. Jolley, Alexis Criscuolo, and Sylvain Brisse. 2021. 'A Dual Barcoding Approach to Bacterial Strain Nomenclature: Genomic Taxonomy of Klebsiella Pneumoniae Strains'. Preprint. Microbiology. https://doi.org/10.1101/2021.07.26.453808.
Hernandez, Catherine A., and Britt Koskella. 2019. 'Phage Resistance Evolution in Vitro Is Not Reflective of in Vivo Outcome in a Plant-Bacteria-Phage System'. Evolution; International Journal of Organic Evolution 73 (12): 2461-75. https://doi.org/10.1111/evo.13833.
Hesse, Shayla, Manoj Rajaure, Erin Wall, Joy Johnson, Valery Bliskovsky, Susan Gottesman, and Sankar Adhya. 2020. 'Phage Resistance in Multidrug-Resistant Klebsiella Pneumoniae ST258 Evolves via Diverse Mutations That Culminate in Impaired Adsorption'. MBio 11 (1): e02530-19. https://doi.org/10.1128/mBio.02530-19.
Hockenberry, Adam J., and Claus O. Wilke. 2021. 'BACPHLIP: Predicting Bacteriophage Lifestyle from Conserved Protein Domains'. PeerJ 9: e11396. https://doi.org/10.7717/peerj.11396.
Hoyles, Lesley, James Murphy, Horst Neve, Knut J. Heller, Jane F. Turton, Jennifer Mahony, Jeremy D. Sanderson, et al. 2015. 'Klebsiella Pneumoniae Subsp. Pneumoniae-Bacteriophage Combination from the Caecal Effluent of a Healthy Woman'. PeerJ 3: e1061. https://doi.org/10.7717/peerj.1061.
Hsu, Bryan B., Travis E. Gibson, Vladimir Yeliseyev, Qing Liu, Lorena Lyon, Lynn Bry, Pamela A. Silver, and Georg K. Gerber. 2019. 'Dynamic Modulation of the Gut Microbiota and Metabolome by Bacteriophages in a Mouse Model'. Cell Host & Microbe 25 (6): 803-814.e5. https://doi.org/10.1016/j.chom.2019.05.001.
Hsu, C. R., T. L. Lin, Y. C. Chen, H. C. Chou, and J. T. Wang. 2011. 'The Role of Klebsiella Pneumoniae RmpA in Capsular Polysaccharide Synthesis and Virulence Revisited'. Microbiology 157 (Pt 12): 3446-57. https://doi.org/10.1099/mic.0.050336-0.
Hung, Chih-Hsin, Chih-Feng Kuo, Chiou-Huey Wang, Ching-Ming Wu, and Nina Tsao. 2011. 'Experimental Phage Therapy in Treating Klebsiella Pneumoniae-Mediated Liver Abscesses and Bacteremia in Mice'. Antimicrobial Agents and Chemotherapy 55 (4): 1358-65. https://doi.org/10.1128/AAC.01123-10.
Keseler, Ingrid M., Julio Collado-Vides, Alberto Santos-Zavaleta, Martin Peralta-Gil, Socorro Gama-Castro, Luis Muriiz-Rascado, César Bonavides-Martinez, et al. 2011. 'EcoCyc: A Comprehensive Database of Escherichia Coli Biology'. Nucleic Acids Research 39 (Database issue): D583-590. https://doi.org/10.1093/nar/gkq1143.
Kirsch, Joshua M., Robert S. Brzozowski, Dominick Faith, June L. Round, Patrick R. Secor, and Breck A. Duerkop. 2021. 'Bacteriophage-Bacteria Interactions in the Gut: From Invertebrates to Mammals'. Annual Review of Virology 8 (1): 95-113. https://doi.org/10.1146/annurev-virology-091919-101238.
Koberg, Sabrina, Erik Brinks, Gregor Fiedler, Christina Hüsing, Gyu-Sung Cho, Marc P. Hoeppner, Knut J. Heller, Horst Neve, and Charles M. A. P. Franz. 2017. 'Genome Sequence of Klebsiella Pneumoniae Bacteriophage PMBT1 Isolated from Raw Sewage'. Genome Announcements 5 (8): e00914-16. https://doi.org/10.1128/genomeA.00914-16 .
Koskella, Britt, Derek M. Lin, Angus Buckling, and John N. Thompson. 2012. 'The Costs of Evolving Resistance in Heterogeneous Parasite Environments'. Proceedings. Biological Sciences 279 (1735): 1896-1903. https://doi.org/10.1098/rspb.2011.2259.
Kuznetsova, Alexandra, Per B. Brockhoff, and Rune H. B. Christensen. 2017. 'LmerTest Package: Tests in Linear Mixed Effects Models'. Journal of Statistical Software 82 (13). https://doi.org/10.18637/jss.v082.i13.
Lam, Margaret M. C., Ryan R. Wick, Louise M. Judd, Kathryn E. Holt, and Kelly L. Wyres. 2021. 'Kaptive 2.0: Updated Capsule and LPS Locus Typing for the Klebsiella Pneumoniae Species Complex'. Preprint. Genomics. https://doi.org/10.1101/2021.11.05.467534.
Lam, Margaret M. C., Ryan R. Wick, Stephen C. Watts, Louise T. Cerdeira, Kelly L. Wyres, and Kathryn E. Holt. 2021. 'A Genomic Surveillance Framework and Genotyping Tool for Klebsiella Pneumoniae and Its Related Species Complex'. Nature Communications 12(1): 4188. https://doi.org/10.1038/s41467-021-24448-3.
Lenth, Russell V. 2016. 'Least-Squares Means: The R Package Lsmeans'. Journal of Statistical Software 69 (1). https://doi.org/10.18637/jss.v069.i01.
Letunic, Ivica, and Peer Bork. 2019. 'Interactive Tree Of Life (ITOL) v4: Recent Updates and New Developments'. Nucleic Acids Research 47 (W1): W256-59. https://doi.org/10.1093/nar/gkz239.
Levin, B. R. 1988. 'Frequency-Dependent Selection in Bacterial Populations'. Philosophical Transactions of the Royal Society of London. Series B, Biological Sciences 319 (1196): 459-72. https://doi.org/10.1098/rstb.1988.0059.
Lin, Tzu-Lung, Pei-Fang Hsieh, Yu-Tsung Huang, Wei-Ching Lee, Yi-Ting Tsai, Po-An Su, Yi-Jiun Pan, Chun-Ru Hsu, Meng-Chuan Wu, and Jin-Town Wang. 2014. 'Isolation of a Bacteriophage and Its Depolymerase Specific for K1 Capsule of Klebsiella Pneumoniae: Implication in Typing and Treatment'. The Journal of Infectious Diseases 210 (11): 1734-44. https://doi.org/10.1093/infdis/jiu332.
Lourenço, Marta, Lorenzo Chaffringeon, Quentin Lamy-Besnier, Thierry Pedron, Pascal Campagne, Claudia Eberl, Marion Bérard, Barbel Stecher, Laurent Debarbieux, and Luisa De Sordi. 2020. 'The Spatial Heterogeneity of the Gut Limits Predation and Fosters Coexistence of Bacteria and Bacteriophages'. Cell Host & Microbe 28 (3): 390-401.e5. https://doi.org/10.1016/j.chom.2020.06.002.
Maciejewska, Barbara, Bartosz Roszniowski, Akbar Espaillat, Agata K 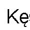 sik-Szeloch, Grazyna Majkowska-Skrobek, Andrew M. Kropinski, Yves Briers, Felipe Cava, Rob Lavigne, and Zuzanna Drulis-Kawa. 2017. 'Klebsiella Phages Representing a Novel Clade of Viruses with an Unknown DNA Modification and Biotechnologically Interesting Enzymes'. Applied Microbiology and Biotechnology 101 (2): 673-84. https://doi.org/10.1007/s00253-016-7928-3.
Majkowska-Skrobek, Grazyna, Pawel Markwitz, Ewelina Sosnowska, Cédric Lood, Rob Lavigne, and Zuzanna Drulis-Kawa. 2021. 'The Evolutionary Trade-Offs in Phage-Resistant Klebsiella Pneumoniae Entail Cross-Phage Sensitization and Loss of Multidrug Resistance'. Environmental Microbiology 23 (12): 7723-40. https://doi.org/10.1111/1462-2920.15476.
Maura, Damien, Eric Morello, Laurence du Merle, Perrine Bomme, Chantal Le Bouguenec, and Laurent Debarbieux. 2012. 'Intestinal Colonization by Enteroaggregative Escherichia Coli Supports Long-Term Bacteriophage Replication in Mice'. Environmental Microbiology 14 (8): 1844-54. https://doi.org/10.1111/j.1462-2920.2011.02644.x.
Mavrich, Travis N., and Graham F. Hatfull. 2017. 'Bacteriophage Evolution Differs by Host, Lifestyle and Genome'. Nature Microbiology 2 (July): 17112. https://doi.org/10.1038/nmicrobioi.2017.112.
Mijalis, Eleni M., Lauren E. Lessor, Jesse L. Cahill, Eric S. Rasche, and Gabriel F. Kuty Everett. 2015. 'Complete Genome Sequence of Klebsiella Pneumoniae Carbapenemase-Producing K. Pneumoniae Myophage Miro'. Genome Announcements 3 (5): e01137-15. https://doi.org/10.1128/genomeA.01137-15.
Mirzaei, Mohammadali Khan, and Corinne F. Maurice. 2017. 'Ménage a Trois in the Human Gut: Interactions between Host, Bacteria and Phages'. Nature Reviews. Microbiology 15 (7): 397-408. https://doi.org/10.1038/nrmicro.2017.30.
Ørskov, I., and F. Ørskov. 1984. '4 Serotyping of Klebsiella'. In Methods in Microbiology, 14:143-64. Elsevier. https://doi.org/10.1016/S0580-9517(08)70449-5.
Pan, Yi-Jiun, Tzu-Lung Lin, Ching-Ching Chen, Yun-Ting Tsai, Yi-Hsiang Cheng, Yi-Yin Chen, Pei-Fang Hsieh, Yi-Tsung Lin, and Jin-Town Wang. 2017. 'Klebsiella Phage ΦK64-1 Encodes Multiple Depolymerases for Multiple Host Capsular Types'. Journal of Virology 91 (6): e02457-16. https://doi.org/10.1128/JVI.02457-16.
Pertics, Botond Zsombor, Alysia Cox, Adrienn Nyúl, Nóra Szamek, Tamás Kovács, and György Schneider. 2021. 'Isolation and Characterization of a Novel Lytic Bacteriophage against the K2 Capsule-Expressing Hypervirulent Klebsiella Pneumoniae Strain 52145, and Identification of Its Functional Depolymerase'. Microorganisms 9 (3): 650. https://doi.org/10.3390/microorganisms9030650.
Prjibelski, Andrey, Dmitry Antipov, Dmitry Meleshko, Alia Lapidus, and Anton Korobeynikov. 2020. 'Using SPAdes De Novo Assembler'. Current Protocols in Bioinformatics 70 (1): e102. https://doi.org/10.1002/cpbi.102.
Provasek, Vincent E., Lauren E. Lessor, Jesse L. Cahill, Eric S. Rasche, and Gabriel F. Kuty Everett. 2015. 'Complete Genome Sequence of Carbapenemase-Producing Klebsiella Pneumoniae Myophage Matisse'. Genome Announcements 3 (5): e01136-15. https://doi.org/10.1128/genomeA.01136-15.
Rawlings, M., and J. E. Cronan. 1992. 'The Gene Encoding Escherichia Coli Acyl Carrier Protein Lies within a Cluster of Fatty Acid Biosynthetic Genes'. The Journal of Biological Chemistry 267 (9): 5751-54.
Rice, Louis B. 2008. 'Federal Funding for the Study of Antimicrobial Resistance in Nosocomial Pathogens: No ESKAPE'. The Journal of Infectious Diseases 197 (8): 1079-81. https://doi.org/10.1086/533452.
Rieger-Hug, D., and S. Stirm. 1981. 'Comparative Study of Host Capsule Depolymerases Associated with Klebsiella Bacteriophages'. Virology 113 (1): 363-78. https://doi.org/10.1016/0042-6822(81)90162-8.
Roach, Dwayne R., and Laurent Debarbieux. 2017. 'Phage Therapy: Awakening a Sleeping Giant'. Emerging Topics in Life Sciences 1 (1): 93-103. https://doi.org/10.1042/ETLS20170002.
Rodrigues, Carla, Siddhi Desai, Virginie Passet, Devarshi Gajjar, and Sylvain Brisse. 2022. 'Genomic Evolution of the Globally Disseminated Multidrug-Resistant Klebsiella Pneumoniae Clonal Group 147'. Microbial Genomics 8 (1). https://doi.org/10.1099/mgen.0.000737.
Rodrigues, Carla, Virginie Passet, Andriniaina Rakotondrasoa, Thierno Abdoulaye Diallo, Alexis Criscuolo, and Sylvain Brisse. 2019. 'Description of Klebsiella Africanensis Sp. Nov., Klebsiella Variicola Subsp. Tropicalensis Subsp. Nov. and Klebsiella Variicola Subsp. Variicola Subsp. Nov.' Research in Microbiology 170 (3): 165-70. https://doi.org/10.1016/j.resmic.2019.02.003.
Rodrigues, Carla, Clara Sousa, João A. Lopes, Ângela Novais, and Luísa Peixe. 2020. 'A Front Line on Klebsiella Pneumoniae Capsular Polysaccharide Knowledge: Fourier Transform Infrared Spectroscopy as an Accurate and Fast Typing Tool'. MSystems 5 (2): e00386-19. https://doi.org/10.1128/mSystems.00386-19.
Rodriguez-Valera, Francisco, Ana-Belen Martin-Cuadrado, Beltran Rodriguez-Brito, Lejla Pasić, T. Frede Thingstad, Forest Rohwer, and Alex Mira. 2009. 'Explaining Microbial Population Genomics through Phage Predation'. Nature Reviews. Microbiology 7 (11): 828-36. https://doi.org/10.1038/nrmicro2235.
Šimoliu̅nas, Eugenijus, Laura Kaliniene, Lidija Truncaitė, Aurelija Zajančkauskaitė, Juozas Staniulis, Algirdas Kaupinis, Marija Ger, Mindaugas Valius, and Rolandas Meškys 2013. 'Klebsiella Phage VB_KleM-RaK2-A Giant Singleton Virus of the Family Myoviridae'. Edited by Mark J. van Raaij. PLoS ONE 8 (4): e60717. https://doi.org/10.1371/journal.pone.0060717.
Sousa, Jorge A. M. de, Amandine Buffet, Matthieu Haudiquet, Eduardo P. C. Rocha, and Olaya Rendueles. 2020. 'Modular Prophage Interactions Driven by Capsule Serotype Select for Capsule Loss under Phage Predation'. The ISME Journal 14 (12): 2980-96. https://doi.org/10.1038/s41396-020-0726-z.
Tan, Demeng, Yiyuan Zhang, Jinhong Qin, Shuai Le, Jingmin Gu, Li-Kuang Chen, Xiaokui Guo, and Tongyu Zhu. 2020. 'A Frameshift Mutation in WcaJ Associated with Phage Resistance in Klebsiella Pneumoniae'. Microorganisms 8 (3): E378. https://doi.org/10.3390/microorganisms8030378.
Tan, Yi Han, Yahua Chen, Wilson H. W. Chu, Lok-To Sham, and Yunn-Hwen Gan. 2020. 'Cell Envelope Defects of Different Capsule-Null Mutants in K1 Hypervirulent Klebsiella Pneumoniae Can Affect Bacterial Pathogenesis'. MolecularMicrobiology 113 (5): 889-905. https://doi.org/10.1111/mmi.14447.
Townsend, Eleanor M., Lucy Kelly, Lucy Gannon, George Muscatt, Rhys Dunstan, Slawomir Michniewski, Hari Sapkota, et al. 2021. 'Isolation and Characterization of Klebsiella Phages for Phage Therapy'. PHAGE (New Rochelle, N.Y.) 2 (1): 26-42 https://doi.org/10.1089/phage.2020.0046.
Wick, Ryan R., Eva Heinz, Kathryn E. Holt, and Kelly L. Wyres. 2018. 'Kaptive Web: User-Friendly Capsule and Lipopolysaccharide Serotype Prediction for Klebsiella Genomes'. Journal of Clinical Microbiology 56 (6): e00197-18. https://doi.org/10.1128/JCM.00197-18.
Wyres, Kelly L, Ryan R. Wick, Louise M. Judd, Roni Froumine, Alex Tokolyi, Claire L. Gorrie, Margaret M. C. Lam, Sebastian Duchene, Adam Jenney, and Kathryn E. Holt. 2019. 'Distinct Evolutionary Dynamics of Horizontal Gene Transfer in Drug Resistant and Virulent Clones of Klebsiella Pneumoniae'. PLoS Genetics 15 (4): e1008114. https://doi.org/10.1371/journal.pgen.1008114.

## Claims

1. A bacteriophage targeting capsular-deficient *Klebsiella pneumoniaee* (Kp), wherein:
a. the bacteriophage comprises a tail fiber polypeptide of SEQ ID NO:1 or SEQ ID NO: 2 or a variant thereof having at least 96% identity with SEQ ID NO: 1 or SEQ ID NO: 2, and
b. said phage is capable of infecting at least two capsular-deficient *Klebsiella pneumoniae* of distinct O-types, in particular at least three distinct O-types of capsular-deficient *Klebsiella pneumoniae.*

2. The bacteriophage of claim 1, which further comprises a polypeptide that is a hinge connector of long tail fiber distal connector of SEQ ID NO: 3 or SEQ ID NO: 4 or a variant thereof having at least 99.9% identity with SEQ ID NO: 3 or SEQ ID NO: 4.

3. The bacteriophage of claim 1 or 2, which further comprises a polypeptide that is a hinge connector of long tail fiber proximal connector of SEQ ID NO: 5 or SEQ ID NO: 6 or a variant thereof having at least 99.5% identity with SEQ ID NO: 5 or SEQ ID NO: 6.

4. The bacteriophage of claims 1 to 3, which further comprises a polypeptide that is a long tail fiber proximal unit of SEQ ID NO: 7 or SEQ ID NO: 8.

5. The bacteriophage of claims 1 to 4, which further comprises a polypeptide that is a distal long tail fiber assembly catalyst of SEQ ID NO: 9 or SEQ ID NO: 10 or a variant thereof having at least 99.8% identity with SEQ ID NO: 9 or SEQ ID NO: 10.

6. The bacteriophage of any one of claim 1 to 5, which has the virion particle structure of a Myoviridae bacteriophage or is a Myoviridae bacteriophage.

7. The bacteriophage of any one of claim 1 to 6, which comprises a genomic sequence having at least 99% of identity with the genomic sequence ofvB_KpM_SB4496_mtp5 represented by SEQ ID NO: 11 or the genomic sequence of vB_KpM_SB4975_mtp7 represented by SEQ ID NO: 12.

8. The bacteriophage of any one of claims 1 to 7, which is:
a. the bacteriophage strain vB_KpM_SB4496_mtp5 deposited at the French National Collection of Microorganisms at the Institut Pasteur under Accession Number CNCM I-5855 on June 27, 2022 or a variant bacteriophage, wherein the variant has the same phenotypic and/or functional features as the parent bacteriophage, or
b. the bacteriophage strain vB_KpM_SB4975_mtp7 deposited at the French National Collection of Microorganisms at the Institut Pasteur under Accession Number CNCM I-5856 on June 27, 2022 or a variant of said bacteriophage, wherein the variant has the same phenotypic and/or functional features as the parent bacteriophage.

9. The bacteriophage of any one of claims 1 to 8, which is capable to produce a lytic infection in capsular-deficient *Klebsiella pneumoniae,* in particular a capsular-deficient *Klebsiella pneumoniae* BJ1-GA, more particularly the *Klebsiella pneumoniae* strain BJ1-GAΔ*wza* or a variant of said strain BJ1-GAΔwza, wherein the variant has the same phenotypic and/or functional features as the parent strain.

10. A pharmaceutical composition comprising the bacteriophage of any one of claims 1 to 9.

11. A composition of at least a bacteriophage of any one of claims 1 to 9 or a pharmaceutical composition of claim 10 on one hand, and at least another bacteriophage or a pharmaceutical composition comprising it on the second hand.

12. A nucleic acid molecule having at least 99% of identity with the genomic sequence of vB_KpM_SB4496_mtp5 represented by SEQ ID NO: 11 or the genomic sequence of vB_KpM_SB4975_mtp7 represented by SEQ ID NO: 12, or a nucleic acid molecule fragment of SEQ ID NO: 11 or SEQ ID NO: 12 or of a sequence having at least 99% of identity with SEQ ID NO: 11 or SEQ ID NO: 12.

13. The bacteriophage of any one of claims 1 to 9, or the pharmaceutical composition of claim 10, or the composition of claim 11, for use as a medicament.

14. The bacteriophage of any one of claims 1 to 9 or the pharmaceutical composition of claim 10, or the composition of claim 11, for use as a medicament against an infection caused by at least one type of *Klebsiella pneumoniae* (Kp), in particular where the infection is a nosocomial infection.

15. The bacteriophage of any one of claims 1 to 9, or the pharmaceutical composition of claim 10, or the composition of claim 11, for use according to claim 14, wherein the infection is a pulmonary infection, a pneumoniae, an urinary tract infection, a blood infection or a sepsis.

16. A method for decreasing the bacterial load of at least one *Klebsiella pneumoniae* (Kp) strain in the environment, comprising putting the environment in contact with an effective amount of a bacteriophage of any one of claims 1 to 9, or the pharmaceutical composition of claim 10, or the composition of claim 11, to the proviso that the environment is not an animal or human body, in particular where the environment is a food product, or a food or non-food contact equipment or surface.
